(19) Europäisches Patentamt
European Patent Office
Office européen des brevets

(11) Veröffentlichungsnummer: **0 275 820**
**B1**

(12) # EUROPÄISCHE PATENTSCHRIFT

(45) Veröffentlichungstag der Patentschrift: **06.02.91**

(51) Int. Cl.$^5$: **C 07 F 9/547,** A 61 K 31/675

(21) Anmeldenummer: **87810662.4**

(22) Anmeldetag: **16.11.87**

(54) **Ungesättigte Phosphonsäure und Derivate.**

(30) Priorität: **21.11.86 US 933702**

(43) Veröffentlichungstag der Anmeldung:
**27.07.88 Patentblatt 88/30**

(45) Bekanntmachung des Hinweises auf die Patenterteilung:
**06.02.91 Patentblatt 91/06**

(84) Benannte Vertragsstaaten:
**AT BE CH DE ES FR GB GR IT LI LU NL SE**

(56) Entgegenhaltungen:
**EP-A-0 203 891**
**GB-A-2 104 079**

(73) Patentinhaber: **CIBA-GEIGY AG**
**Klybeckstrasse 141**
**CH-4002 Basel (CH)**

(72) Erfinder: **Hutchison, Alan J.**
**35 Lynwood Road**
**Verona New Jersey 07044 (US)**
Erfinder: **Shaw, Kenneth R.**
**176 Millburn Avenue**
**Millburn New Jersey 07041 (US)**
Erfinder: **Schneider, Josef A.**
**99 Main Street**
**Millburn New Jersey 07041 (US)**

**EP 0 275 820 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung bezieht sich auf Verbindungen der Formel I,

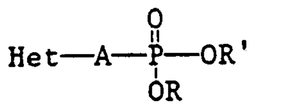

(I)

worin Het eine 2-$R^1$-Pyrrolidinyl-, 2-$R^1$-2-5-Dihydropyrrolyl-, 2-$R^1$-1,2,3,6-Tetrahydropyridinyl-, 2-$R^1$-1,2,5,6-Tetrahydropyridinyl-, 2-$R^1$-Piperidinyl-, 2-$R^1$-Tetrahydrochinolinyl-, 2-$R^1$-Perhydrochinolinyl-, 2-$R^1$-2,3-Dihydroindolyl- oder 2-$R^1$-Perhydroindolylgruppe bedeutet, welche auch durch $C_1$—$C_4$-Alkyl, Phenyl-$C_1$—$C_4$-alkyl, $C_2$—$C_7$-Alkoxycarbonyl, Benzyloxycarbonyl oder $C_2$—$C_7$-Alkanoyl N-substituiert und/oder im heterocyclischen 5- oder 6-Ring durch $C_1$—$C_4$-Alkyl oder Phenyl-$C_1$—$C_4$-alkyl bzw. im gegebenenfalls vorhandenen ankondensierten Benzo- oder Cyclohexanorest durch $C_1$—$C_4$-Alkyl, $C_1$—$C_4$-Alkoxy, Halogen oder Trifluormethyl C-substituiert sein kann, $R^1$ Carboxy, $C_1$—$C_4$-Alkoxycarbonyl, Carbamyl oder N-Mono oder N,N-Di-$C_1$—$C_4$-alkylcarbamyl bedeutet, A $C_2$—$C_4$-Alkenylen bedeutet und R und R′ unabhängig voneinander Wasserstoff, $C_1$—$C_4$-Alkyl, Benzyl, im Phenylteil durch Halogen, $C_1$—$C_4$-Alkyl oder $C_1$—$C_4$-Alkoxy substituiertes Benzyl, $C_2$—$C_7$-Alkanoyloxymethyl oder im Oxymethylteil durch $C_1$—$C_4$-Alkyl oder $C_3$—$C_8$-Cycloalkyl substituiertes $C_2$—$C_7$-Alkanoyloxymethyl darstellen; und ihres Salze. Diese Verbindungen eignen sich als Antagonisten des N-Methyl-D-aspartatsensiblen Aminosäureizrezeptors bei Säugetieren.

Die vorliegende Erfindung betrifft ferner Verfahren zur Herstellung benannter Verbindungen, pharmazeutische Präparate, die benannte Verbindungen enthalten, bzw. Verfahren zur Herstellung solcher pharmazeutischer Präparate sowie die Verwendung der erfindungsgemässen Verbindungen zur Herstellung von für die Behandlung von Zuständen und Erkrankungen bei Säugetieren, die auf die Einwirkung eines Aminosäurereizrezeptorantagonisten ansprechen, bestimmter Arzneimitteln.

Die erfindungsgemässen Verbindungen sind wirksam und brauchbar bei Säugetieren als selektive Antagonisten des N-Methyl-D-aspartatsensiblen (NMDA) Aminosäurereizrezeptors. Die erfindungsgemässen Verbindungen eignen sich daher auch, allein oder kombiniert Säugern verabreicht, für die Behandlung von Erkrankungen, die auf eine Blockade des NMDA-Rezeptors ansprechen, beispielsweise cerebrale Ischaemie, Muskelspasmen (Spastizität), konvulsive Erkrankungen (Epilepsie) und Angstgefühle. Die erfindungsgemässen Verbindungen werden auch als wirksam angsehen bei der Behandlung der Huntingtonschen Krankheit (Chorea hereditiva).

Eine Kondensation ausgehend von benachbarten Kohlenstoffatomen mit einem carbocyclischen Sechsring ist eine Kondensation mit beispielsweise Cyclohexyl oder Phenyl derart, dass der kondensierte heterocyclische Ring in Formel I dargestellt wird durch ein bicyclisches Ringsystem mit 9 oder 10 ringbildenden Atomen, je nach dem Wert von m in Formel I.

Eine bevorzugte Ausführungsart der Erfindung bezieht sich auf Verbindungen der Formel II,

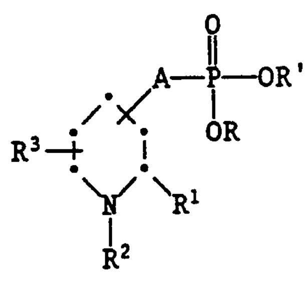

(II),

und Verbindungen der Formel II mit einer Doppelbindung zwischen C-3 und C-4 oder zwischen C-4 und C-5 des Piperidinylringes worin R und R′ unabhängig voneinander Wasserstoff, $C_1$—$C_4$-Alkyl, Benzyl, im Phenylteil durch Halogen, $C_1$—$C_4$-Alkyl oder $C_1$—$C_4$-Alkoxy substituiertes Benzyl, $C_2$—$C_7$-Alkanoyloxymethyl oder im Oxymethylteil durch $C_1$—$C_4$-Alkyl oder $C_3$—$C_8$-Cycloalkyl substituiertes $C_2$—$C_7$-Alkanoyloxymethyl darstellen, $R^1$ Carboxy, $C_1$—$C_4$-Alkoxycarbonyl, Carbamyl oder N-Mono- oder N,N-Di-$C_1$—$C_4$-alkylcarbamyl bedeutet, $R^2$ Wasserstoff, $C_1$—$C_4$-Alkyl, Phenyl-$C_1$—$C_4$-alkyl, $C_2$—$C_7$-Alkoxycarbonyl, Benzyloxycarbonyl oder $C_2$—$C_7$-Alkanoyl darstellt, $R^3$ Wasserstoff, $C_1$—$C_4$-Alkyl oder Phenyl-$C_1$—$C_4$-alkyl bedeutet und A $C_2$—$C_4$-Alkenylen bedeutet; und ihre Salze.

Bevorzugt sind die Verbindungen der Formel II, worin R und R′ unabhängig voneinander Wasserstoff, $C_1$—$C_4$-Alkyl, Benzyl, $C_2$—$C_7$-Alkanoyloxymethyl oder $C_2$—$C_7$-Alkanoyloxymethyl, welches am Oxymethylteil durch $C_1$—$C_4$-Alkyl, Cyclohexyl oder Cyclopentyl substituiert ist, bedeuten, $R^1$ Carboxy, Carbamoyl oder $C_1$—$C_4$-Alkoxycarbonyl bedeutet; $R^2$ und $R^3$ Wasserstoff oder $C_1$—$C_4$-Alkyl bedeuten und A Alkenylen mit 2 bis 4 Kohlenstoffatomen bedeutet; sowie pharmazeutisch annehmbare Salze davon.

Weiter bevorzugt sind die Verbindungen der Formel II, worin R und R′ unabhängig voneinander Wasserstoff, $C_2$—$C_7$-Alkanoyloxymethyl oder $C_2$—$C_7$-Alkanoyloxymethyl, welches am Oxymethylteil durch $C_1$—$C_4$-Alkyl substituiert ist, bedeuten, $R^1$ Carboxy, Carbamoyl oder $C_1$—$C_4$-Alkoxycarbonyl bedeutet, $R^2$ und $R^3$ Wasserstoff bezeichnen, A sich in 4-Position befindet und Alkenylen mit 3 oder 4 Kohlenstoffatomen

und der Doppelbindung benachbart zur Phosphonogruppe bedeutet; sowie pharmazeutisch annehmbare Salze davon.

Insbesondere bevorzugt sind die Verbindungen der Formel III

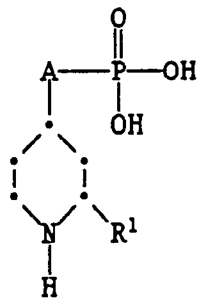

(III),

worin A 1,3-Propenylen bedeutet, vorzugsweise mit der Doppelbindung benachbart zur Phosphonogruppe; $R^1$ Carboxy oder $C_1$—$C_4$-Alkoxycarbonyl bedeutet; sowie pharmazeutisch annehmbare Salze davon.

Eine bevorzugte Ausführungsart bezieht sich auf die Verbindungen der Formel III, worin die 2- und 4-Substituenten zueinander in cis-Stellung stehen.

Ein weiterer Gegenstand der Erfindung sind Verbindungen der Formel IV

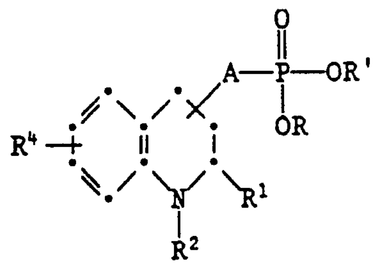

(IV)

oder Perhydroderivate davon, worin R und R' unabhängig voneinander Wasserstoff, $C_1$—$C_4$-Alkyl, Benzyl oder Benzyl, welches am Phenylteil durch Halogen, $C_1$—$C_4$-Alkyl oder $C_1$—$C_4$-Alkoxy substituiert ist, $C_2$—$C_7$-Alkanoyloxymethyl oder $C_2$—$C_7$-Alkanoyloxymethyl, welches am Oxymethylteil durch $C_1$—$C_4$-Alkyl oder $C_3$—$C_8$-Cycloalkyl substituiert ist, bedeutet, $R^1$ Carboxy $C_1$—$C_4$-Alkoxycarbonyl, Carbamyl oder N-Mono- oder N,N'-Di-$C_1$—$C_4$-Alkylcarbamyl bedeutet, $R^2$ Wasserstoff, $C_1$—$C_4$-Alkyl, Phenyl-$C_1$—$C_4$-alkyl, $C_1$—$C_7$-Alkoxycarbonyl, Benzyloxycarbonyl oder $C_2$—$C_7$-Alkanoyl bedeutet; $R^4$ Wasserstoff, $C_1$—$C_4$-Alkyl, $C_1$—$C_4$-Alkoxy, Halogen oder Trifluormethyl bedeutet und A für $C_2$—$C_4$-Alkenylen steht; sowie Salze davon.

Bevorzugt sind die Verbindungen der Formel V

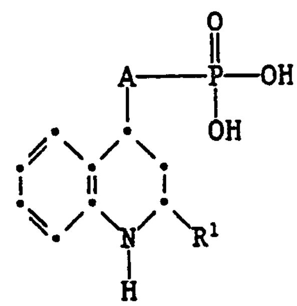

(V)

oder Perhydrochinolin-Derivate davon, worin A 1,3-Propenylen mit der Doppelbindung benachbart zur Phosphonogruppe darstellt und $R^1$ Carboxy oder $C_1$—$C_4$-Alkoxycarbonyl bedeutet; sowie pharmazeutisch annehmbare Salze dieser Verbindungen, die eine salzbildende funktionelle Gruppe aufweisen.

Am meisten bevorzugt sind die Verbindungen der Formel V, worin die 2- und 4-Substituenten in cis-Stellung zueinander stehen.

Ein weiterer Gegenstand der Erfindung sind Verbindungen der Formel VI

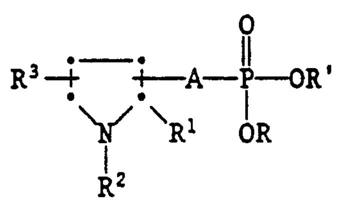

(VI)

und die Verbindungen der Formel VI mit einer Doppelbindung zwischen C-3 und C-4 des Pyrrolidinylringes, worin R und R' unabhängig voneinander Wasserstoff, $C_1$—$C_4$-Alkyl, Benzyl, Benzyl, welches am Phenylteil durch Halogen, $C_1$—$C_4$-Alkyl oder $C_1$—$C_4$-Alkoxy substituiert ist, $C_2$—$C_7$-Alkanoyloxymethyl oder $C_2$—$C_7$-Alkanoyloxymethyl, welches am Oxymethylteil durch $C_1$—$C_4$-Alkyl oder $C_3$—$C_8$-Cycloalkyl substituiert ist, bedeuten, $R^1$ Carboxy $C_1$—$C_4$-Alkoxycarbonyl, Carbamyl oder N-Mono- oder N,N-Di-$C_1$—$C_4$-alkylcarbamyl bedeutet, $R^2$ Wasserstoff, $C_1$—$C_4$-Alkyl, $C_1$—$C_7$-Alkoxycarbonyl, Benzyloxycarbonyl oder $C_2$—$C_7$-Alkanoyl bedeutet, $R^3$ Wasserstoff, $C_1$—$C_4$-Alkyl oder Aryl-$C_1$—$C_4$-alkyl bedeutet und A für $C_2$—$C_4$-Alkenylen steht; sowie Salze davon.

Bevorzugt sind die Verbindungen der Formel VI, worin die Phosphonotragende Gruppe in der Stellung 3 angelagert ist; R und R' Wasserstoff bedeuten, $R^1$ Carboxy oder $C_1$—$C_4$-Alkoxycarbonyl bedeutet, $R^2$ und $R^3$ Wasserstoff bedeuten und A für 1,3-Propenylen mit der Doppelbindung benachbart zur Phosphono-Gruppe bedeutet; sowie pharmazeutisch annehmbare Salze davon.

Die allgemeinen hier verwendeten Definitionen haben im Zusammenhang mit der Erfindung folgende Bedeutung:

Der Ausdruck "nieder", der vor- und nachstehend im Zusammenhang mit organischen Gruppen, Radikalen oder Verbindungen verwendet wird, definiert solche mit bis zu und einschliesslich 7 Kohlenstoffatomen, vorzugsweise solche mit bis zu und einschliesslich 4 und vorteilhafterweise solche mit 1, 2 oder 3 Kohlenstoffatomen.

$C_1$—$C_4$-Alkyl ist beispielsweise Ethyl, Propyl, Butyl oder vorteilhafterweise Methyl.

$C_2$—$C_4$-Alkenylen in der Bedeutung A bezeichnet z.B. Ethenylen, 1,3-Propenylen, 1-4-But-1-enylen, oder 1,4-But-2-enylen, vorzugsweise mit der Doppelbindung benachbart zur Phosphonogruppe.

$C_1$—$C_4$-Alkoxy bezeichnet beispielsweise Ethoxy, Propoxy oder vorteilhafterweise Methoxy.

$C_2$—$C_7$-Alkanoyl bezeichnet vorteilhafterweise Acetyl, Propionyl, n-Butyryl, Isobutyryl oder Pivaloyl, kann jedoch auch Formyl sein.

$C_2$—$C_7$-Alkanoyloxy bezeichnet vorteilhafterweise Acetoxy, Propionyloxy, n- oder i-Butyryloxy oder Pivaloyloxy.

$C_3$—$C_8$-Cycloyalkyl bezeichnet beispielsweise Cyclohexyl oder Cyclopentyl.

Halogen ist vorzugsweise Fluor und Chlor, kann jedoch auch Brom oder lod darstellen.

Phenyl-$C_1$—$C_4$-Alkyl bezeichnet vorzugsweise Benzyl oder 2-Phenylethyl.

$C_1$—$C_7$-Alkoxycarbonyl enthält vorzugsweise 1—4 Kohlenstoffatome im Alkoxy-Teil und bezeichnet beispielsweise Methoxycarbonyl, Propoxycarbonyl, Isopropoxycarbonyl oder vorteilhafterweise Ethoxycarbonyl.

N-$C_1$—$C_4$-Alkylcarbamyl ist beispielsweise N-Methylcarbamoyl, N-Propylcarbamoyl oder vorteilhafterweise N-Ethylcarbamoyl.

N,N-Di-$C_1$—$C_4$-Alkylcarbamyl bezeichnet z.B. N,N-Dimethylcarbamoyl, N-Ethyl-N-methylcarbamoyl und vorteilhafterweise N,N-Diethylcarbamoyl.

Salze der erfindungsgemässen Verbindungen sind vorzugsweise pharmazeutisch annehmbare Salze, einerseits Metall- oder Ammoniumsalze der erfindungemässen Verbindungen mit einer freien Phosphon- oder Carboxy-Gruppe, insbesondere Alkali- oder Erdalkalimetallsalze, z.B. Natrium-, Kalium-, Magnesium- oder Calciumsalze; oder vorteilhafterweise kristallisierende Ammoniumsalze, abgeleitet von Ammoniak oder organischen Aminen, wie beispielsweise Methylamin, Diethylamin, Triethylamin, Dicyclohexylamin, Triethanolamin, Ethylendiamin, tris-(Hydroxymethyl)aminomethan oder Benzyltrimethylammoniumhydroxid. Andererseits bilden die erfindungsgemässen Verbindungen, die basische Amine sind, Säureadditionssalze mit vorzugsweise pharmazeutisch annehmbaren anorganischen oder organischen Säuren, wie mit starken Mineralsäuren, wie beispielsweise Halogenwasserstoffsäuren, z.B. Chlorwasserstoffsäure oder Bromwasserstoffsäure; Schwefel, Phosphor- oder Salpetersäure; aliphatischen oder aromatischen Carbon- oder Sulfonsäuren, z.B. Essigsäure, Propionsäure, Bernsteinsäure, Glycolsäure, Milchsäure, Aepfelsäure, Weinsäure, Gluconsäure, Zitronensäure, Ascorbinsäure, Maleinsäure, Fumarsäure, Brenztraubensäure, Pamoasäure, Nicotinsäure, Methansulfonsäure, Ethansulfonsäure, Hydroxyethansulfonsäure, Benzolsulfonsäure, p-Toluolsulfon- oder Naphthalinsulfonsäure.

Zu Isolier- oder Reinigungszwecken können Salze erhalten werden, die sich für pharmazeutische Zwecke nicht eignen. Es werden jedoch für therapeutische Zwecke lediglich pharmazeutisch annehmbare Salze verwendet, diese Salze sind daher bevorzugt.

Die erfindungsgemässen Verbindungen zeigen wertvolle pharmakologische Eigenschaften, z.B. eine selektive Blockierung der N-Methyl-D-aspartatsensiblen Aminosäurereizrezeptoren bei Säugetieren. Die Verbindungen eignen sich daher zur Behandlung von Erkrankungen, die auf eine Aminosäurereizblockade bei Säugetieren ansprechen, also beispielsweise Erkrankungen des Nervensystems, insbesondere krampfartige Erkrankungen (Epilepsie) und Angstzustände.

Diese Wirkungen lassen sich in in vitro-Versuchen oder in vivo-Tierversuchen demonstrieren, wobei vorteilhafterweise Säugetiere oder Gewebe- oder Enzymzubereitungen derselben verwendet werden, z.B. Mäuse, Ratten oder Affen. Benannte Verbindungen können enteral oder parenteral verabreicht werden, vorteilhafterweise oral oder transdermal, oder aber subcutan, intravenös oder intraperitoneal, beispielsweise in Gelatinekapseln oder in Form wässriger Suspensionen oder Lösungen. Die verabreichtet in-vivo-Dosis kann ungefähr zwischen 0.01 und 100 mg/kg liegen, vorzugsweise zwischen ungefähr 0,05

und 50 mg/kg und vorteilhafterweise ungefähr zwischen 0,1 und 10 mg/kg. Benannte Verbindungen können in vitro verabreicht werden in form beispielsweise wässriger Lösungen, wobei die Dosis ungefähr zwischen $10^{-4}$ und $10^{-8}$ Molkonzentrationen liegt.

Die Inhibitorwirkung auf die Aminosäurereizrezeptoren vom NMDA-Typ wird in vitro bestimmt durch Messen der Inhibition der NMDA-ausgelösten $^3$H-Acetylcholin ($^3$H-ACh)-Freisetzung aus corpus striatum-Gewebe des Rattengehirns nach J. Lehmann und B. Scatton, Brain Research *252,* 77—89 (1982) und Nature 297, 422—424 (1982).

Antagonisten vom Aminosäurereizrezeptoren vom NMDA-Typ wirken der NMDA-ausgelösten $^3$H-Acetylcholin ($^3$H-ACh)-Freisetzung aus corpus striatum-Gewebe des Gehirns kompetitiv entgegen.

Die Inhibition der NMDA-ausgelösten $^3$H-Acetylcholin ($^3$H-ACh)-Freisetzung aus Striatumgewebescheiben von Ratten durch eine erfindungsgemässe Verbindung wird ausgedrückt als Prozentzahl der Freisetzung von $^3$H-ACh als Reaktion auf einen Reiz mit 50 µM NMDA im Vergleich zum Kontrollversuch. Die Versuche sind zweischwänzig mit einem Minimum von n = 4 in jeder Gruppe. Die $IC_{50}$-Werte bezeichnen die Konzentration der Testverbindung, die erforderlich ist, um die durch NMDA erhöhte $^3$H-ACh-Freisetzung um 50% zu inhibieren.

Die Inhibitionswirkung der Aminosäurereizrezeptoren vom NMDA-Typ wird in vivo demonstriert durch die Inhibierung von NMDA-ausgelösten Krämpfen bei Mäusen.

Repräsentative Verbindungen der Erfindung verhindern NMDA-ausgelöste Krämpfe bei Mäusen bei Dosierungen bis herunter zu ungefähr 1,2 mg/kg i.p.

Ein weiteres Anzeichen für die antikonvulsive Aktivität besteht darin, dass die erfindungemässen Verbindungen wirksam audiogen ausgelöste Anfälle bei DBA/2-Mäusen verhindern [Chapman et al., Arzneim.-Forsch. *34*; 1261, (1984)].

Aus der GB—A—2104079 waren 2-Amino-ω-phosphono-alkensäuren und funktionelle Derivate derselben mit NMDA-antagonistischen Eigenschaften vorbekannt. Die erfindungsgemäss bereitgestellten Verbindungen unterscheiden sich von diesen durch das Strukturmerkmal der heterocyclischen Gruppe Het anstelle der Gruppe

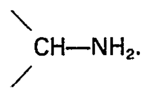

Die Wirkung wird folgendermassen bestimmt: 45 Minuten nach der Verabreichung der Verbindung oder des Trägers kommen die Mäuse einzeln in eine schalldichte Kammer. Nach einer Akkomodationszeit von 30 s werden die Mäuse 1 Minute lang einer Schallreizung von 110 dB ausgesetzt, oder aber solange, bis ein tonisch-clonischer Anfall auftritt. Kontrollanfälle bestehen aus einer Anfangsphase wilden Laufens. Die Verhinderung des wilden Laufens ist ein Anzeichen für eine antikonvulsive Wirkung.

Prüfverbindungen werden entweder als Lösung in destilliertem Wasser oder als Suspension in einer 3-%igen kolloidalen Maisstärke mit 5 Masse-% Polyethylenglycol 400 und 0,34% Tween 80 oral oder intraperitoneal in einer Menge von 10 ml/kg Körpergewicht verabreicht.

Ein Hinweis auf anxiolytische Aktivität ist der Umstand, dass die erfindungsgemässen Verbindungen in dem Cook/Davidson-Konfliktmodell [Psychopharmacologia *15,* 159—168 (1969)] wirksam sind.

Die cerebral antiischaemische Aktivität, das heisst der Effekt der erfindungsgemässen Verbindungen, Gehirnschädigungen bei Säugern als Folge einer vorübergehenden cerebralen Ischaemie (wie bei einem Schlaganfall) zu verbindern oder zu reduzieren, kann anhand des mongolischen Gerbil-Ischaemie-Modells bestimmt werden, z.B. des Modells, das von T. Kirino in Brain Research 239, 57—69 (1982) beschrieben wird.

Gemessen wird der inhibierende Effekt auf die beobachtete Hyperaktivität und auf die Degeneration von Neuronen in der Hippocampus-Region des Gehirns im Anschluss an eine fünfminütige Ischaemie. Die Testverbindung wird i.p. 15 Minuten vor der Ischaemie oder 2, 4 und 6 Stunden nach der Ischaemie gegeben.

Repräsentative Verbindungen der Erfindung, die vor oder nach der Ischaemie-Episode in einer Dosis von 10 mg/kg i.p. verabreicht wurden, inhibieren die Ischaemie-induzierte Hyperaktivität des Gerbil und vermindern die Degenierung cerebraler Neuronen, wie sich in der Hippocampus-Region des Gehirns messen lässt.

Die oben erwähnten vorteilhaften Eigenschaften lassen die erfindungsgemässen Verbindungen brauchbar erscheinen als Antagonisten des N-Methyl-D-aspartatsensiblen Aminosäurereizrezeptors bei Säugetieren und für die Behandlung der diesbezüglichen Zustände, wie beispielsweise Angstzustände und Krampferkrankungen.

Die erfindungsgemässen Verbindungen, d.h. die oben aufgeführten, lassen sich durch konventionelle Verfahren herstellen, z.B. durch

a) Kondensation eines Aldehydes oder Ketons der Formel VII

$$Het{-}A' \qquad (VII)$$

worin Het und $R^1$ wie für Formel I definiert sind, wobei aber $R^1$ und Aminogruppen in geschützter Form

vorliegen, und A' $C_1$—$C_3$-Alkyl bedeutet, welches durch Oxo substituiert ist und ein Kohlenstoffatom weniger aufweist als die Alkenylgruppe A, mit einem Tetraesterderivat von Methylendiphosphonsäure unter basischen Bedingungen und erforderlichenfalls durch Abspalten von Schutzgruppen von dem erhaltenen Produkt, so dass eine Verbindung der Formel I erhalten wird, worin die Doppelbindung innerhalb der Gruppe A der Phosphongruppe unmittelbar benachbart ist; oder

b) Kondensation einer Verbindung der Formel VIII,

Het—A—X (VIII)

worin Het, A und $R^1$ wie für Formel I definiert sind und X reaktionsfähiges verestertes Hydroxy darstellt, mit einer Verbindung, die die Phosphonsäurehälfte einführen kann, und die eine der Formeln IX oder X

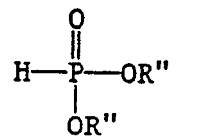

(IX) $P(R''')_3$ (X)

aufweist, worin R'' $C_1$—$C_4$-Alkyl bezeichnet und R''' Halogen oder $C_1$—$C_4$-Alkoxy ist, und erforderlichenfalls Umwandlung des entstehenden Phosphonsäurederivats in die Phosphonsäure oder ein anderes Esterderivat derselben; oder

c) Umwandlung in $R^1$ eines anderen Substituenten als $R^1$ in Stellung 2 am heterocyclischen Ring in einer Verbindung, die sonst identisch mit einer erfindungsgemässen ist; ferner durch Ausführung dieser Verfahren mit erforderlichenfalls vorübergehendem Schutz aller störenden reaktionsfähigen Gruppen und anschliessender Freisetzung der entstehenden erfindungsgemässen Verbindung; gewünschtenfalls Umwandlung einer entstehenden erfindungsgemässen Verbindung in eine andere erfindungsgemässe Verbindung und/oder gewünschtenfalls Umwandlung der entstehenden freien Verbindung in ein Salz oder eines entstehenden Salzes in die freie Verbindung oder ein anderes Salz; und/oder Trennen eines Isomerengemisches oder Gemisches von Racematen in die einzelnen Isomere oder Racemate; und/oder gewünschtenfalls Trennung eines Racemats in die optischen Antipoden.

Reaktionsfähiges verestertes Hydroxy in jedem der hier aufgeführten Verfahren, z.B. X in einer Verbindung der Formel VIII, ist durch eine starke Säure verestert, insbesondere eine Halogenwasserstoffsäure, beispielsweise Chlorwasserstoffsäure, Bromwasserstoff oder Iodwasserstoffsäure oder Schwefelsäure oder eine starke organische Säure, insbesondere eine starke organische Sulfonsäure, wie beispielsweise eine aliphatische oder aromatische Sulfonsäure, z.B. Methansulfonsäure, 4-Methylphenylsulfonsäure oder 4-Bromphenylsulfonsäure. Besagtes reaktionsfähiges verestertes Hydroxy ist insbesondere Halogen-, z.B. Chlor-, Brom- oder Iod-, oder aliphatisch oder aromatisch substituiertes Sulfonyloxy, z.B. Methansulfonyloxy, Phenylsulfonyloxy oder 4-Methylphenylsulfonyloxy (Tosyloxy).

In den Ausgangsverbindungen und Zwischenprodukten, die in die erfindungsgemässen Verbindungen in der hier beschriebenen Weise umgewandelt werden, werden daher anwesende funktionelle Gruppen wie Carboxy, Amino (einschliesslich Ring-NH) und Hydroxy-Gruppen gewünschtenfalls durch konventionelle Schutzgruppen geschützt, wie sie in der präparativen organischen Chemie üblich sind. Geschützte Carboxy-, Amino und Hydroxy-Gruppen sind solche, die unter milden Bedingungen in freie Carboxy-, Amino- und Hydroxy-Gruppen umgewandelt werden können, ohne dass das Molekülskelett zerstört wird oder dass andere unerwünschte Nebenreaktionen ablaufen.

Der Zweck der Einführung von Schutzgruppen besteht im Schutz der funktionellen Gruppen gegen unerwünschte Reaktionen mit den Reaktionskomponenten und unter den Bedingungen, unter denen eine gewünschte chemische Umwandlung ausgeführt wird. Die Notwendigkeit für Schutzgruppen und die Wahl derselben für eine spezielle Reaktion ist den Fachleuten bekannt und hängt von der Beschaffenheit der zu schützenden funktionellen Gruppen ab (Carboxy-Gruppe, Amino-Gruppe usw.), weiter von Struktur und Stabilität des Moleküls, dessen Bestandteil der Substituent ist, und von den Reaktionsbedindungen.

Allgemein bekannte Schutzgruppen, die diesen Bedingungen entsprechen, sowie deren Einführung und Entfernung werden beispielsweise beschrieben in J. F. W. McOmie, "Protective Groups in Organic Chemistry", Plenum Press, London, New York 1973, T. W. Greene, "Protective Groups in Organic Synthesis", Wiley, New York 1981 und auch in "The Peptides", Vol. I, Schroeder und Leubke, Academic Press, London, New York 1965 sowie in Houben-Weyl, "Methoden der Organischen Chemie", Vol. 15/1, Georg Thieme Verlag, Stuttgart, 1974.

Die Herstellung der erfindungsgemässen Verbindungen, in denen die Doppelbindungen der Phosphongruppe benachbart ist gemäss Verfahren (a), gemäss dem ein Aldehyd oder Keton mit beispielsweise einem Niederalkylester von Methylendiphosphonsäure kondensiert wird, wird gemäss Verfahren ausgeführt, die dem Fachmann für solche Kondensationen bekannt sind, in Gegenwart einer starken wasserfreien Base, z.B. Butyllithium, in einem inerten polaren Lösungsmittel, wie Tetrahydrofuran, vorzugsweise unter Erhitzen unter Rückfluss. Die Ausgangsaldehyde oder -ketone der Formel VII können z.B. durch Oxidation der entsprechenden Alkohole hergestellt werden, beispielsweise mit Pyridiniumchlorochromat, oder gemäss anderen Verfahren, wie sie z.B. in den Beispielen illustriert werden.

Die jeweiligen Alkohole können ihrerseits auf an sich bekannte Weise hergestellt werden, z.B. durch Reduktion der entsprechenden aromatischen Alkohole unter Anwendung von Verfahren, die der Fachwelt für die Reduktion von Pyrrol-, Pyridin-, Indol- und Chinolinverbindungen bekannt sind. Beispielsweise wird die Reduktion des Pyridin- oder Chinolinriges vorzugsweise mit einem organometallischen Reduktionsmittel oder durch katalytische Hydrierung durchgeführt, z.B. in Gegenwart von Platinoxid und eines sauren Lösungsmittels, wie Essigsäure, so dass die entsprechenden Tetrahydropyridine, Piperidine, 1,2,3,4-Tetrahydrochinoline oder Perhydrochinoline gemäss der Erfindung erhalten werden, z.B. gemäss Formel II oder IV sowie Derivate davon. Quartäre Chinolinium- und Pyridinium-Verbindungen, z.B. solche, in denen $R^2$ Niederalkyl oder Aryl-Niederalkyl bedeutet, können auf ähnliche Weise reduziert werden.

Die auf diese Weise erhaltenen Alkohole, Aldehyde oder Ketone können auch in Aldehyde oder Ketone grösserer Kettenlänge übergeführt werden, wobei konventionelle Verfahren verwendet werden können, z.B. durch eine Wittig-Kondensation eines Aldehyds mit Methoxy-methyl-triphenylphosphoniumchlorid, wobei der homologe Aldehyd erhalten wird, und durch andere an sich bekannte Reaktionssequenzen, wie sie z.B. in den Beispielen beschrieben werden.

Die vorstehend erwähnten aromatischen 2-Carboxy-heterocyclyl-substituierten Niederalkanole, beispielsweise die 2-Carboxy-pyridinyl-oder 2-Carboxy-chinolinyl-substituierten Niederalkanole oder Derivate davon können ihrerseits hergestellt werden, indem die 2-unsubstituerten Pyridinyl- oder 2-unsubstituierten Chinolinyl-substituierten Niederalkanole, in geeigneterweise geschützter Form, mit beispielsweise einer Persäure, wie m-Chlorperbenzoesäure, behandelt werden, so dass man die entsprechenden Pyridin-N-oxide oder Chinolin-N-oxide erhält. Kondensation mit einem reaktionsfähigen Cyanid, z.B. einem Trialkylsilylcyanid wie Trimethylsilylcyanid, vorzugsweise unter basischen Bedingungen, z.B. in der Gegenwart von Triethylamin, ergibt die entsprechenden 2-Cyanpyridin- oder 2-Cyanchinolin-Derivate, die sodann, wiederum auf an sich bekannte Weise, in die 2-$R^1$-(Carboxy, verestertes oder amidiertes Carboxy)-substituierten Pyridin- und Chinolin-Derivate übergeführt werden.

Die Kondensation gemäss Verfahren (b) wird vorteilhafterweise für die Herstellung von Verbindungen der Formel I angewandet, in denen die Doppelbindung innerhalb der Alkenylengruppe A nicht unmittelbar benachbart zur Phosphonogruppe vorliegt.

Die Kondensation gemäss Verfahren b) einer Verbindung der Formel VIII mit einer Verbindung der Formel X, z.B. Triethylphosphit, wird durchgeführt beispielsweise durch Erwärmen in einem inerten Lösungsmittel und unter den Bindingungen, die für eine Michaelis-Arbuzov-Reaktion nach Angew. Chem. Int. Ed. *16*, 477 (1977) und Chem. Rev. *81*, 415 (1981) bekannt sind. Auf ähnliche Weise liefert eine Kondensation z.B. mit Phosphortrichlorid und eine anschliessende Hydrolyse eine Verbindung der Formel I.

Die Kondensation einer Verbindung von Formel VIII nach Verfahren b) mit einer Verbindung von Formel IX, z.B. Diethylphosphonat (Diethylphosphit), erfolgt beispielsweise in einem stark basischen Medium, z.B. in Anwesenheit eines Alkalimetalles, wie Natrium, eines Alkalimetallhydrids wie Natriumhydrid, eines Alkalimetallalkoxids wie Kalium-t-Butoxid, in einem inerten Lösungsmittel wie Toluol oder Dimethylformamid.

Ausgangsverbindungen der Formel VIII und reaktionsfähige Derivate davon können auf an sich bekannte Weise hergestellt werden, wobei man von Zwischenprodukten der Formel VII ausgeht, worin A' durch Oxo substituiertes Niederalkyl bedeutet, deren Herstellung vorstehend beschrieben ist.

Beispielsweise wird das Ausgangsmaterial der Formel VIII, worin A Propenylen bedeutet, durch Kondensation eines Aldehydes der Formel VIII, worin die Gruppe A' für Formyl (CH=O) steht, mit einem geeigneten Wittig-Reagens, z.B. Formylmethylen-triphenylphosphoran, hergestellt. Der erhaltene ungesättigte Aldehyd (in dem die Kette um zwei Kohlenstoffatome verlängert worden ist) wird zum Alkohol reduziert, z.B. mit Natriumborhydrid, und der erhaltene ungesättigte Alkohol wird in ein reaktionsfähiges Derivat übergeführt, z.B. in das Bromid mit Triphenylphosphin/N-Bromsuccinimid.

Umwandlungen nach Verfahren c) werden nach allgemein bekannten Methoden durchgeführt.

Gruppen, die sich in $R^1$ unwandeln lassen, sind beispielsweise Carboxy-Gruppen in Form von Anhydriden oder Säurehalogeniden, Cyan-, Amidino-Gruppen, einschliesslich cyclischer Amidinogruppen, wie 5-Tetrazolyl, Iminoether-Gruppen, einschliesslich cyclischer Iminoether-Gruppen, beispielsweise Dihydro-2-oxazolinyl- oder Dihydro-2-oxazolinyl-Gruppen, substituiert durch Niederalkyl, und auch Hydroxymethyl, verethertes Hydroxymethyl, Niederalkanoyloxymethyl, Trialkoxymethyl, Acetyl, Trihaloacetyl, Halomethyl, Carboxycarbonyl (COCOOH), Formyl (CHO), di-(Nieder)alkoxymethyl, Alkylendioxymethyl oder Vinyl.

Einige bei den Verfahren verwendete Ausdrücke haben die nachfolgend festgelegten Bedeutungen.

Durch Oxo substituiertes Niederalkyl bedeutet vorzugsweise Formyl, Formylmethyl, Formylethyl oder 2-Oxo-propyl.

Trialkoxymethyl bezeichnet vorzugsweise tri-(Niederalkoxy)-Methyl, insbesondere Triethoxy- oder Trimethoxymethyl.

Verethertes Hydroxymethyl bezeichnet vorzugsweise Niederalkoxymethyl, Niederalkoxyalkoxymethyl, z.B. Methoxymethoxymethyl oder 2-Oxa- oder 2-Thiacyclo-Alkoxymethyl, insbesondere 2-Tetrahydropyranyloxymethyl.

Halomethyl bezeichnet insbesondere Chlormethyl, kann jedoch auch Brommethyl oder Iodmethyl sein.

Ein Alkalimetall bezeichnet vorzugsweise Lithium, kann jedoch auch Kalium oder Natrium sein.

Gruppen, die in $R^1$ mit der Bedeutung Carboxy umwandelbar sind, umfassen verestertes und amidiertes Carboxy und sind nicht auf verestertes und amidiertes Carboxy beschränkt, wie dies hierin für $R^1$ festgelegt worden ist. Eine Umwandlung in Carboxy wird normalerweise durch Solvolyse mit einer Säure oder Base durchgeführt.

Benzyloxycarbonyl oder Nitrobenzyloxycarbonyl können auch durch katalytische Hydrierung in Carboxy umgewandelt werden, die letztere Gruppe auch mit Hilfe chemischer Reduktionsmittel, z.B. mit Natriumdithionit oder mit Zink under einer Carbonsäure. Darüberhinaus kann tert-Butyloxycarbonyl auch mit Trifluoressigsäure gespalten werden.

Acetyl lässt sich oxidativ in Carboxy überführen durch Umwandelung zuerst in Trihaloacetyl, z.B. Tribrom- oder Triiodacetyl, durch Behandlung z.B. mit Natriumhypobromit, anschliessend durch Aufspaltung z.B. einer wässrigren Base, beispielsweise Natriumhydroxid.

Formyl, di-(Nieder)alkoxymethyl oder Alkylendioxymethyl (Formyl geschützt in Form eines Acetals), z.B. als Dimethylacetal, werden beispielsweise mit Silbernitrat, Pyridiniumdichromat oder Ozon zu Carboxy oxidiert.

Vinyl lässt sich in Carboxy umwandeln durch Ozonolyse zu Formyl, das wiederum zu Carboxy oxidiert wird.

Eine Hydrolyse von Trialkoxymethyl zu Carboxy erfolgt vorteilhafterweise mit anorganischen Säuren, z.B. Halogenwasserstoff oder Schwefelsäure. Die Hydrolyse von verethertem Hydroxymethyl zu Hydroxymethyl erfolgt am besten mit Lösungen anorganischer Säuren, z.B. einer Halogenwasserstoffsäure. Hydroxymethyl wird wiederum zu Carboxy mit einem Oxidationsmittel oxidiert, z.B. mit Pyridiniumdichromat.

Halomethyl kann auch in die entsprechenden Carboxaldehyde umgewandelt werden, z.B. mit Dimethylsulfoxid in Anwesenheit von Triethylamin und Silbertetrafluoroborat oder mit Chromtrioxid und Pyridin in Dichlormethan.

Die Umwandlung von Cyan in Niederalkoxycarbonyl erfolgt vorteilhafterweise durch Behandlung zuerst mit einem Niederalkanol, z.B. wasserfreiem Ethanol, in Anwesenheit einer starken Säure, z.B. Chlorwasserstoffsäure, vorzugsweise bei Rückflüsstemperatur, mit anschliessender Hydrolyse mit Wasser.

Darüberhinaus wird die Umwandlung von Cyan in Carbamoyl vorzugsweise durchgeführt durch Behandlung mit einem Alkalimetallhydroxid, z.B. verdünnten Natriumhydroxid, und Wasserstoffperoxid, vorzugsweise bei Raumtemperatur.

Verestertes Carboxy, wie Niederalkoxycarbonyl, kann amidiert werden mit Ammoniak, mono- oder di(Nieder)alkylaminen wie Methylamin, Dimethylamin, in einem inerten Lösungsmittel, z.B. einem Niederalkanol, wie Butanol, zu unsubstituiertem, mono- oder di(Nieder)alkylcarbamoyl.

Die erfindungsgemässen Verbindungen lassen sich also in andere erfindungsgemässe Verbindungen umwandeln zum Beispiel durch Umwandlung funktioneller Gruppen wie dies allgemein bekannt ist.

So wird beispielsweise die Umwandlung von Carbonsäureestern und -amiden in Carbonsäuren vorteilhafterweise ausgeführt durch Hydrolyse mit anorganischen Säuren wie beispielsweise einer Halogenwasserstoff- oder Schwefelsäure oder mit wässrigen Alkalien, vorzugsweise Alkalimetallhydroxiden wie Lithium- oder Natriumhydroxid.

Freie Carbonsäuren können verestert werden mit Niederalkanolen, wie Ethanol, in Anwesenheit einer starken Säure, beispielsweise Schwefelsäure, oder mit Diazo(nieder)alkanen, z.B. Diazomethan, in einem Lösungsmittel wie Ethylether, und zwar vorteilhafterweise bei Raumtemperatur, wodurch die entsprechenden Niederalkylester entstehen.

Darüberhinaus lassen sich die freien Carbonsäuren durch Behandlung eines reaktionsfähigen Derivates derselben, z.B. eines Acylhalogenids wie dem Säurechlorid oder eines gemischten Anhydrids, z.B. eines, das aus einem Niederalkylhalogencarbonat, wie Chlorkohlensäureethylester abgeleitet ist, mit Ammoniak, mono- oder di(Nieder)alkylaminen, in einem inerten Lösungsmittel wie Dichlormethan vorzugsweise in Anwesenheit eines basischen Katalysators, wie Pyridin, in Verbindungen umwandeln, bei denen $R^1$ unsubstituiertes, mono- oder di-(Nieder)alkylcarbamoyl bezeichnet.

Phosphonsäureester werden in die entsprechenden Phosphonsäuren umgewandelt durch Behandlung mit einer Säure wie wässriger Chlorwasserstoffsäure oder Bromwasserstoffsäure in Eisessig oder mit Bromtrimethylsilan nach J. Chem. Soc. Chem. Comm. *1979,* 739. Benzylester können durch Hydrogenolyse in die Säuren umgewandelt werden.

Phosphonsäuren werden in Ester umgewandelt, z.B. in gegebenenfalls substituierte Niederalkylester, beispielsweise durch Kondensation mit einem gegebenenfalls substituierten Niederalkylhalogenid vorzugsweise in einem basischen nicht-wässrigen Medium wie beispielsweise in Anwesenheit von Triethylamin.

In einer bevorzugten Ausführungsform der Erfindung wird eine Verbindung der Formel I, worin Het durch $C_2$—$C_7$-Alkanoyl oder $C_2$—$C_7$-Alkoxycarbony N-substituiert ist, R und R′ für $C_1$—$C_4$-Alkyl, Benzyl, Benzyl, welches am Phenylteil durch Halogen, $C_1$—$C_4$-Alkyl oder $C_1$—$C_4$-Alkoxy substituiert ist, $C_2$—$C_7$-Alkanoyloxymethyl oder $C_2$—$C_7$-Alkanoyloxymethyl, welches am Oxymethylteil durch $C_1$—$C_4$-Alkyl oder $C_3$—$C_8$-Cycloalkyl substituiert ist, stehen, und $R^1$ $C_1$—$C_4$-Alkoxycarbonyl, Carbamyl oder N-Mono- oder N,N-Di-$C_1$—$C_4$-alkylcarbonyl bedeutet, durch Behandeln mit einer anorganischen Säure oder mit wässrigen Alkalien in eine entsprechende N-unsubstituierte Verbindung der Formel I überführt, worin R und R′ Wasserstoff bedeuten und $R^1$ Carboxy beduetet, dabei kommen als anorganische Säuren

Halogenwasserstoffsäure oder Schwefelsäure, insbesondere Salzsäure, und als wässrige Alkalien Alkalimetallhydroxiden, insbesondere Lithium- oder Natriumhydroxid, vorzugsweise bei erhöhter Temperatur, in Betracht.

Die vorstehend erwähnten Reaktionen werden nach Standardmethoden durchgeführt, in Anwesenheit oder Abwesenheit von Verdünnungsmitteln, vorzugsweise solchen, die gegenüber den Reaktionspartnern inert und Lösungsmittel derselben sind, weiter von Katalysatoren, von Kondensations- oder benannten anderen Mitteln und/oder in einer inerten Atmosphäre, bei niedrigen Temperaturen, Raumtemperatur oder erhöhten Temperaturen, vorzugsweise am Siedepunkt der verwendeten Lösungsmittel und bei atmosphärischem oder superatmosphärischem Druck. Die bevorzugten Lösungsmittel, Katalysatoren und Reaktionsbedingungen werden in den nachfolgenden veranschaulichenden Beispielen angeführt.

Die Erfindung erstreckt sich weiter auf alle Varianten der vorliegenden Verfahren, in denen ein auf einer beliebigen Stufe derselben erhältliches Zwischenprodukt als Ausgangsmaterial benutzt wird und die restlichen Stufen durchgeführt werden, oder aber bei denen das Verfahren auf einer beliebigen Stufe desselben abgebrochen wird oder wo die Ausgangsmaterialien unter den Reaktionsbedingungen entstehen, oder wo die Reaktionskomponenten in Form ihrer Salze oder reiner Antipoden verwendet werden.

In der Hauptsache sollten solche Ausgangsmaterialien in den benannten Reaktionen verwendet werden, die zur Entstehung derjenigen Verbindungen führen, wie sie vorstehend als besonders bevorzugt aufgeführt worden sind.

Die Erfindung bezieht sich auch auf alle neuartigen Ausgangsmaterialien und Verfahren für ihre Herstellung.

Je nach der Wahl der Ausgangsmaterialien und der Methoden können die neuen Verbindungen in Form eines der möglichen Isomere oder als Gemische davon vorliegen, beispielsweise je nach der Zahl asymmetrischer Kohlenstoffatome als reine optische Isomere wie Antipoden oder als Gemische optischer Isomere wie Racemate oder als Gemische von Diastereoisomeren oder geometrischen Isomeren. Die erwähnten möglichen Isomere und deren Gemische gehören zum Umfang der vorliegenden Erfindung; verschiedene spezielle Isomere sind bevorzugt, wie vorderhand bereits erwähnt.

Entstehende Gemische aus Diastereoisomeren oder Gemische von Racematen können aufgrund der physiko-chemischen Unterscheide der Komponenten in bekannter Weise in reine Isomere, Diastereoisomere, Racemate oder geometrische Isomere getrennt werden, beispielsweise durch Chromatographie und/oder fraktionierte Kristallisation.

Entstehende Racemate lassen sich mit Hilfe bekannter Methoden in die optischen Antipoden auftrennen, beispielsweise durch Reaktion eines sauren Endproduktes mit einer optisch aktiven Base, die Salze mit der racemischen Säure bildet, und Trennen der so erhaltenen Salze, beispielsweise durch fraktionierte Kristallisation, in die diastereoisomeren Salze, aus denen die optisch aktive freie Carbon- oder Phosphonsäureantipoden nach Ansäuerung freigesetzt werden können. Die basischen racemischen Produkte können ebenso in die optischen Antipoden aufgetrennt werden, z.B. durch Trennen der diastereoisomeren Salze derselben mit einer optisch aktiven Säure und Freisetzung der optisch aktiven basischen Verbindung durch Behandlung mit einer Standardbase. Racemische Produkte der Erfindung lassen sich also in ihre optischen Antipoden auftrennen, z.B. durch fraktionierte Kristallisation von d- oder l-(Tartraten, Mandelaten, Camphersulfonaten) oder d- oder l-(α-Methylbenzylamin, Cinchonidin, Cinchonin, Chinin, Chinidin, Ephedrin, Dehydrobietylamin, Brucin oder Strychnin) Salzen. Die sauren Verbindungen der Erfindung lassen sich auch auftrennen durch Trennen der diastereomeren Ester- oder Amid-Derivate, hergestellt aus einem optisch aktiven Alkohol oder Amin, und Freisetzen der abgetrennten optisch aktiven Verbindung. Vorteilhafterweise wird der aktivere der zwei Antipoden isoliert.

Schliesslich erhält man die erfindungsgemässen Verbindungen entweder in freier Form oder als Salze. Jede entstehende Base kann in ein entsprechendes Säureadditionssalz umgewandelt werden, vorzugsweise unter Anwendung einer therapeutisch brauchbaren Säure- oder Anionenaustauschzubereitung, oder die entstehenden Salze lassen sich umwandeln in die entsprechenden freien Basen, beispielsweise durch Anwendung einer stärkeren Base, wie eines Metall- oder Ammoniumhydroxids oder eines basischen Salzes, z.B. eines Alkalimetallhydroxids oder -carbonats, oder einer Kationenaustauschzubereitung oder eines Alkylenoxids wie beispielsweise Propylenoxid. Eine erfindungsgemässe Verbindung mit einer freien Carbon- oder Phosphonsäuregruppe lässt sich also in die entsprechenden Metall- oder Ammoniumsalze umwandeln. Diese oder weitere Salze, beispielsweise die Picrate, lassen sich auch zur Reinigung der erhaltenen Basen verwenden; die Basen werden in Salze umgewandelt, die Salze werden getrennt, und die Basen aus den Salzen freigesetzt.

Im Hinblick auf die enge Verwandtschaft zwischen den freien Verbindungen und den Verbindungen in Form ihrer Salze ist, sofern eine Verbindung in diesem Zusammenhang aufgeführt wird auch, das entsprechende Salz mitumfasst, vorausgesetzt, dass dies unter den jeweiligen Umständen möglich oder geeignet ist.

Die Verbindungen einschliesslich ihrer Salze lassen sich auch in Form ihrer Hydrate erhalten oder enthalten andere Lösungsmittel, die bei ihrer Kristallisation benutzt wurden.

Die pharmazeutischen Präparate gemäss der vorliegenden Erfindung sind diejenigen, die sich für eine enterale, also beispielsweise orale oder rectale, transdermale und parenterale Verabreichung an Säuger einschliesslich des Menschen, zur Blockierung des N-Methyl-D-aspartatsensiblen Aminosäurereizrezeptors

und zur Behandlung von Erkrankungen eignen, die auf die Blockierung des N-Methyl-D-aspartatsensiblen Aminosäurereizrezeptors ansprechen, also beispielsweise cerebrale Isachemie, krampfartige Erkrankungen und Angstzustände, und die eine wirksame Menge einer pharmakologisch aktiven Verbindung der vorliegenden Erfindung allein oder zusammen mit einem oder mehreren pharmazeutisch annehmbaren Trägern enthalten.

Die pharmakologisch aktiven erfindungsgemässen Verbindungen eignen sich für die Herstellung pharmazeutischer Präparate, die eine wirksame Menge derselben unter Beifügung oder Zumischung von Trägern enthalten, die sich für eine enterale oder parenterale Verabreichung eignen. Bevorzugt sind Tabletten und Gelatinekapseln, in denen der aktive Bestandteil enthalten ist zusammen mit a) Verdünnungsmitteln, beispielsweise Lactose, Dextrose, Sucrose, Mannit, Sorbit, Cellulose und/oder Glycin; b) Gleitmitteln, z.B. Siliziumdioxid, Talk, Stearinsäure, deren Magnesium- oder Calciumsalze und/oder Polyethylenglycol; bei Tabletten auch c) Bindemitteln, wie Magnesiumaluminiumsilikat, Stärkepaste, Gelatine, Tragant, Methylcellulose, Natriumcarboxymethylcellulose und/oder Polyvinylpyrrolidon; gewünschtenfalls d) Aufschlussmitteln, beispielsweise Stärken, Agar-agar, Alginsäure oder deren Natriumsalz oder Schäummischungen; und/oder e) Absorbenzien, Färbemitteln, Geschmacksmitteln und Süssungsmitteln. Injizierbare Präparate sind vorzugsweise wässrige isotonische Lösungen oder Suspensionen, und Suppositorien werden vorteilhafterweise aus Fettemulsionen oder Suspensionen hergestellt. Benannte Präparate können sterilisiert sein und/oder Adjuvanzien enthalten, beispielsweise Konservierungs-, Stabilisierungs-, Benetzungs- oder Emulgiermittel, Lösungsförderungsmittel, Salze zum Regulieren des osmotischen Druckes und/oder Puffer. Darüberhinaus können sie auch andere therapeutisch wertvolle Substanzen enthalten. Diese Präparate werden mit Hilfe konventioneller Misch-, Granulier- oder Ueberzugsverfahren hergestellt und enthalten ungefähr 0,1 bis 75%, vorzugsweise ungefähr 1 bis 50% der aktiven Komponenten.

Geeignete Formulierungen für die transdermale Verabreichung umfassen eine wirksame Menge einer Verbindung der Formel I mit Träger. Vorteilhafte Träger sind absorbierbare pharmakologisch annehmbare Lösungsmittel, die den Durchgang durch die Haut des Wirtes unterstützen. Im charakteristischen Falle haben transdermale Systeme die Form eines Pflasters mit einer Schutzschicht, einem Wirkstoffreservoir, gegebenenfalls mit Trägern, gegebenenfalls eine Durchflussmengenregulierungseinrichtung, die den Wirkstoff der Haut des Wirtes mit einer geregelten und im voraus festgelegten Menge über einen längeren Zeitraum hinweg zuführt, und Hilfsmittel zum Befestigen des Systems auf der Haut.

Die Erfindung bezieht sich auch auf eine Verfahren zur Blockierung des N-Methyl-D-aspartatsensiblen Aminosäurereizrezeptors bei Säugern und eine Behandlungsmethode für Erkrankungen bei Säugern, beispielsweise solchen, die auf eine Blockierung des N-Methyl-D-aspartatsensiblen Aminosäurereizrezeptors anpsrechen, also beispielsweise cerebrale Ischaemie, krampfartige Erkrankungen und Angstzustände, wobei ene wirksame Menge einer erfindungsgemässen Verbindung als pharmakologisch aktive Substanz verwendet wird, und zwar vorzugsweise in Form der oben aufgeführten pharmazeutischen Präparate.

Eine besondere Ausführungsform davon bezieht sich auf ein Verfahren zur Behandlung von cerebraler Ischaemie und zur Verhinderung von Gehirnschädigungen als Folge von cerebraler Ischaemie (bei einem Schlagenfall) in Säugern, welches darin besteht, dass eine wirksame Menge einer N-Methyl-D-aspartat blockierenden erfindungsgemässen Verbindung oder eines pharmazeutischen Präparates, welches eine solche Verbindung enthält, an einen Säuger, der einer solchen Behandlung bedarf, verabreicht wird.

Die Dosierung der aktiven verabreichten Verbindung ist abhängig von der Gattung des Warmblüters (Säuger), dem Körpergewicht, dem Alter, dem individuellen Zustand und der Verabreichungsform.

Eine Einheitsdosierung für einen Säuger von ungefähr 50 bis 70 kg kann zwischen etwa 5 und 100 mg der aktiven Komponente enthalten.

Die folgenden Beispiele dienen zur Veranschaulichung der Erfindung und sollen keine Einschränkung derselben darstellen. Die Temperaturen sind in Grad Celsius angegeben. Sofern nichts Abweichendes angegeben ist, werden alle Verdampfungen unter reduziertem Druck durchgeführt, vorzugsweise ungefähr zwischen 2 und 13 kPa.

Beispiel 1

4-[1-(3-Diethylphosphonoprop-2-enyl)]-1-tert.-butoxycarbonylpiperidin-2-carbonsäureethylester wird mit 6N Salzsäure hydrolisiert. Man erhält so die 4-[-(3-Phosphonoprop-2-enyl)]-piperidin-2-carbonsäure.

Das Ausgangsmaterial wird wie folgt hergestellt: 4-(2-Hydroxyethyl)-pyridin wird zum 4-(2-Hydroxyethyl)-pyridin-N-oxid oxidiert, welches seinerseits mit Trimethylsilylcyanid behandelt wird, so dass 4-(2-Hydroxyethyl)-2-cyanpyridin erhalten wird. Letzteres wird in den 4-(2-Hydroxyethyl)-2-pyridincarbonsäureethylester übergeführt und dann zum 4-(2-Hydroxyethyl)-piperidin-2-carbonsäureethylester hydriert.

Eine Lösung von 2,01 g 4-(2-Hydroxyethyl)-piperidin-2-carbonsäureethylester in 5 ml Dichlormethan wird zu einer Lösung von 2,20 g Di-tert.-butyldicarbonat in 10 ml Dichlormethan gegeben. Nach Stehenlassen für 10 Minuten wird das Lösungsmittel abgezogen, und der Rückstand wird einer Blitzchromatographie mit Hexan/Essigester (1:1) unterzogen. Man erhält so den 4-(2-Hydroxyethyl)-1-tert.-butoxycarbonylpiperidin-2-carbonsäureethylester.

Zu einer Lösung von 1,56 g Dimethylsulfoxid in 10 ml Dichlormethan werden bei −78°C 2,2 g

Oxalylchlorid gegeben. Nach 20 Minuten werden 2,4 g 4-(2-Hydroxyethyl)-1-tert.-butoxycarbonylpiperidin-2-carbonsäureethylester in 5 ml Dichlormethan zugegeben. Der Ansatz wird eine Stunde gerührt, und 2,2 g Triethylamin werden zugesetzt. Das Eisbad wird entfernt, die Lösungsmittel abgezogen, und der Rückstand wird mit Hexan/Essigester (7:3) einer Blitzchromatographie unterzogen. Man erhält so den 1-tert.-Butoxycarbonylpiperidin-2-ethoxycarbonyl-4-acetaldehyd.

Bei −78°C werden 2,73 ml m-Butyllithium zu 2,02 g bis(Diethylphosphono)methan in 20 ml wasserfreiem Tetrahydrofuran gegeben. Nach 5 Minuten werden 2,08 g 1-tert.-Butoxycarbonylpiperidin-2-ethoxycarbonyl-4-acetaldehyd in 5 ml Tetrahydrofuran zugegeben. Das Gemisch wird dann 16 Stunden unter Rückfluss erhitzt. Der abgekühlte Ansatz wird eingeengt und einer Blitzchromatographie (95:5 Dichlormethan/Methanol) unterzogen. Man erhält so den 4-[1-(3-Diethylphosphonoprop-2-enyl)]-1-tert.-butoxycarbonylpiperidin-2-carbonsäureethylester.

Beispiel 2

Die folgenden Verbindungen können gemäss den Verfahren hergestellt werden, die in allgemeiner Form in vorstehenden Beispiel beschrieben sind:

(a) trans 3-[1-(4-Phosphonobut-3-enyl)]-pyrrolidin-2-carbonsäure;

(b) trans 3-[1-(4-Phosphonobut-2-enyl)]-pyrrolidin-2-carbonsäure.

Das Ausgangsmaterial für Verbindung (a) lässt sich wie folgt herstellen: trans N-Ethoxycarbonyl-pyrrolidin-2-ethoxycarbonyl-3-acetaldehyd wird mit $(C_6H_5)_3P{=}CHOCH_3$ unter den Bedingungen der Wittig-Reaktion kondensiert, so dass man trans N-Ethoxycarbonylpyrrolidin-2-ethoxycarbonyl-3-propionaldehyd erhält. Kondensation mit bis(Diethylphosphono)methan unter vorstehend beschriebenen Bedingungen (vergleiche Beispiel 1) ergbit den trans 3-[1-(4-Diethylphosphonobut-3-enyl)]-pyrrolidin-2-carbonsäureethylester.

Das Ausgangsmaterial für Verbindung (b) kann wie folgt hergestellt werden: Der Alkohol trans N-Ethoxycarbonyl-3-(2-hydroxyethyl)-pyrrolidin-2-carbonsäureethylester wird zum Aldehyd trans N-Ethoxycarbonylpyrrolidin-2-ethoxycarbonyl-3-acetaldehyd oxidiert, welcher mit Triphenylphosphoranylidenacetaldehyd unter Bedingungen der Wittig-Reaktion kondensiert wird; der erhaltene α,β-ungesättigte-$C_4$-Aldehyd wird zum entsprechenden Alkohol reduziert, der in das Bromid übergeführt wird. Kondensation mit Triethylphosphit ergbit den 3-[-(4-Diethylphosphonobut-2-enyl)]-pyrrolidin-2-carbonsäureethylester.

Beispiel 3

4-[1-(3-Phosphonoprop-1-enyl)]-piperidin-2-carbonsäure kann auf ähnliche Weise hergestellt werden, indem der 4-Hydroxymethyl-N-ethoxycarbonylpiperidin-2-carbonsäureethylester als Zwischenprodukt verwendet wird.

Beispiel 4

Bei −78°C werden 20,3 ml Butyllithium (1,64 M) zu 8,5 ml Tetraethylmethylendiphosphonat [Bis(diethylphosphono)-methan] in 100 ml wasserfreiem Tetrahydrofuran gegeben. Nach 5 Minuten werden 9,26 g 1-(tert.-Butoxycarbonyl)-2-ethoxycarbonylpiperidin-4-acetaldehyd in 125 ml wasserfreiem Tetrahydrofuran dazugegeben. Das Gemisch wird 18 Stunden unter Rückfluss erhitzt, abgekühlt, eingeengt und durch Blitzchromatographie gereinigt, wobei Essigester/Hexan (75:25 bis 9:1) verwendet wird. Man erhält so den 4-[1-(3-Diethylphosphonoprop-2-enyl)]-1-(tert.-butoxycarbonyl)-piperidin-2-carbonsäureethylester.

Das Ausgangsmaterial wird wie folgt hergestellt: Eine Lösung von 4-Pyridylessigsäure in 500 ml wasserfreiem Ethanol, welche 75 ml konzentrierte Schwefelsäure enthält, wird 18 Stunden unter Rückfluss erhitzt. Die Lösung wird auf 0°C abgekühlt und durch Zugabe von Natriumhydroxidlösung und gesättigter wässriger Natriumcarbonatlösung neutralisiert. Extraktion mit Essigester ergibt nach Einengen im Vakuum den 4-Pyridylessigsäureethylester.

Eine Lösung von 23,8 g 4-Pyridylessigsäureethylester in 100 ml wasserfreiem Tetrahydrofuran wird tropfenweise zu 5,5 g Lithiumaluminiumhydrid in 150 ml wasserfreiem Tetrahydrofuran unter Stickstoff gegeben. Das Gemisch wird 40 Minuten auf 50°C (Badtemperatur) erwärmt, dann in einem Eisbad abgekühlt. Ueberschüssiges Lithiumaluminiumhydrid wird durch Zugabe von 6,6 ml Wasser zersetzt, gefolgt von 6,6 ml 15%-igem wässrigen Natriumhydroxid und 20 ml Wasser. Der Feststoff wird abfiltriert und das Filtrat im Vakuum eingeengt, man erhält so 4-Pyridylethanol.

Eine Lösung von 16,3 g 4-Pyridylethanol, 21,8 g Chlor-tert.-Butyl-dimethylsilan und 10,9 g Imidazol in 150 ml Dimethylformamid wird 1 Stunde bei Raumtemperatur gerührt. Das Produkt wird durch Extraktion in Essigester/Hexan (1:1) isoliert und viermal mit 400 ml Wasser gewaschen. Der Extrakt wird durch Silikagel filtriert und im Vakuum eingeengt. Man erhält so 4-(tert.-Butyl-dimethylsilyloxyethyl)-pyridin.

Zu einer gerührten Lösung von 28,8 g 4-(tert.-Butyl-dimethylsilyloxyethyl)-pyridin in 300 ml Dichlormethan werden 24 g m-Chlorperbenzoesäure gegeben. Nach 4 Stunden wird die Lösung mit wässriger Natriumcarbonatlösung gewaschen, sodann mit Wasser. Die Lösung wird über Natriumsulfat getrocknet, filtriert und im Vakuum eingeengt. Man erhält so das 4-(tert-Butyl-dimethylsilyloxyethyl)-pyridin-N-oxid.

Eine Lösung von 28,6 g 4-(tert.-Butyl-dimethylsilyloxyethyl)-pyridin-N-oxid, 60,3 ml

Trimethylsilylcyanid und 31,5 ml Triethylamin wird unter Stickstoff 3 Stunden unter Rückfluss erhitzt und gerührt. Die dunkle Lösung wird in einem Eisbad abgekühlt, 30 ml Ethanol werden zugegeben, danach 400 ml Essigester und 200 ml Hexan. Die Lösung wird zweimal mit 150 ml Wasser gewaschen, über Natriumsulfat getrocknet, filtriert, im Vakuum eingeengt und durch Blitzchromatographie unter Verwendung von Essigester/Hexan (1:10) gereinigt. Man erhält so 4-(tert.-Butyl-dimethylsilyloxyethyl)-2-cyanpyridin.

Eine Lösung 22,2 g 4-(tert.-Butyl-dimethylsilyloxyethyl)-2-cyanpyridin in 220 ml wasserfreiem Ethanol, welche 0.19 g Natrium enthält, wird 24 Stunden bei Raumtemperatur gerührt. Die Lösung wird dann auf 0°C abgekühlt und 22 ml 6N Salzsäure werden zugegeben. Die Lösung wird 16 Stunden bei Raumtemperatur gerührt, auf 0°C abgekühlt, 7,5 ml 6N Natriumhydroxid werden zugesetzt, danach 75 ml gesättigtes wässriges Natriumbicarbonat. Extraktion mit Dichlormethan und Blitzchromatographie unter Verwendung von Essigester ergibt den 4-(2-Hydroxyethyl)-pyridin-2-carbonsäureethylester.

Ein Gemisch von 8,37 g 4-(2-Hydroxyethyl)-pyridin-2-carbonsäureethylester in 130 ml Essigsäure und 4 g Platinoxid wird bei 345 kPa hydriert. Filtrieren, Einengen im Vakuum, Neutralisieren mit Kaliumcarbonat und Extrahieren mit Dichlormethan ergibt eine Oel, das durch Blitzchromatographie unter Verwendung von Dichlormethan/Methanol gesättigt mit Ammoniak (20:1) gereinigt wird. Man erhält so den 4-(2-Hydroxyethyl)piperidin-2-carbonsäureethylester.

Eine Lösung von 7,9 g 4-(2-Hydroxyethyl)-piperidin-2-carbonsäureethylester und 9,0 g Di-tert.-butyldicarbonat in 80 ml Dichlormethan wird 2 Stunden bei Raumtemperatur gerührt und dann im Vakuum eingeengt. Man erhält so 1-(tert.-Butoxycarbonyl)-4-(2-hydroxyethyl)-piperidin-2-carbonsäureethylester.

Eine Lösung von 11,8 g 1-(tert.-Butoxycarbonyl)-4-(2-hydroxyethyl)-piperidin-2-carbonsäureethylester und 12,6 g Pyridiniumchlorochromat in 175 ml Dichlormethan wird unter Stickstoff bei Raumtemperatur 80 Minuten gerührt. Das Gemisch wird filtriert und durch Blitzchromatographie unter Verwendung von Essigester/Hexan (25:75) gereinigt. Man erhält so den 1-(tert.-Butoxycarbonyl)-2-ethoxycarbonyl-piperidin-4-acetaldehyd.

Beispiel 5

Ein Gemisch von 4,67 g 4-[1-(3-Diethylphosphonoprop-2-enyl)]-1-(tert.-butoxycarbonyl)-piperidin-2-carbonsäureethylester und 75 ml 6N Salzsäure wird 12 Stunden unter Rückfluss erhitzt. Die Lösung wird im Vakuum zur Trockne eingeengt. Der Rückstand wird in 50 ml Ethanol aufgenommen, und 3,8 ml Propylenoxid werden zugegeben. Der Feststoff, der sich abtrennt, wird abfiltriert und im Vakuum getrocknet. Man erhält so die cis-4-[1-(3-Phosphonoprop-2-enyl)]-piperidin-2-carbonsäure, Fp. 163—165°C.

Beispiel 6

Bei −78°C werden 6,9 ml Butyllithium (1.6 M) zu 2,9 ml Tetraethylmethylendiphosphonat in 30 ml wasserfreiem Tetrahydrofuran gegeben. Nach 5 Minuten werden 2,47 g trans 1-Acetylpiperidin-2-ethoxycarbonyl-4-acetaldehyd in 55 ml wasserfreiem Tetrahydrofuran zugegeben. Das Gemisch wird 18 Stunden unter Rückfluss erhitzt, abgekühlt, eingeengt und durch Blitzchromatographie unter Verwendung von Dichlormethan/Ethanol (100:3) gereinigt. Man erhält so den trans 4-[(3-Diethylphosphonoprop-2-enyl)]-1-acetyl-piperidin-2-carbonsäureethylester.

Das Ausgangsmaterial wird wie folgt hergestellt: Bei 0°C werden 9,4 ml Essigsäureanhydrid zu einer gerührten Lösung von 13,5 g 4-(2-Hydroxyethyl)-piperidin-2-carbonsäureethylester in 75 ml Pyridin gegeben. Nach Rühren bei Raumtemperatur während 30 Minuten wird die Lösung im Vakuum eingeengt und Essigester (300 ml) wird zugesetzt. Die Lösung wird zweimal mit 2N Salzsäure gewaschen, einmal mit Wasser und einmal mit gesättigter Natriumbicarbonatlösung, über Natriumsulfat getrocknet, filtriert und im Vakuum eingeengt. Der Rückstand wird in 100 ml Ethanol aufgenommen, 5 g pulverisiertes Kaliumcarbonat wird zugesetzt, und die Mischung wird eine Stunde bei Raumtemperatur gerührt. Die Lösung wird filtriert, im Vakuum eingeengt und durch Blitzchromatographie unter Verwendung von Dichlormethan/Methanol (95:5) gereinigt. Man erhält so den trans 1-Acetyl-4-(2-hydroxyethyl)-piperidin-2-carbonsäureethylester.

Eine Mischung von 3,7 g trans 1-Acetyl-4-(2-hydroxyethyl)-piperidin-2-carbonsäureethylester und 5,4 g Pyridiniumchlorochromat in 75 ml Dichlormethan wird unter Stickstoff 2 Stunden gerührt. Das Gemisch wird filtriert und durch Blitzchromatographie unter Verwendung von Essigester/Hexan (7:3 bis 8:2) gereinigt. Man erhält so den trans 1-Acetylpiperidin-2-ethoxycarbonyl-4-acetaldehyd.

Beispiel 7

Ein Gemisch von 2,5 g trans 4-[1-(3-Diethylphosphonoprop-2-enyl)]-1-acetylpiperidin-2-carbonsäureethylester und 40 ml 6N Salzsäure wird 12 Stunden unter Rückfluss erhitzt. Die Lösung wird im Vakuum zur Trockne eingeengt. Der Rückstand wird in 20 ml Ethanol aufgenommen, und 2,3 ml Propylenoxid werden zugegeben. Der Feststoff, der sich abscheidet, wird abfiltriert und im Vakuum getrocknet. Man erhält so die trans 4-[1-(3-Phosphonoprop-2-enyl)]-piperidin-2-carbonsäure, Fp. 132—140°C.

Beispiel 8

Eine Lösung von 0,334 g trans 4-[1-(3-Bromoprop-1-enyl)]-1-(tert.-butoxycarbonyl)-piperidin-2-

carbonsäureethylester und 3,5 ml Triethylphosphit wird unter Stickstoff 70 Minuten unter Rückfluss erhitzt. Die abgekühlte Lösung wird im Vakuum eingeengt und durch Blitzchromatographie unter Verwendung von Dichlormethan/Methanol (100:3) gereinigt. Man erhält so den trans 4-[1-(3-Diethylphosphonoprop-1-enyl)]-1-(tert.-butoxycarbonyl)-piperidin-2-carbonsäureethylester.

Das Ausgangsmaterial wird wie folgt hergestellt: Eine Lösung von 20 g 4-Pyridylcarbinol, 28,9 g Chlor-tert.-butyl-dimethylsilan und 14,4 g Imidazol in 200 ml Dimethylformamid wird 3 Stunden bei Raumtemperatur gerührt. Das Produkt wird durch Extraktion in Essigester/Hexan (1:1) isoliert, und der Extrakt wird viermal mit 400 ml Wasser gewaschen. Die Lösung wird über Silikagel filtriert und im Vakuum eingeengt. Man erhält so 4-(tert.-Butyl-dimethylsilyloxymethyl)pyridin.

Eine Lösung von 40,6 g 4-(tert.-Butyl-dimethylsilyloxymethyl)pyridin und 40 g m-Chlorperbenzoesäure in 500 ml Dichlormethan wird 16 Stunden bei Raumtemperatur gerührt. Die Lösung wird mit 2N Natronlauge und Wasser gewaschen, über Natriumsulfat getrocknet, filtriert und im Vakuum eingeengt. Man erhält so 4-(tert.-Butyldimethylsilyloxymethyl)-pyridin-N-oxide.

Eine Lösung von 37,9 g 4-(tert.-Butyldimethylsilyloxymethyl)-pyridin-N-oxide, 84 ml Trimethylsilylcyanid und 44 ml Triethylamin wird unter Stickstoff 2 1/2 Stunden unter Rückfluss erhitzt und gerührt. Die dunkle Lösung wird in einem Eisbad abgekühlt, dann werden 40 ml Ethanol zugegeben, danach 500 ml Essigester. Die Lösung wird zweimal mit Wasser gewaschen, über Natriumsulfat getrocknet, filtriert, im Vakuum eingeengt und durch Blitzchromatographie unter Verwendung von Essigester/Hexan (1:10) gereingt. Man erhält so 4-(tert.-Butyl-dimethylsilyloxymethyl)-2-cyanpyridin.

Eine Lösung von 21,7 g 4-(tert.-Butyl-dimethylsilyloxymethyl)-2-cyanpyridin in 220 ml wasserfreiem Ethanol, welche 0,2 g Natrium enthält, wird bei Raumtemperatur 18 Stunden gerührt, auf 0°C abgekühlt, sodann werden 22 ml 6N Salzsäure zugegeben. Die Lösung wird bei Raumtemperatur 18 Stunden gerührt, auf 0°C abgekühlt, 7,5 ml 6N Natriumlauge werden zugegeben, danach 20 ml gesättigte wässrige Natriumcarbonatlösung.

Extrahieren mit Dichlormethan, danach Trocknen, Filtrieren und Einengen des Extraktes ergibt ein Oel, das aus Ether kristallisiert. Man erhält so den 4-(Hydroxymethyl)pyridin-2-carbonsäureethylester.

Ein Gemisch von 13,9 g 4-(Hydroxymethyl)pyridin-2-carbonsäureethylester und 5 g Platinoxid in 250 ml Essigsäure wird bei 344 kPa hydriert. Filtrieren, Einengen im Vakuum und Neutralisieren mit Kaliumcarbonat in Dichlormethan ergibt ein Oel, das durch Blitzchromatographie unter Verwendung von Dichlormethan/Methanol gesättigt mit Ammoniak (20:1) gereinigt wird. Man erhält so 4-(Hydroxymethyl)piperidin-2-carbonsäureethylester.

Eine Lösung von 5,16 g 4-(Hydroxymethyl)piperidin-2-carbonsäureethylester und 6,33 g Di-tert.-butyldicarbonat in 100 ml Dichlormethan wird bei Raumtemperatur 18 Stunden gerührt. Die Lösung wird im Vakuum eingeengt, man erhält so 1-(tert.-Butoxycarbonyl)-4-(hydroxymethyl)-piperidin-2-carbonsäurethylester.

Eine Lösung von 7,9 g 1-(tert.-Butoxycarbonyl)-4-(hydoxymethyl)-piperidin-2-carbonsäureethylester und 9,9 g Pyridiniumchlorochromat in 175 ml Dichlormethan wird bei Raumtemperatur 3 Stunden gerührt. Das Gemisch wird filtriert und durch Blitzchromatographie unter Verwendung von Essigester/Hexan (25:75) gereinigt. Man erhält so den 1-(tert.-Butoxycarbony)-2-ethoxycarbonylpiperdin-4-carboxaldehyd.

Eine Lösung von 1 g 1-(tert.-Butoxycarbonyl)-2-ethoxycarbonylpiperidin-4-carboxaldehyd und 2 g Formylmethylen-triphenylphosphoran in 16 ml Toluol wird 2 Stunden auf 100°C (Badtemperatur) erhitzt. Die Lösung wird abgekühlt und durch Blitzchromatographie unter Verwendung von Essigester/Hexan (25:75) gereinigt. Man erhält so den 1-(tert.-Butoxycarbonyl)-2-ethoxycarbonylpiperidin-4-acrylaldehyd.

Eine Lösung von 0.83 g 1-(tert.-Butoxycarbonyl)-2-ethoxycarbonylpiperidin-4-acrylaldehyd und 0,11 g Natriumborohydrid in 8 ml Ethanol wird bei 0°C 30 Minuten gerührt. 0,2 ml Essigsäure werden zugegeben, danach eiskaltes Wasser, und das Gemisch wird mit Dichlormethan extrahiert. Der Extrakt wird über Natriumsulfat getrocknet, filtriert und im Vakuum eingeengt. Reinigung durch Blitzchromatographie unter Verwendung von Essigester/Hexan (25:75) ergibt den trans 1-(tert.-Butoxycarbonyl)-4-[1-(3-hydroxyprop-1-enyl)]piperidin-2-carbonsäureethylester.

Eine Lösung von 0,367 g trans 1-(tert.-Butoxycarbonyl)-4-[1-(3-hydroxyprop-1-enyl)]-piperidin-2-carbonsäurethylester, 0,35 g Triphenylphosphin und 0,23 g Bromsuccinimid in 8 ml Dichlormethan wird zu Beginn bei 0°C und dann bei Raumtemperatur 40 Minuten gerührt. Reinigung durch Blitzchromatographie unter Verwendung von Essigester/Hexan (1:9) ergibt den trans 4-[1-(3-Bromprop-1-enyl)]-1-(tert.-butoxycarbonyl)-piperidin-2-carbonsäurethylester.

## Beispiel 9

Eine Lösung von 0,39 g trans 4-[1-(3-Diethylphosphonoprop-1-enyl)]-1-(tert.-butoxycarbonyl)-piperidin-2-carbonsäureethylester und 2,3 ml Trifluoressigsäure in 8 ml Dichlormethan wird bei Raumtemperatur 30 Minuten gerührt. Gesättigte wässrige Natriumbicarbonatlösung wird zugegeben, und die Mischung wird mit Dichlormethan extrahiert. Reinigung durch Blitzchromatographie unter Verwendung von Dichlormethan/Methanol gesättigt mit Ammoniak (20:1) ergibt den trans 4-[1-(3-Diethylphosphonoprop-1-enyl)]-piperidin-2-carbonsäureethylester.

## Beispiel 10

Eine Lösung von 0,265 g trans 4-[1-(3-Diethylphosphonoprop-1-enyl)]-piperidin-2-carbonsäureethylester in 5 ml wasserfreiem Ethanol, welche 0,074 ml Butyllithium (1,6 M) enthält, wird 72 Stunden auf 80°C (Badtemperatur) erhitzt. Nach Abkühlen werden 0,025 ml Essigsäure zugegeben, danach überschüssige gesättigte Natriumbicarbonatlösung, und das Gemisch wird mit Dichlormethan extrahiert. Einengen des Dichloromethanextraktes im Vakuum ergibt ein Oel. Dessen Reinigung durch Blitzchromatographie unter Verwendung von Dichlormethan/Isopropanol gesättigt mit Ammoniak (20:1) ergibt cis 4-[1-(3-Diethylphosphonoprop-1-enyl)]-piperidin-2-carbonsäureethylester.

Beispiel 11

a) Ein Gemisch von 0,142 g cis 4-[1-(3-Diethylphosphonoprop-1-enyl)]piperidin-2-carbonsäureethylester und 2,5 ml 6N Salzsäure wird 12 Stunden unter Rückfluss erhitzt. Die Lösung wird im Vakuum zur Trockne eingeengt.

Der Rückstand wird in 2 ml Ethanol aufgenommen, und 0,15 ml Propylenoxid werden zugegeben. Der Feststoff, der sich abscheidet, wird abfiltriert und im Vakuum getrocknet. Man erhält so die cis 4-[1-(3-Phosphonoprop-1-enyl)]-piperidin-2-carbonsäure, Fp. 175°C (Zersetzung).

b) Auf analoge Weise ergibt die Hydrolyse des trans-Esters von Beispiel 9 die entsprechende trans-Säure, Fp. 130—135°C.

c) Eine Lösung von cis 4-[1-(3-Phosphonoprop-1-enyl)]-piperidin-2-carbonsäure in mit Chlorwasserstoff gesättigtem Ethanol wird über Nacht unter Rückfluss erhitzt. Das Lösungsmittel wird im Vakuum entfernt. Eine Lösung des Rückstandes in Ethanol wird mit Propylenoxid behandelt und zur Trockne eingeengt. Man erhält so den cis 4-[1-(3-Phosphonoprop-1-enyl)]piperidin-2-carbonsäureethylester.

Beispiel 12

Zu einer Lösung von 0,234 g trans 1-Ethoxycarbonyl-2-ethoxycarbonyl-pyrrolidin-3-propionaldehyd in 5 ml wasserfreiem Tetrahydrofuran unter Stickstoff, gekühlt auf −78°C, wird langsam eine Lösung von 0,23 ml Tetraethylmethylendiphoshonat und 0,57 ml Butyllithium (1,6 M) in 5 ml wasserfreiem Tetrahydrofuran, ebenfalls bei −78°C, gegeben. Die Lösung wird 14 Stunden unter Rückfluss erhitzt, abgekühlt und mit 0,15 ml Essigsäure behandelt. Der Ansatz wird eingeengt, mit Wasser verdünnt und mit Dichlormethan extrahiert. Der Extrakt wird über Natriumsulfat getrocknet, filtriert und eingeengt. Reinigung durch Blitzchromatographie unter Verwendung von Essigester ergibt den trans 1-Ethoxycarbonyl-3-[1-(4-diethylphosphonobut-3-enyl)]-pyrrolidin-2-carbonsäureethylester.

Das Ausgansmaterial wird wie folgt hergestellt: Ein Gemisch von 50 g N-(2-Cyanethyl)glycin in 300 ml Ethanol, gesättigt mit Chlorwasserstoffgas, wird 1 Stunde bei Raumtemperatur gerührt, dann 1 Stunde unter Rückfluss erhitzt. Nach Abkühlen wird der Feststoff abfiltriert und das Filtrat mit Natriumbicarbonat neutralisiert, wiederum filtriert und eingeengt. Man erhält so N-(Ethoxycarbonylmethyl)-β-alaninethylester.

Zu einer Mischung von 49 g N-(Ethoxycarbonylmethyl)-β-alaninethylester und 30 ml Wasser, gekühlt auf −10°C, wird tropfenweise 27,6 ml Chlorkohlensäureethylester, gefolgt von einer Lösung von 12,7 g Natriumcarbonat in 50 ml Wasser, gegeben. Das Gemisch wird auf Raumtemperatur erwärmt, dann 50 Minuten auf 55°C erhitzt. Das Gemisch wird gekühlt, mit Toluol extrahiert, der Extrakt wird mit 2N Salzsäure und Wasser gewaschen. Der Extrakt wird über Natriumsulfat getrocknet, filtriert, eingeengt und im Vakuum destilliert. Man erhält so N-(Ethoxycarbonylmethyl)-N-(ethoxycarbonyl)-β-alaninethylester.

Eine Lösung von 53,9 g N-(Ethoxycarbonylmethyl)-N-(ethoxycarbonyl)-β-alaninethylester in 200 ml Toluol wird tropfenweise zu einer gerührten Lösung von Kaliumhexamethyldisilazid (429 ml, 0,653M) in 125 ml Toluol unter Stickstoff gegeben und auf 0°C abgekühlt. Nach 45 Minuten bei 0°C werden 21,6 ml Essigsäure zugegeben, danach eine Lösung von 100 g Natriumphosphat (einbasisch) in 1 Liter Wasser. Die Phasen werden getrennt, die organische Phase wird mit einem pH 7 Puffer gewaschen, über Natriumsulfat getrocknet, filtriert, eingeengt und durch Blitzchromatographie unter Verwendung von Essigester/Hexan (3:7) gereinigt. Man erhält so 1-Ethoxycarbonyl-3-oxo-pyrrolidin-2-carbonsäureethylester.

Zu einer gerührten Suspension von 24 g Benzyloxycarbonylmethyl-triphenylphosphoniumbromid in 225 ml wasserfreiem Tetrahydrofuran, unter Stickstoff und auf 0°C gekühlt, werden 73 ml Kaliumhexamethyldisilazid (0,652 M) gegeben. Nach 10 Minuten wird eine Lösung von 11 g 1-Ethoxycarbonyl-3-oxo-pyrrolidin-2-carbonsäurethylester in 50 ml Tetrahydrofuran zugegeben, und das Gemisch wird 2 1/2 Stunden unter Rückfluss erhitzt. Nach Abkühlen wird das Gemisch filtriert und im Vakuum eingeengt. Der Rückstand wird in Essigester aufgenommen, mit Wasser gewaschen, über Natriumsulfat getrocknet, filtriert, eingeengt und durch Blitzchromatographie unter Verwendung von Essigester/Hexan (1:4) gereinigt. Man erhält so ein Oel, welches in 200 ml Ethanol mit 5 g 10% Palladium auf Kohle hydriert wird. Man erhält so nach Filtrieren und Einengen des Filtrats 1-Ethoxycarbonyl-2-ethoxycarbonylpyrrolidin-3-essigsäure.

Eine Lösung von 10 g 1-Ethoxycarbonyl-2-ethoxycarbonylpyrrolidin-3-essigsäure in 50 ml Tetrahydrofuran wird auf 0°C gekühlt, und 55 ml Boran/Tetrahydrofuran (1 M) werden tropfenweise zugegeben. Nach 2 Stunden Rühren bei 0°C werden 20 ml Wasser zugegeben, das Gemisch wird mit Essigester extrahiert, zweimal mit Wasser gewaschen und über Natriumsulfat getrocknet. Die Lösung wird filtriert, eingeengt und durch Blitzchromatographie unter Verwendung von Essigester/Hexan (1:1 bis 7:3) gereinigt. Man erhält so cis 1-Ethoxycarbonyl-2-ethoxycarbonylpyrrolidin-3-ethanol.

Eine Lösung von 6 g cis 1-Ethoxycarbonyl-2-ethoxycarbonylpyrrolidin-3-ethanol in 75 ml

Dichlormethan, welche 7,3 ml Diisopropylethylamin enthält, der 5,1 ml 85% Benzyloxymethylchlorid zugesetzt sind, wird 3 1/2 Stunden bei Raumtemperatur gerührt. Die Lösung wird mit Wasser gewaschen und mit gesättigter wässriger Natriumbicarbonatlösung. Die Lösung wird dann über Natriumsulfat getrocknet, filtriert, eingeengt und durch Blitzchromatographie unter Verwendung von Essigester/Hexan (1:4) gereinigt. Man erhält so cis 1-Ethoxycarbonyl-3-[2-(benzyloxymethoxy)ethyl]-pyrrolidin-2-carbonsäureethylester.

Eine Lösung von 6,2 g cis 1-Ethoxycarbonyl-3-[2-(benzyloxymethoxy)ethyl]-pyrrolidin-2-carbonsäureethylester in 75 ml wasserfreiem Ethanol, welche 1 ml Butyllithium (1,6M) enthält, wird unter Stickstoff 6 Tage unter Rückfluss erhitzt. Nach dem Abkühlen werden 1,7 ml 1N Salzsäure zugegeben, die Lösung wird im Vakuum eingeengt, kaltes Wasser wird zugegeben, und das Gemisch wird mit Dichlormethan extrahiert. Der Extrakt wird über Natriumsulfat getrocknet, filtriert, eingeengt und durch Hochdruckflüssigschromatographie unter Verwendung von Essigester/Hexan (15:85) gereinigt. Man erhält so trans 1-Ethoxycarbonyl-3-[2-(benzyloxymethoxy)-ethyl]-pyrrolidin-2-carbonsäureethylester.

Ein Gemisch von 1,64 g trans 1-Ethoxycarbonyl-3-[2-(benzyloxymethoxy)ethyl]-pyrrolidin-2-carbonsäureethylester und 2 g 10% Palladium auf Kohle in 35 ml Essigsäure wird bei 310 kPa hydriert. Das Gemisch wird filtriert, im Vakuum eingeengt und durch Blitzchromatographie unter Verwendung von Essigester/Hexan (6:4 bis 7:3) gereinigt. Man erhält so trans 1-Ethoxycarbonyl-2-ethoxycarbonylpyrrolidin-3-ethanol.

Eine Lösung von 0,991 g trans 1-Ethoxycarbonyl-2-ethoxycarbonylpyrrolidin-3-ethanol und 1,24 g Pyridiniumchlorochromat in 20 ml Dichlormethan wird unter Stickstoff 4 Stunden gerührt. Das Gemisch wird filtriert und durch Blitzchromatographie unter Verwendung von Essigester/Hexan (1:1) gereinigt. Man erhält so den trans 1-Ethoxycarbonyl-2-ethoxycarbonylpyrrolidin-3-acetaldehyd.

Zu 0,4 g trans 1-Ethoxycarbonyl-2-ethoxycarbonylpyrrolidin-3-acetaldehyd in 5 ml wasserfreiem Tetrahydrofuran unter Stickstoff, gekühlt auf −78°C, wird tropfenweise eine Lösung von 1,17 g Methoxymethyl-triphenylphosphoniumchlorid in 15 ml wasserfreiem Tetrahydrofuran gegeben, welche 1,55 ml Kalium-tert.-butoxid/Tetrahydrofuran (1,6 M) enthält. Die Lösung wird bei Raumtemperatur 4 Stunden gerührt. 11 ml 2N Salzsäure werden zugegeben, und das Gemisch wird 45 Minuten gerührt. Die Lösung wird eingeengt und dann mit Dichlormethan extrahiert. Der Extrakt wird über Natriumsulfat getrocknet, filtriert, eingeengt, und der Rückstand wird durch Blitzchromatographie unter Verwendung von Essigester/Hexan (1:5 bis 3:7) gereinigt. Man erhält so den trans 1-Ethoxycarbonyl-2-ethoxycarbonylpyrrolidin-3-propionaldehyd.

Beispiel 13

Ein Gemisch von 0,193 g trans 1-Ethoxycarbonyl-3-[1-(4-diethylphosphonobut-3-enyl)]-pyrrolidin-2-carbonsäureethylester und 4 ml konzentrierter Salzsäure wird 16 Stunden unter Rückfluss erhitzt. Die Lösung wird abgekühlt und im Vakuum zur Trockne eingeengt. Der Feststoff, der sich abscheidet, wird abfiltriert und im Vakuum getrocknet. Man erhält so die trans 3-[1-(4-Phosphonobut-3-enyl)]-pyrrolidin-2-carbonsäure, Fp. 110—115°C (Zersetzung).

Beispiel 14

Zu einer Lösung von 0,426 g cis 1-Ethoxycarbonyl-2-ethoxycarbonylpyrrolidin-3-propionaldehyd in 10 ml wasserfreiem Tetrahydrofuran, unter Stickstoff und gekühlt auf −78°C, wird langsam eine Lösung von 0,45 ml Tetraethylmethylendiphosphonat und 1,06 ml Butyllithium (1,6 M) in 10 ml wasserfreiem Tetrahydrofuran, ebenfalls bei −78°C, gegeben. Die Lösung wird 14 Stunden unter Rückfluss erhitzt, abgekühlt und mit 0,3 ml Essigsäure versetzt. Der Ansatz wird eingeengt, Wasser wird zugegeben, und die Mischung wird mit Dichlormethan extrahiert. Der Extrakt wird über Natriumsulfat getrocknet, filtriert, eingeengt und durch Blitzchromatographie unter Verwendung von Essigester gereinigt. Man erhält so cis 1-Ethoxycarbonyl-3-[1-(4-diethylphosphonobut-3-enyl)]-pyrrolidin-2-carbonsäureethylester.

Das Ausgangsmaterial wird wie folgt hergestellt: Eine Lösung von 0,908 g cis 1-Ethoxycarbonyl-2-ethoxycarbonylpyrrolidin-3-ethanol und 1,14 g Pyridiniumchlorochromat in 20 ml Dichlormethan wird 4 Stunden unter Stickstoff gerührt. Das Gemisch wird filtriert und durch Blitzchromatographie unter Verwendung von Essigester/Hexan (1:1) gereinigt. Man erhält so den cis 1-Ethoxycarbonyl-2-ethoxycarbonylpyrrolidin-3-acetaldehyd.

Zu 0,663 g cis 1-Ethoxycarbonyl-2-ethoxycarbonylpyrrolidin-3-actaldehyd in 10 ml wasserfreiem Tetrahydrofuran unter Stickstoff, gekühlt auf −78°C, wird tropfenweise eine Lösung von 2,21 g Methoxymethyl-triphenylphosphoniumchlorid in 20 ml wasserfreiem Tetrahydrofuran gegeben, welche 2,6 ml Kalium-tert.-butoxid/Tetrahydrofuran (1,6 M) enthält. Die Lösung wird 4 Stunden bei Raumtemperatur gerührt. 15 ml 2N Salzsäure werden zugegeben, und das Gemisch wird 45 Minuten gerührt. Die Lösung wird eingeengt und dann mit Dichlormethan extrahiert. Der Extrakt wird über Natriumsulfat getrocknet, filtriert und eingeengt. Reinigung durch Blitzchromatographie unter Verwendung von Essigester/Hexan (1:5 bis 3:7) ergibt den cis 1-Ethoxycarbonyl-2-ethoxycarbonylpyrrolidin-3-propionaldehyd.

Beispiel 15

Ein Gemisch von 0,176 g cis 1-Ethoxycarbonyl-3-[1-(4-diethylphosphonobut-3-enyl)]-pyrrolidin-2-carbonsäureethylester und 3,5 ml konzentrierter Salzsäure wird 18 Stunden unter Rückfluss erhitzt. Die Lösung wird im Vakuum zur Trockne eingeengt, der Rückstand wird in 1,5 ml Isopropanol/Methanol (5:1) aufgenommen, und 0,2 ml Propylenoxid werden zugegeben. Der Feststoff, der sich abscheidet, wird abfiltriert und im Vakuum getrocknet. Man erhält so cis 3-[1-(4-Phosphonobut-3-enyl)]pyrrolidin-2-carbonsäure, Fp. 132—140°C.

Beispiel 16

Eine Lösung von 0,0665 g trans 1-Ethoxycarbonyl-3-[1-(4-brombut-2-enyl)]-pyrrolidin-2-carbonsäureethylester in 0,75 ml Triethylphosphit wird 20 Minuten unter Rückfluss erhitzt. Die Lösung wird im Vakuum eingeengt, und der Rückstand wird durch Blitzchromatographie unter Verwendung von Dichloromethan/Ethanol (30:1) gereinigt. Man erhält so den trans 1-Ethoxycarbonyl-3-[1-(4-diethylphosphonobut-2-enyl)]pyrrolidin-2-carbonsäureethylester.

Das Ausgangsmaterial wird wie folgt hergestellt: Eine Lösung von 0,225 g trans 1-Ethoxycarbonyl-2-ethoxycarbonylpyrrolidin-3-acetaldehyd und 0,4 g (Formylmethylen)-triphenylphosphoran in 2 ml Dichlormethan wird 44 Stunden unter Rückfluss erhitzt. Die Lösung wird eingeengt, und der Rückstand wird durch Blitzchromatographie unter Verwendung von Essigester/Hexan (2:3) gereinigt. Man erhält so den trans 1-Ethoxycarbonyl-3-[1-(4-oxobut-2-enyl)]-pyrrolidin-2-carbonsäureethylester.

Zu einer gerührten Lösung von 0,102 g trans 1-Ethoxycarbonyl-3-[1-(4-oxobut-2-enyl)]-pyrrolidin-2-carbonsäureethylester in 2 ml Ethanol, auf 0°C gekühlt, werden 0,035 g Natriumborhydrid gegeben. Nach 30 Minuten bei 0°C und 30 Minuten bei Raumtemperatur werden 0,05 ml Essigsäure zugegeben, die Lösung wird eingeengt, Wasser zugegeben, und das Gemisch wird mit Dichlormethan extrahiert. Die Lösung wird über Natriumsulfat getrocknet, filtriert und im Vakuum eingeengt. Man erhält so den trans 1-Ethoxycarbonyl-3-[1-(4-hydroxbut-2-enyl)]-pyrrolidin-2-carbonsäureethylester.

Zu einer gerührten Lösung von 0,0937 g trans 1-Ethoxycarbonyl-3-[1-(4-hydroxybut-2-enyl)]-pyrrolidin-2-carbonsäureethylester und 0,1 g Triphenylphosphin in 2 ml Dichlormethan werden bei 0°C 0,067 g N-Bromsuccinimid gegeben. Nach 30 Minuten bei Raumtemperatur wird das Gemisch durch Blitzchromatographie unter Verwendung von Essigester/Hexan (1:3) gereinigt. Man erhält so den trans 1-Ethoxycarbonyl-3-[1-(4-brombut-2-enyl)]-pyrrolidin-2-carbonsäureethylester.

Beispiel 17

Ein Gemisch von 0,053 g trans 1-Ethoxycarbonyl-3-[1-(4-diethylphosphononbut-2-enyl)]-pyrrolidin-2-carbonsäureethylester und 1,5 ml konzentrierter Salzsäure wird 16 Stunden unter Rückfluss erhitzt. Die Lösung wird im Vakuum zur Trockne eingeengt, der Rückstand wird in 1 ml Methanol aufgenommen, 0,2 ml Propylenoxid werden zugegeben, und die Lösung wird eingeengt. Der Rückstand wird durch Ionenaustauschchromatographie gereinigt, wobei mit 0,1N Ammoniumhydroxidlösung eluiert wird. Man erhält so die trans 3-[1-(4-Phosphonobut-2-enyl)]-pyrrolidin-2-carbonsäure, Fp. 138—145°C.

Beispiel 18

Herstellung einer injizierbaren Formulierung, die pro 5 ml Lösung 10 mg der aktiven Verbindung enthält:

Zusammensetzung

| | |
|---|---|
| cis 4-[1-(3-Phosphonoprop-1-enyl)]-piperidin-2-carbonsäure | 10,0 g |
| Propylparaben | 0,5 g |
| Wasser | ad 5000 ml |

Der aktive Wirkstoff und Konservierungsstoffe werden in 3500 ml Wasser für Injektionszwecke gelöst, und die Lösung wird auf 5000 ml verdünnt. Die Lösung wird durch ein steriles Filter filtriert und unter sterilen Bedingungen in Injektionsampullen abgefüllt, wobei jede Ampulle 5 ml der Lösung enthält.

Beispiel 19

a) Herstellung von 10,000 Tabletten mit je 10,0 mg Wirkstoff:

Bestandteile

| | |
|---|---|
| cis 4-[1-(3-Phosphonoprop-1-enyl)]-piperidin-2-carbonsäure | 100,00 g |
| Lactose | 2535,00 g |
| Maisstärke | 125,00 g |
| Polyethylenglycol 6000 | 150,00 g |
| Magnesiumstearat | 40,00 g |
| Wasser (steril) | q.s. |

Das pulverige Material wird durch ein Sieb mit Oeffnungen von 0,6 mm gedrückt. Anschliessend mischt man in einem Mischer den Wirkstoff mit der Lactose, dem Magnesiumstearat und der halben Menge Stärke. Die andere halbe Menge Stärke wird in 65 ml Wasser suspendiert, und diese Suspension wird zu der siedenden Lösung des Polyethylenglycols in 260 ml Wasser gegeben. Die gebildete Paste wird zu der Pulvermischung gegeben, welche gegebenenfalls mit weiterem Wasser granuliert wird. Man trocknet das Granulat über Nacht bei 35°C, treibt dieses durch ein Sieb mit 1,2 mm weiten Oeffnungen und komprimiert zu konkaven Tabletten mit oberer Bruchkerbe.

Auf analoge Weise werden Tabletten hergestellt, die etwa 1—50 mg einer der anderen vorstehend offenbarten Verbindungen enthalten.

b) Herstellung von 1000 Kapseln mit je 5 mg Wirkstoff:

Bestandteile

| | |
|---|---|
| cis 4-[1-(3-Phosphonoprop-1-enyl)]-piperidin-2-carbonsäure | 5,00 g |
| Lactose | 212,00 g |
| Stärke | 80,00 g |
| Magnesiumstearat | 3,00 g |

Jedes Pulver wird durch ein Sieb mit Oeffnungen von 0,6 mm Durchmesser gedrückt. Anschliessend mischt man in einem Mischer den Wirkstoff mit dem Magnesiumstearat, dann mit der Lactose und der Stärke, bis man eine homogene Masse erhält. Man füllt Nr. 2 Hartgelatinkapseln mit jeweils 300 mg der vorbereiteten Mischung.

Analog kann man Kapseln, welche 1—50 mg einer der anderen vorstehend offenbarten Verbindungen enthälten, herstellen.

**Patentansprüche für die Vertragsstaaten: AT BE CH DE FR GB IT LI LU NL SE**

1. Verbindungen der Formel I,

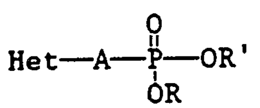

(I)

worin Het eine 2-$R^1$-Pyrrolidinyl-, 2-$R^1$,2,5-Dihydropyrrolyl-, 2-$R^1$-1,2,3,6-Tetrahydropyridinyl-, 2-$R^1$-1,2,5,6-Tetrahydropyridinyl-, 2-$R^1$-Piperidinyl-, 2-$R^1$-Tetrahydrochinolinyl-, 2-$R^1$-Perhydrochinolinyl-, 2-$R^1$-2,3-Dihydroindolyl- oder 2-$R^1$-Perhydroindolylgruppe bedeutet, welche auch durch $C_1$—$C_4$-Alkyl, Phenyl-$C_1$—$C_4$-alkyl, $C_2$—$C_7$-Alkoxycarbonyl, Benzyloxycarbonyl oder $C_2$—$C_7$-Alkanoyl N-substituiert und/oder im heterocyclischen 5- oder 6-Ring durch $C_1$—$C_4$-Alkyl oder Phenyl-$C_1$—$C_4$-alkyl bzw. im gegebenenfalls vorhandenen ankondensierten Benzo- oder Cyclohexanorest durch $C_1$—$C_4$-Alkyl, $C_1$—$C_4$-Alkoxy, Halogen oder Trifluormethyl C-substitiert sein kann, $R^1$ Carboxy, $C_1$—$C_4$-Alkoxycarbonyl, Carbamyl oder N-Mono- oder N,N-Di-$C_1$—$C_4$-alkylcarbamyl bedeutet, A $C_2$—$C_4$-Alkenylen bedeutet und R und R′ unabhängig voneinander Wasserstoff, $C_1$—$C_4$-Alkyl, Benzyl, im Phenylteil durch Halogen, $C_1$—$C_4$-Alkyl oder $C_1$—$C_4$-Alkoxy substituiertes Benzyl, $C_2$—$C_7$-Alkanoyloxymethyl oder im Oxymethylteil durch $C_1$—$C_4$-Alkyl oder $C_3$—$C_8$-Cycloalkyl substituiertes $C_2$—$C_7$-Alkanoyloxymethyl darstellen; und ihre Salze.

2. Verbindungen gemäss Anspruch 1 der Formel II

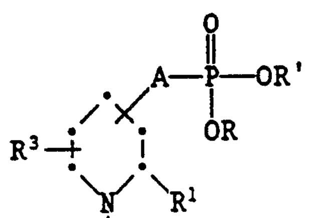

(II),

und Verbindungen der Formel II mit einer Doppelbindung zwischen C-3 und C-4 oder zwischen C-4 und C-5 des Piperidinylringes worin R und R′ unabhängig voneinander Wasserstoff, $C_1$—$C_4$-Alkyl, Benzyl, im Phenylteil durch Halogen, $C_1$—$C_4$-Alkyl oder $C_1$—$C_4$-Alkoxy substituiertes Benzyl, $C_2$—$C_7$-Alkanoyloxymethyl oder im Oxymethylteil durch $C_1$—$C_4$-Alkyl oder $C_3$—$C_8$-Cycloalkyl substituiertes $C_2$—$C_7$-Alkanoyloxymethyl darstellen, $R^1$ Carboxy, $C_1$—$C_4$-Alkoxycarbonyl, Carbamyl oder N-Mono- oder N,N-Di-$C_1$—$C_4$-alkylcarbamyl bedeutet, $R^2$ Wasserstoff, $C_1$—$C_4$-Alkyl, Phenyl-$C_1$—$C_4$-alkyl, $C_2$—$C_7$-Alkoxycarbonyl, Benzyloxycarbonyl oder $C_2$—$C_7$-Alkanoyl darstellt, $R^3$ Wasserstoff, $C_1$—$C_4$-Alkyl oder Phenyl-$C_1$—$C_4$-alkyl bedeutet und A $C_2$—$C_4$-Alkenylen bedeutet; und ihre Salze.

3. Verbindungen der Formel II gemäss Anspruch 2, worin R und R′ unabhängig voneinander

Wasserstoff, $C_1$—$C_4$-Alkyl, Benzyl, $C_2$—$C_7$-Alkanoyloxymethyl oder $C_2$—$C_7$-Alkanoyloxymethyl, welches am Oxymethylteil durch $C_1$—$C_4$-Alkyl, Cyclohexyl oder Cyclopentyl substituiert ist, bedeuten, $R^1$ Carboxy, Carbamoyl oder $C_1$—$C_4$-Alkoxycarbonyl bedeutet; $R^2$ und $R^3$ Wasserstoff oder $C_1$—$C_4$-Alkyl bedeuten und A Alkenylen mit 2 bis 4 Kohlenstoffatomen bedeutet; sowie pharmazeutisch annehmbare Salze davon.

4. Verbindungen der Formel II gemäss Anspruch 2, worin R und R' unabhängig voneinander Wasserstoff, $C_2$—$C_7$-Alkanoyloxymethyl oder $C_2$—$C_7$-Alkanoyloxymethyl, welches am Oxymethylteil durch $C_1$—$C_4$-Alkyl substituiert ist, bedeuten, $R^1$ Carboxy, Carbamoyl oder $C_1$—$C_4$-Alkoxycarbonyl bedeutet, $R^2$ und $R^3$ Wasserstoff bezeichnen, A sich in 4-Position befindet und Alkenylen mit 3 oder 4 Kohlenstoffatom und der Doppelbindung benachbart zur Phosphonogruppe bedeutet; sowie pharmazeutisch annehmbare Salze davon.

5. Verbindungen gemäss Anspruch 4 der Formel III

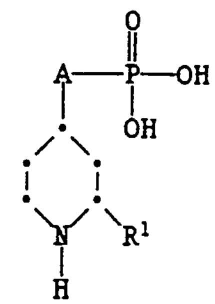

(III),

worin A 1,3-Propenylen bedeutet, vorzugsweise mit der Doppelbindung benachbart zur Phosphonogruppe, und $R^1$ Carboxy oder $C_1$—$C_4$-Alkoxycarbonyl bedeutet; sowie pharmazeutisch annehmbare Salze davon.

6. 4-[1-(3-Phosphonoprop-2-enyl)]-piperidin-2-carbonsäure gemäss Anspruch 1 und pharmazeutisch annehmbare Salze davon.

7. 4-[1-(3-Phosphonoprop-1-enyl)]-piperidin-2-carbonsäure gemäss Anspruch 1 und pharmazeutisch annehmbare Salze davon.

8. Verbindungen gemäss Anspruch 1 der Formel IV

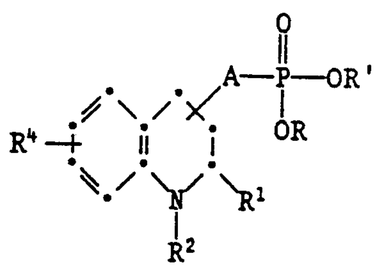

(IV)

oder Perhydroderivate davon, worin R und R' unabhängig voneinander Wasserstoff, $C_1$—$C_4$-Alkyl, Benzyl oder Benzyl, welches am Phenylteil durch Halogen, $C_1$—$C_4$-Alkyl oder $C_1$—$C_4$-Alkoxy substituiert ist, $C_2$—$C_7$-Alkanoyloxymethyl oder $C_2$—$C_7$-Alkanoyloxymethyl, welches am Oxymethylteil durch $C_1$—$C_4$-Alkyl oder $C_3$—$C_8$-Cycloalkyl substituiert ist, bedeutet, $R^1$ Carboxy $C_1$—$C_4$-Alkoxycarbonyl, Carbamyl oder N-Mono- oder N,N'-Di-$C_1$—$C_4$-Alkylcarbamyl bedeutet, $R^2$ Wasserstoff, $C_1$—$C_4$-Alkyl, Phenyl-$C_1$—$C_4$-alkyl, $C_1$—$C_7$-Alkoxycarbonyl, Benzyloxycarbonyl oder $C_2$—$C_7$-Alkanoyl bedeutet; $R^4$ Wasserstoff, $C_1$—$C_4$-Alkyl, $C_1$—$C_4$-Alkoxy, Halogen oder Trifluormethyl bedeutet und A für $C_2$—$C_4$-Alkenylen steht; sowie Salze davon.

9. Verbindungen gemäss Anspruch 8 der Formel V

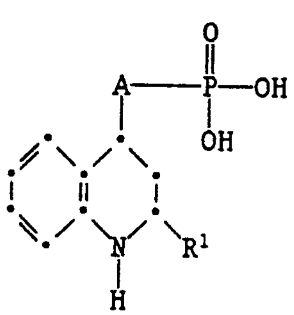

(V)

oder Perhydrochinolin-Derivate davon, worin A 1,3-Propenylen mit der Doppelbindung benachbart zur Phosphonogruppe darstellt und $R^1$ Carboxy oder $C_1$—$C_4$-Alkoxycarbonyl bedeutet; sowie pharmazeutisch annehmbare Salze dieser Verbindungen, die eine salzbildende funktionelle Gruppe aufweisen.

10. Verbindungen gemäss Anspruch 1 der Formel VI

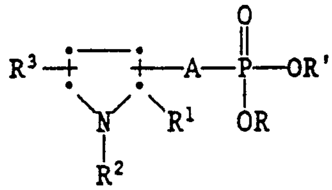

(VI)

und die Verbindungen der Formel VI mit einer Doppelbindung zwischen C-3 und C-4 des Pyrrolidinylringes, worin R und R' uabhängig voneinander Wasserstoff, $C_1$—$C_4$-Alkyl, Benzyl, Benzyl, welches am Phenylteil durch Halogen, $C_1$—$C_4$-Alkyl oder $C_1$—$C_4$-Alkoxy substituiert ist, $C_2$—$C_7$-Alkanoyloxymethyl oder $C_2$—$C_7$-Alkanoyloxymethyl, welches am Oxymethylteil durch $C_1$—$C_4$-Alkyl oder $C_3$—$C_8$-Cycloalkyl substituiert ist, bedeuten, $R^1$ Carboxy $C_1$—$C_4$-Alkoxycarbonyl, Carbamyl oder N-Mono- oder N,N-Di-$C_1$—$C_4$-alkylcarbamyl bedeutet, $R^2$ Wasserstoff, $C_1$—$C_4$-Alkyl, $C_1$—$C_7$-Alkoxycarbonyl, Benzyloxycarbonyl oder $C_2$—$C_7$-Alkanoyl bedeutet, $R^3$ Wasserstoff, $C_1$—$C_4$-Alkyl oder Aryl-$C_1$—$C_4$-alkyl bedeutet und A für $C_2$—$C_4$-Alkenylen steht; sowie Salze davon.

11. Verbindungen der Formel VI gemäss Anspruch 10, worin die Phosphonotragende Gruppe in der Stellung 3 angelagert ist; R und R' Wasserstoff bedeuten, $R^1$ Carboxy oder $C_1$—$C_4$-Alkoxycarbonyl bedeutet, $R^2$ und $R^3$ Wasserstoff bedeuten und A für 1,3-Propenylen mit der Doppelbindung benachbart zur Phospheno-Gruppe bedeutet; sowie pharmazeutisch annehmbare Salze davon.

12. trans-3-[1-(4-Phosphonobut-3-enyl)]-pyrrolidin-2-carbonsäure gemäss Anspruch 1 und pharmazeutisch annehmbare Salze davon.

13. trans-3-[1-(4-Phosphonobut-2-enyl)]-pyrrolidin-2-carbonsäure gemäss Anspruch 1 und pharmazeutisch annehmbare Salze davon.

14. Die in den Ansprüchen 1 bis 13 genannten Verbindungen zur Anwendung in einem Verfahren zur therapeutischen Behandlung des menschlichen oder tierischen Körpers.

15. Pharmazeutische Präparate enthaltend eine Verbindung gemäss einem der Ansprüche 1 bis 14 zusammen mit einem pharmazeutisch geeigneten Trägermaterial.

16. Die in den Ansprüchen 1 bis 14 genannten Verbindungen als NMDA-Antagonisten.

17. Verwendung der in den Ansprüchen 1 bis 14 genannten Verbindungen zur Herstellung von pharmazeutischen Präparaten.

18. Verwendung der in den Ansprüchen 1 bis 14 genannten Verbindungen zur Herstellung von pharmazeutischen Präparaten zur Verwendung als NMDA-Antagonisten.

19. Verbindungen gemäss Anspruch 5 ausgewählt aus der Gruppe bestehend aus cis-4-[1-(3-Phosphonoprop-2-enyl)]-piperidin-2-carbonsäure, trans 4-[1-(3-Phosphonoprop-2-enyl)]-piperidin-2-carbonsäure, cis 4-[1-(3-Phosphonoprop-1-enyl)]-piperidin-2-carbonsäure und trans 4-[1-(3-Phosphonoprop-1-enyl)]-piperidin-2-carbonsäure und pharmazeutisch annehmbare Salze davon.

20. Verbindungen gemäss Anspruch 10 ausgewählt aus cis 3-[1-(4-Phosphonobut-3-enyl)]-pyrrolidin-2-carbonsäure und pharmazeutisch annehmbaren Salzen davon.

21. Pharmazeutische Präparate enthaltend eine Verbindung gemäss einem der Ansprüche 19 und 20 zusammen mit einem pharmazeutisch geeigneten Trägermaterial.

22. Die in den Ansprüchen 19 und 20 genannten Verbindungen zur Anwendung in einem Verfahren zur therapeutischen Behandlung des menschlichen oder tierischen Körpers.

23. Die in den Ansprüchen 19 und 20 genannten Verbindungen als NMDA-Antagonisten.

24. Verwendung der in den Ansprüchen 19 und 20 genannten Verbindungen zur Herstellung von pharmazeutischen Präparaten.

25. Verwendung der in den Ansprüchen 19 und 20 genannten Verbindungen zur Herstellung von pharmazeutischen Präparaten zur Verwendung als NMDA-Antagonisten.

26. Verfahren zur Herstellung von Verbindungen gemäss Anspruch 1 der Formel I, in der alle Symbole die in Anspruch 1 angegebenen Bedeutungen haben, und deren Salzen, gekennzeichnet durch

a) Kondensation eines Aldehydes oder Ketons der Formel VII

$$\text{Het—A'} \qquad \text{(VII)}$$

worin Het und $R^1$ wie für Formel I definiert sind, wobei aber $R^1$ und Aminogruppen in geschützter Form vorliegen, und A' $C_1$—$C_3$-Alkyl bedeutet, welches durch Oxo substituiert ist und ein Kohlenstoffatom weniger aufweist als die Alkenylengruppe A, mit einem Tetraesterderivat von Methylendiphosphonsäure unter basischen Bedingungen und erforderlichenfalls durch Abspalten von Schutzgruppen von dem erhaltenen Produkt, so dass eine Verbindung der Formel I erhalten wird, worin die Doppelbindung innerhalb der Gruppe A der Phosphonogruppe unmittelbar benachbart ist; oder

b) Kondensation einer Verbindung der Formel VIII,

$$\text{Het—A—X} \qquad \text{(VIII)}$$

worin Het, A und $R^1$ wie für Formel I definiert sind und X reaktionsfähiges verestertes Hydroxy darstellt, mit einer Verbindung, die die Phosphonsäurehälfte einführen kann, und die eine der Formeln IX oder X

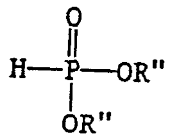 (IX)

$P(R''')_3$ (X)

aufweist, worin R'' $C_1$—$C_4$-Alkyl bezeichnet und R''' Halogen oder $C_1$—$C_4$-Alkoxy ist, und erforderlichenfalls Umwandlung des entstehenden Phosphonsäurederivats in die Phosphonsäure oder ein anderes Esterderivat derselben; oder

c) Umwandlung in $R^1$ eines anderen Substituenten als $R^1$ in Stellung 2 am heterocyclischen Ring in einer Verbindung, die sonst identisch mit einer erfindungsgemässen ist; ferner durch Ausführung dieser Verfahren mit erforderlichenfalls vorübergehendem Schutz aller störenden reaktionsfähigen Gruppen und anschliessender Freisetzung der entstehenden erfindungsgemässen Verbindung; gewünschtenfalls Umwandlung einer entstehenden erfindungsgemässen Verbindung in eine andere erfindungsgemässe Verbindung und/oder gewünschtenfalls Umwandlung der entstehenden freien Verbindung in ein Salz oder eines entstehenden Salzes in die freie Verbindung oder ein anderes Salz; und/oder Trennen eines Isomerengemisches oder Gemisches von Racematen in die einzelnen Isomere oder Racemate; und/oder gewünschtenfalls Trennung eines Racemats in die optischen Antipoden.

27. Verfahren nach Anspruch 26, dadurch gekennzeichnet, dass man eine Verbindung der Formel I, worin Het durch $C_2$—$C_7$-Alkanoyl oder $C_2$—$C_7$-Alkoxycarbonyl N-substituiert ist, R und R' für $C_1$—$C_4$-Alkyl, Benzyl, Benzyl, welches am Phenylteil durch Halogen, $C_1$—$C_4$-Alkyl oder $C_1$—$C_4$-Alkoxy substituiert ist, $C_2$—$C_7$-Alkanoyloxymethyl oder $C_2$—$C_7$-Alkanoyloxymethyl, welches am Oxymethylteil durch $C_1$—$C_4$-Alkyl oder $C_3$—$C_8$-Cycloalkyl substituiert ist, stehen, und $R^1$ $C_1$—$C_4$-Alkoxycarbonyl, Carbamyl oder N-Mono- oder N,N-Di-$C_1$—$C_4$-alkylcarbonyl bedeutet, durch Behandeln mit einer anorganischen Säure oder mit wässrigen Alkalien in eine entsprechende N-unsubstituierte Verbindung der Formel I überführt, worin R und R' Wasserstoff bedeuten und $R^1$ Carboxy bedeutet.

28. Verfahren nach Anspruch 26, dadurch gekennzeichnet, dass man eine Verbindung der Formel I, worin Het durch $C_2$—$C_7$-Alkanoyl oder $C_2$—$C_7$-Alkoxycarbonyl N-substituiert ist, R und R' $C_1$—$C_4$-Alkyl bedeuten und $R^1$ $C_1$—$C_4$-Alkoxycarbonyl bedeutet, durch Behandeln mit einer anorganischen Säure oder mit wässrigen Alkalien in eine entsprechende N-unsubstituierte Verbindung der Formel I überführt, worin R und R' Wasserstoff bedeuten und $R^1$ Carboxy bedeutet.

**Patentansprüche für die Vertragsstaaten: ES GR**

1. Verfahren zur Herstellung von Verbindungen gemäss Anspruch 1 der Formel I,

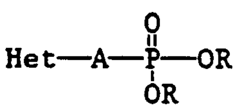 (I)

worin Het eine 2-$R^1$-Pyrrolidinyl-, 2-$R^1$-2,5-Dihydropyrrolyl-, 2-$R^1$-1,2,3,6-Tetrahydropyridinyl-, 2-$R^1$-1,2,5,6-Tetrahydropyridinyl-, 2-$R^1$-Piperidinyl-, 2-$R^1$-Tetrahydrochinolinyl-, 2-$R^1$-Perhydrochinolinyl-, 2-$R^1$-2,3-Dihydroindolyl- oder 2-$R^1$-Perhydroindolylgruppe bedeutet, welche auch durch $C_1$—$C_4$-Alkyl, Phenyl-$C_1$—$C_4$-alkyl, $C_2$—$C_7$-Alkoxycarbonyl oder $C_2$—$C_7$-Alkanoyl N-substituiert und/oder im heterocyclischen 5- oder 6-Ring durch $C_1$—$C_4$-Alkyl oder Phenyl-$C_1$—$C_4$-alkyl bzw. im gegebenenfalls vorhandenen ankondensierten Benzo- oder Cyclohexanorest durch $C_1$—$C_4$-Alkyl, $C_1$—$C_4$-Alkoxy, Halogen oder Trifluormethyl C-substitiert sein kann, $R^1$ Carboxy, $C_1$—$C_4$-Alkoxycarbonyl, Carbamyl oder N-Mono- oder N,N-Di-$C_1$—$C_4$-alkylcarbamyl bedeutet, A $C_2$—$C_4$-Alkenylen bedeutet und R und R' unabhängig voneinander Wasserstoff, $C_1$—$C_4$-Alkyl, Benzyl, im Phenylteil durch Halogen, $C_1$—$C_4$-Alkyl oder $C_1$—$C_4$-Alkoxy substituiertes Benzyl, $C_2$—$C_7$-Alkanoyloxymethyl oder im Oxymethylteil durch $C_1$—$C_4$-Alkyl oder $C_3$—$C_8$-Cycloalkyl substituiertes $C_2$—$C_7$-Alkanoyloxymethyl darstellen; und ihre Salze, gekennzeichnet durch

a) Kondensation eines Aldehydes oder Ketons der Formel VII

Het—A' (VII)

worin Het und $R^1$ wie für Formel I definiert sind, wobei aber $R^1$ und Aminogruppen in geschützter Form vorliegen, und A' $C_1$—$C_3$-Alkyl bedeutet, welches durch Oxo substituiert ist und ein Kohlenstoffatom weniger aufweist als die Alkenylengruppe A, mit einem Tetraesterderivat von Methylendiphosphonsäure unter basischen Bedingungen und erforderlichenfalls durch Abspalten von Schutzgruppen von dem erhaltenen Produkt, so dass eine Verbindung der Formel I erhalten wird, worin die Doppelbindung innerhalb der Gruppe A der Phosphonogruppe unmittelbar benachbart ist; oder

b) Kondensation einer Verbindung der Formel VIII,

Het—A—X (VIII)

worin Het, A und $R^1$ wie für Formel I definiert sind und X reaktionsfähiges verestertes Hydroxy darstellt, mit einer Verbindung, die die Phosphonsäurehälfte einführen kann, und die eine der Formeln IX oder X

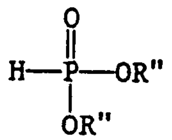 (IX) $P(R''')_3$ (X)

aufweist, worin R'' $C_1$—$C_4$-Alkyl bezeichnet und R''' Halogen oder $C_1$—$C_4$-Alkoxy ist, und erforderlichenfalls Umwandlung des entstehenden Phosphonsäurederivats in die Phosphonsäure oder ein anderes Esterderivat derselben; oder

c) Umwandlung in $R^1$ eines anderen Substituenten als $R^1$ in Stellung 2 am heterocyclischen Ring in einer Verbindung, die sonst identisch mit einer erfindungsgemässen ist; ferner durch Ausführung dieser Verfahren mit erforderlichenfalls vorübergehendem Schutz aller störenden reaktionsfähigen Gruppen und anschliessender Freisetzung der entstehenden erfindungsgemässen Verbindung; gewünschtenfalls Umwandlung einer entstehenden erfindungsgemässen Verbindung in eine andere erfindungsgemässe Verbindung und/oder gewünschtenfalls Umwandlung der entstehenden freien Verbindung in ein Salz oder eines entstehenden Salzes in die freie Verbindung oder ein anderes Salz; und/oder Trennen eines Isomerengemisches oder Gemisches von Racematen in die einzelnen Isomere oder Racemate; und/oder gewünschtenfalls Trennung eines Racemats in die optischen Antipoden.

2. Verfahren nach Anspruch 1, dadurch gekennzeichnet, dass man eine Verbindung der Formel I, worin Het durch $C_2$—$C_7$-Alkanoyl oder $C_2$—$C_7$-Alkoxycarbonyl N-substituiert ist, R und R' für $C_1$—$C_4$-Alkyl, Benzyl, Benzyl, welches am Phenylteil durch Halogen, $C_1$—$C_4$-Alkyl oder $C_1$—$C_4$-Alkoxy substituiert ist, $C_2$—$C_7$-Alkanoyloxymethyl oder $C_2$—$C_7$-Alkanoyloxymethyl, welches am Oxymethylteil durch $C_1$—$C_4$-Alkyl oder $C_3$—$C_8$-Cycloalkyl substituiert ist, stehen, und $R^1$ $C_1$—$C_4$-Alkoxycarbonyl, Carbamyl oder N-Mono- oder N,N-Di-$C_1$—$C_4$-alkylcarbonyl bedeutet, durch Behandeln mit einer anorganischen Säure oder mit wässrigen Alkalien in eine entsprechende N-unsubstituierte Verbindung der Formel I überführt, worin R und R' Wasserstoff bedeuten und $R^1$ Carboxy bedeutet.

3. Verfahren nach Anspruch 1, dadurch gekennzeichnet, dass man eine Verbindung der Formel I, worin Het durch $C_2$—$C_7$-Alkanoyl oder $C_2$—$C_7$-Alkoxycarbonyl N-substituiert ist, R und R' $C_1$—$C_4$-Alkyl bedeuten und $R^1$ $C_1$—$C_4$-Alkoxycarbonyl bedeutet, durch Behandeln mit einer anorganischen Säure oder mit wässrigen Alkalien in eine entsprechende N-unsubstituierte Verbindung der Formel I überführt, worin R und R' Wasserstoff bedeuten und $R^1$ Carboxy bedeutet.

4. Verfahren gemäss einem der Ansprüche 1 bis 3, dadurch gekennzeichnet, dass man Verbindungen der Formel II

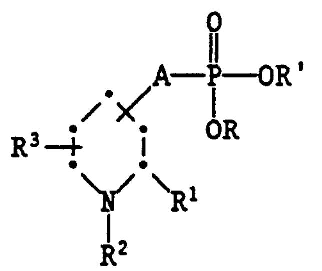 (II),

oder Verbindungen der Formel II mit einer Doppelbindung zwischen C-3 und C-4 oder zwischen C-4 und C-5 des Piperidinylringes worin R und R' unabhängig voneinander Wasserstoff, $C_1$—$C_4$-Alkyl, Benzyl, im Phenylteil durch Halogen, $C_1$—$C_4$-Alkyl oder $C_1$—$C_4$-Alkoxy substituiertes Benzyl, $C_2$—$C_7$-Alkanoyloxymethyl oder im Oxymethylteil durch $C_1$—$C_4$-Alkyl oder $C_3$—$C_8$-Cycloalkyl substituiertes $C_2$—$C_7$-Alkanoyloxymethyl darstellen, $R^1$ Carboxy, $C_1$—$C_4$-Alkoxycarbonyl, Carbamyl oder N-Mono- oder N,N-Di-$C_1$—$C_4$-alkylcarbamyl bedeutet, $R^2$ Wasserstoff, $C_1$—$C_4$-Alkyl, Phenyl-$C_1$—$C_4$-alkyl, $C_2$—$C_7$-Alkoxycarbonyl, Benzyloxycarbonyl oder $C_2$—$C_7$-Alkanoyl darstellt, $R^3$ Wasserstoff, $C_1$—$C_4$-Alkyl oder Phenyl-$C_1$—$C_4$-alkyl bedeutet und A $C_2$—$C_4$-Alkenylen bedeutet; und ihre Salze herstellt.

5. Verfahren gemäss einem der Ansprüche 1 bis 3, dadurch gekennzeichnet, dass man Verbindungen der Formel II gemäss Anspruch 2, worin R und R' unabhängig voneinander Wasserstoff, $C_1$—$C_4$-Alkyl, Benzyl, $C_2$—$C_7$-Alkanoyloxymethyl oder $C_2$—$C_7$-Alkanoyloxymethyl, welches am Oxymethylteil durch $C_1$—$C_4$-Alkyl, Cyclohexyl oder Cyclopentyl substituiert ist, bedeuten, $R^1$ Carboxy, Carbamoyl oder $C_1$—$C_4$-Alkoxycarbonyl bedeutet; $R^2$ und $R^3$ Wasserstoff oder $C_1$—$C_4$-Alkyl bedeuten und A Alkenylen mit 2 bis 4 Kohlenstoffatomen bedeutet; oder pharmazeutisch annehmbare Salze davon herstellt.

6. Verfahren gemäss einem der Ansprüche 1 bis 3, dadurch gekennzeichnet, dass man Verbindungen der Formel II gemäss Anspruch 2, worin R und R' unabhängig voneinander Wasserstoff, $C_2$—$C_7$-Alkanoyloxymethyl oder $C_2$—$C_7$-Alkanoyloxymethyl, welches am Oxymethylteil durch $C_1$—$C_4$-Alkyl substituiert ist, bedeuten $R^1$ Carboxy, Carbamoyl oder $C_1$—$C_4$-Alkoxycarbonyl bedeutet, $R^2$ und $R^3$ Wasserstoff bezeichnen, A sich in 4-Position befindet und Alkenylen mit 3 oder 4 Kohlenstoffatomen und der Doppelbindung benachbart zur Phosphonogruppe bedeutet; oder pharmazeutisch annehmbare Salze davon herstellt.

7. Verfahren gemäss einem der Ansprüche 1 bis 3 und 6, dadurch gekennzeichnet, dass man Verbindungen der Formel III

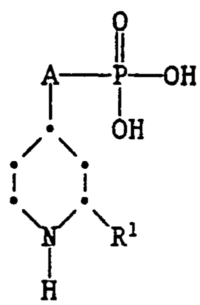

(III),

worin A 1,3-Propenylen bedeutet, vorzugsweise mit der Doppelbindung benachbart zur Phosphonogruppe, und $R^1$ Carboxy oder $C_1$—$C_4$-Alkoxycarbonyl bedeutet; oder pharmazeutisch annehmbare Salze davon herstellt.

8. Verfahren gemäss einem der Ansprüche 1 bis 3, dadurch gekennzeichnet, dass man 4-[1-(3-Phosphonoprop-2-enyl)]-piperidin-2-carbonsäure oder pharmazeutisch annehmbare Salze davon herstellt.

9. Verfahren gemäss einem der Ansprüche 1 bis 3, dadurch gekennzeichnet, dass man 4-[1-(3-Phosphonoprop-1-enyl)]-piperidin-2-carbonsäure oder pharmazeutisch annehmbare Salze davon herstellt.

10. Verfahren gemäss einem der Ansprüche 1 bis 3, dadurch gekennzeichnet, dass man Verbindungen gemäss Anspruch 1 der Formel IV

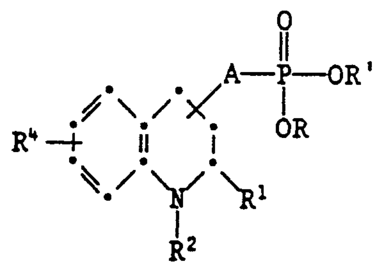

(IV)

oder Perhydroderivate davon, worin R und R' unabhängig voneinander Wasserstoff, $C_1$—$C_4$-Alkyl, Benzyl oder Benzyl, welches am Phenylteil durch Halogen, $C_1$—$C_4$-Alkyl oder $C_1$—$C_4$-Alkoxy substituiert ist, $C_2$—$C_7$-Alkanoyloxymethyl oder $C_2$—$C_7$-Alkanoyloxymethyl, welches am Oxymethylteil durch $C_1$—$C_4$-Alkyl oder $C_3$—$C_8$-Cycloalkyl substituiert ist, bedeutet, $R^1$ Carboxy $C_1$—$C_4$-Alkoxycarbonyl, Carbamyl oder N-Mono- oder N,N'-Di-$C_1$—$C_4$-Alkylcarbamyl bedeutet, $R^2$ Wasserstoff, $C_1$—$C_4$-Alkyl, Phenyl-$C_1$—$C_4$-alkyl, $C_1$—$C_7$-Alkoxycarbonyl, Benzyloxycarbonyl oder $C_2$—$C_7$-Alkanoyl bedeutet; $R^4$ Wasserstoff, $C_1$—$C_4$-Alkyl, $C_1$—$C_4$-Alkoxy, Halogen oder Trifluormethyl bedeutet und A für $C_2$—$C_4$-Alkenylen steht; oder Salze davon herstellt.

11. Verfahren gemäss einem der Ansprüche 1 bis 3, dadurch gekennzeichnet dass man Verbindungen der Formel V

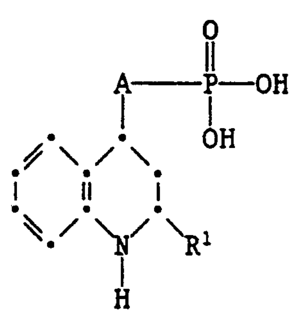

(V)

oder Perhydrochinolin-Derivate davon, worin A 1,3-Propenylen mit der Doppelbindung benachbart zur Phosphonogruppe darstellt und $R^1$ Carboxy oder $C_1$—$C_4$-Alkoxycarbonyl bedeutet; oder pharmazeutisch annehmbare Salze dieser Verbindungen, die eine salzbildende funktionelle Gruppe aufweisen, herstellt.

12. Verfahren gemäss einem der Ansprüche 1 bis 3, dadurch gekennzeichnet dass man Verbindungen der Formel VI

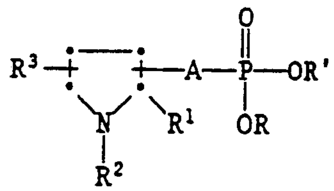

(VI)

oder die Verbindungen der Formel VI mit einer Doppelbindung zwischen C-3 und C-4 des Pyrrolidinylringes, worin R und R' uabhängig voneinander Wasserstoff, $C_1$—$C_4$-Alkyl, Benzyl, Benzyl, welches am Phenylteil durch Halogen, $C_1$—$C_4$-Alkyl oder $C_1$—$C_4$-Alkoxy substituiert ist, $C_2$—$C_7$-Alkanoyloxymethyl oder $C_2$—$C_7$-Alkanoyloxymethyl, welches am Oxymethylteil durch $C_1$—$C_4$-Alkyl oder $C_3$—$C_8$-Cycloalkyl substituiert ist, bedeuten, $R^1$ Carboxy $C_1$—$C_4$-Alkoxycarbonyl, Carbamyl oder N-Mono- oder N,N-Di-$C_1$—$C_4$-alkylcarbamyl bedeutet, $R^2$ Wasserstoff, $C_1$—$C_4$-Alkyl, $C_1$—$C_7$-Alkoxycarbonyl, Benzyloxycarbonyl oder $C_2$—$C_7$-Alkanoyl bedeutet, $R^3$ Wasserstoff, $C_1$—$C_4$-Alkyl oder Aryl-$C_1$—$C_4$-alkyl bedeutet und A für $C_2$—$C_4$-Alkenylen steht; oder Salze davon herstellt.

13. Verfahren gemäss einem der Ansprüche 1 bis 3, dadurch gekennzeichnet, dass man Verbindungen der Formel VI gemäss Anspruch 12, worin die Phosphono-tragende Gruppe in der Stellung 3 angelagert ist; R und R' Wasserstoff bedeuten, $R^1$ Carboxy oder $C_1$—$C_4$-Alkoxycarbonyl bedeutet, $R^2$ und $R^3$ Wasserstoff bedeuten und A für 1,3-Propenylen mit der Doppelbindung benachbart zur Phosphono-Gruppe bedeutet; oder pharmazeutisch annehmbare Salze davon herstellt.

14. Verfahren gemäss einem der Ansprüche 1 bis 3, dadurch gekennzeichnet, dass man trans-3-[1-(4-Phosphonobut-3-enyl)]-pyrrolidin-2-carbonsäure oder pharmazeutisch annehmbare Salze davon herstellt.

15. Verfahren gemäss einem der Ansprüche 1 bis 3, dadurch gekennzeichnet, dass man trans-3-[1-(4-Phosphonobut-2-enyl)]-pyrrolidin-2-carbonsäure oder pharmazeutisch annehmbare Salze davon herstellt.

16. Verfahren zur Herstellung von pharmazeutischen Präparaten, dadurch gekennzeichnet, dass man eine Verbindung, erhältlich gemäss einem der Ansprüche 1 bis 15 mit einem pharmazeutisch geeigneten Trägermaterial vermischt.

17. Verfahren gemäss Anspruch 16 zur Herstellung von pharmazeutischen Präparaten zur Verwendung als NMDA-Antagonisten.

18. Verfahren gemäss einem der Ansprüche 1 bis 3 zur Herstellung von Verbindungen ausgewählt aus der Gruppe bestehend aus cis-4-[1-(3-Phosphonoprop-2-enyl)]-piperidin-2-carbonsäure, trans 4-[1-(3-Phosphonoprop-2-enyl)]-piperidin-2-carbonsäure, cis 4-[1-(3-Phosphonoprop-1-enyl)]-piperidin-2-carbonsäure und trans 4-[1-(3-Phosphonoprop-1-enyl)]-piperidin-2-carbonsäure und pharmazeutisch annehmbaren Salze davon.

19. Verfahren gemäss einem der Ansprüche 1 bis 3 zur Herstellung von cis 3-[1-(4-Phosphonobut-3-enyl)]-pyrrolidin-2-carbonsäure und pharmazeutisch annehmbarer Salze davon.

20. Verfahren zur Herstellung von pharmazeutischen Präparaten, dadurch gekennzeichnet, dass man eine Verbindung, erhältlich gemäss einem der Ansprüche 18 und 19 zusammen mit einem pharmazeutisch geeigneten Trägermaterial vermischt.

21. Verfahren gemäss Anspruch 20 zur Herstellung von pharmazeutischen Präparaten zur Verwendung als NMDA-Antagonisten.

**Revendications pour les Etats contractants: AT BE CH DE FR GB IT LI LU NL SE**

1. Composés de formule I

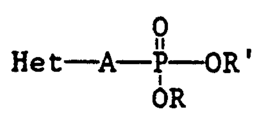

(I)

dans laquelle Het représente un groupe 2-$R^1$-pyrrolidinyle, 2-$R^1$-2,5-dihydropyrrolyle, 2-$R^1$-1,2,3,6-tétrahydropyridinyle, 2-$R^1$-1,2,5,6-tétrahydropyridinyle, 2-$R^1$-pipéridinyle, 2-$R^1$-tétrahydroquinoléinyle, 2-$R^1$-perhydroquinoléinyle, 2-$R^1$-2,3-dihydroindolyle ou 2-$R^1$-perhydroindolyle qui peut également être substitué à l'azote par des groupes alkyle en $C_1$—$C_4$, phényl-alkyle en $C_1$—$C_4$, alcoxycarbonyle en $C_2$—$C_7$, benzyloxycarbonyle ou alcanoyle en $C_2$—$C_7$ et/ou substitué sur le carbone dans le noyau hétérocyclique à 5 ou 6 chaînons par des groupes alkyle en $C_1$—$C_4$ ou phénylalkyle en $C_1$—$C_4$ et/ou substitué sur le carbone dans un radical benzo ou cyclohexano condensé éventuel par des groupes alkyle en $C_1$—$C_4$, alcoxy en $C_1$—$C_4$, des halogènes ou des groupes trifluorométhyle, $R^1$ représente un groupe carboxy, (alcoxy en $C_1$—$C_4$)-carbonyle, carbamyle ou N-mono- ou N,N-di-(alkyle en $C_1$—$C_4$)-carbamyle, A représente un groupe alcénylène en $C_2$—$C_4$ et R et R' représentent chacun, indépendamment l'un de l'autre, l'hydrogène, un groupe alkyle en $C_1$—$C_4$, benzyle, benzyle substitué dans la partie phényle par des halogènes, des groupes alkyle en $C_1$—$C_4$ ou alcoxy en $C_1$—$C_4$, alcanoyloxyméthyle en $C_2$—$C_7$ ou alcanoyloxyméthyle en $C_2$—$C_7$ substitué dans la partie oxyméthyle par un groupe alkyle en $C_1$—$C_4$ ou cycloalkyle en $C_3$—$C_8$; et leurs sels.

2. Composés selon la revendication 1, de formule II

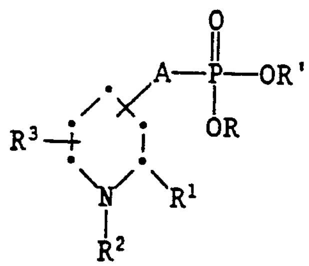

(II),

et composés de formule II contenant une double liaison entre les carbones C-3 et C-4 ou entre les carbones C-4 et C-5 du cycle pipéridinyle, pour lesquels R et R′ représentent chacun, indépendamment l'un de l'autre, l'hydrogène, un groupe alkyle en $C_1$—$C_4$, benzyle, benzyle substitué dans la partie phényle par des halogènes, des groupes alkyle en $C_1$—$C_4$ ou alcoxy en $C_1$—$C_4$, alcanoyloxyméthyle en $C_2$—$C_7$ ou alcanoyloxyméthyle en $C_2$—$C_7$ substitué dans la partie oxyméthyle par un groupe alkyle en $C_1$—$C_4$ ou cycloalkyle en $C_3$—$C_8$, $R^1$ représente un groupe carboxy, (alcoxy en $C_1$—$C_4$)-carbonyle, carbamyle ou N-mono- ou N,N-di-(alkyle en $C_1$—$C_4$)-carbamyle, $R^2$ représente l'hydrogène, un groupe alkyle en $C_1$—$C_4$, phénylalkyle en $C_1$—$C_4$, alcoxycarbonyle en $C_2$—$C_7$, benzyloxycarbonyle ou alcanoyle en $C_2$—$C_7$, $R^3$ représente l'hydrogène, un groupe alkyle en $C_1$—$C_4$ ou phényl-alkyle en $C_1$—$C_4$ et A représente un groupe alcénylène en $C_2$—$C_4$; et leurs sels.

3. Composés de formule II selon la revendication 2, dans laquelle R et R′ représentent chacun, indépendamment l'un de l'autre, l'hydrogène, un groupe alkyle en $C_1$—$C_4$, benzyle, alcanoyloxyméthyle en $C_2$—$C_7$ ou alcanoyloxyméthyle en $C_2$—$C_7$ substitué dans la partie oxyméthyle par un groupe alkyle en $C_1$—$C_4$, cyclohexyle ou cyclopentyle, $R^1$ représente un groupe carboxy, carbamoyle ou (alcoxy en $C_1$—$C_4$)-carbonyle, $R^2$ et $R^3$ représentent l'hydrogène ou des groupes alkyle en $C_1$—$C_4$ et A représente un groupe alcénylène en $C_2$—$C_4$; et leurs sels acceptables pour l'usage pharmaceutique.

4. Composés de formule II selon la revendication 2, dans laquelle R et R′ représentent chacun, indépendamment l'un de l'autre, l'hydrogène, un groupe alcanoyloxyméthyle en $C_2$—$C_7$ ou alcanoyloxy-méthyle en $C_2$—$C_7$ substitué dans la partie oxyméthyle par un groupe alkyle en $C_1$—$C_4$, $R^1$ représente un groupe carboxy, carbamoyle ou (alcoxy en $C_1$—$C_4$)-carbonyle, $R^2$ et $R^3$ représentent l'hydrogène, A est en position 4 et représente un groupe alcénylène en $C_3$ ou $C_4$ avec la double liaison voisine du groupe phosphono; et leurs sels acceptables pour l'usage pharmaceutique.

5. Composés selon la revendication 4, de formule III

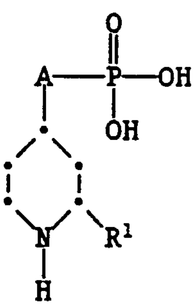

(III),

dans laquelle A représente un groupe 1,3-propénylène, de préférence avec la double liaison voisine du groupe phosphono, et $R^1$ représente un groupe carboxy ou (alcoxy en $C_1$—$C_4$)-carbonyle; et leurs sels acceptables pour l'usage pharmaceutique.

6. L'acide 4-[1-(3-phosphonopropa-2-ényl)]-pipéridine-2-carboxylique selon la revendication 1 et ses sels acceptables pour l'usage pharmaceutique.

7. L'acide 4-[1-(3-phosphonopropa-1-ényl)]-pipéridine-2-carboxylique selon la revendication 1, et ses sels acceptables pour l'usage pharmaceutique.

8. Composés selon la revendication 1, de formule IV

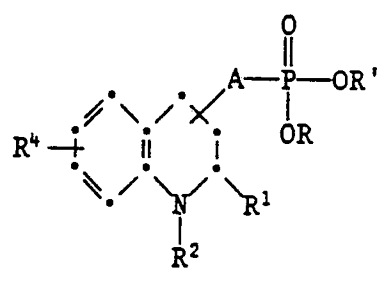

(IV)

ou leurs dérivés perhydrogénés, pour lesquels R et R' représentent chacun, indépendamment l'un de l'autre, l'hydrogène, un groupe alkyle en $C_1$—$C_4$, benzyle ou benzyle substitué dans la partie phényle par des halogènes, des groupes alkyle en $C_1$—$C_4$ ou alcoxy en $C_1$—$C_4$, un groupe alcanoyloxyméthyle en $C_2$—$C_7$ ou alcanoyloxyméthyle en $C_2$—$C_7$ substitué dans la partie oxyméthyle par un groupe alkyle en $C_1$—$C_4$ ou cycloalkyle en $C_3$—$C_8$, $R^1$ représente un groupe carboxy ou (alcoxy en $C_1$—$C_4$)-carbonyle, carbamyle ou N-mono ou N,N'-di-(alkyle en $C_1$—$C_4$)-carbamyle, $R^2$ représente l'hydrogène, un groupe alkyle en $C_1$—$C_4$, phényl-alkyle en $C_1$—$C_4$, (alcoxy en $C_1$—$C_7$)-carbonyle, benzyloxycarbonyle ou alcanoyle en $C_2$—$C_7$; $R^4$ représente l'hydrogène, un groupe alkyle en $C_1$—$C_4$, alcoxy en $C_1$—$C_4$, un halogène ou un groupe trifluorométhyle et A représente un groupe alcénylène en $C_2$—$C_4$; et leurs sels.

9. Composés selon la revendication 8, de formule V

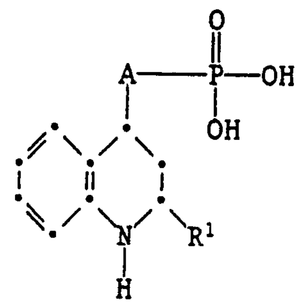

(V)

ou leurs dérivés perhydroquinoléiniques, pour lesquels A représente un groupe 1,3-propénylène dont la double liaison est voisine du groupe phosphono et $R^1$ représente un groupe carboxy ou (alcoxy en $C_1$—$C_4$)-carbonyle; et les sels acceptables pour l'usage pharmaceutique de ces composés qui contiennent un groupe fonctionnel salifiable.

10. Composés selon la revendication 1, de formule VI

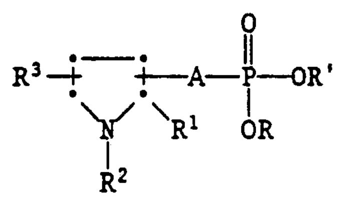

(VI)

et les composés de formule VI contenant une double liaison entre les carbones C-3 et C-4 du cycle pyrrolidinyle, pour lesquels R et R' représentent chacun, indépendamment l'un de l'autre, l'hydrogène, un groupe alkyle en $C_1$—$C_4$, benzyle, benzyle substitué dans la partie phényle par des halogènes, des groupes alkyle en $C_1$—$C_4$ ou alcoxy en $C_1$—$C_4$, un groupe alcanoyloxyméthyle en $C_2$—$C_7$ ou alcanoyloxyméthyle en $C_2$—$C_7$ substitué dans la partie oxyméthyle par un groupe alkyle en $C_1$—$C_4$ ou cycloalkyle en $C_3$—$C_8$, $R^1$ représente un groupe carboxy ou (alcoxy en $C_1$—$C_4$)-carbonyle, carbamyle ou N-mono- ou N,N-di-(alkyle en $C_1$—$C_4$)-carbamyle, $R^2$ représente l'hydrogène, un groupe alkyle en $C_1$—$C_4$, (alcoxy en $C_1$—$C_7$)-carbonyle, benzyloxycarbonyle ou alcanoyle en $C_2$—$C_7$, $R^3$ représente l'hydrogène, un groupe alkyle en $C_1$—$C_4$ ou aryl-alkyle en $C_1$—$C_4$ et A représente un groupe alcénylène en $C_2$—$C_4$; et leurs sels.

11. Composés de formule VI selon la revendication 10, dans lesquels le groupe portant la fonction phosphono est fixé en position 3; R et R' représentent l'hydrogène, $R^1$ représente un groupe carboxy ou (alcoxy en $C_1$—$C_4$)-carbonyle, $R^2$ et $R^3$ représentent l'hydrogène et A un groupe 1,3-propénylène dont la double liaison est voisine du groupe phosphono; et leurs sels acceptables pour l'usage pharmaceutique.

12. L'acide trans-3-[1-(4-phosphonobuta-3-ényl)]-pyrrolidine-2-carboxylique selon la revendication 1 et ses sels acceptables pour l'usage pharmaceutique.

13. L'acide trans-3-[1-(4-phosphonobuta-2-ényl)]-pyrrolidine-2-carboxylique selon la revendication 1, et ses sels acceptables pour l'usage pharmaceutique.

14. Les composés des revendications 1 à 13, pour l'utilisation dans un procédé pour le traitement thérapeutique de l'organisme humain ou animal.

15. Compositions pharmaceutiques contenant un composé selon l'une des revendications 1 à 14 avec un véhicule approprié à l'usage pharmaceutique.

16. Les composés des revendications 1 à 14 en tant qu'antagonistes du NMDA.

17. Utilisation des composés des revendications 1 à 14 pour la préparation de compositions pharmaceutiques.

18. Utilisation des composés des revendications 1 à 14 pour la préparation de compositions pharmaceutiques utilisées en tant qu'antagonistes du NMDA.

19. Composés selon la revendication 5, choisis dans le groupe consistant en l'acide cis-4-[1-(3-phosphonopropa-2-ényl)]-pipéridine-2-carboxylique, l'acide trans-4-[1-(3-phosphonopropa-2-ényl)]-pipéridine-2-carboxylique, l'acide cis-4-[1-(3-phosphonopropa-1-ényl)]-pipéridine-2-carboxylique et l'acide trans-4-[1-(3-phosphonopropa-1-ényl)]-pipéridine-2-carboxylique et leurs sels acceptables pour l'usage pharmaceutique.

20. Composés selon la revendication 10, choisis parmi l'acide cis-3-[1-(4-phosphonobuta-3-ényl)]-pyrrolidine-2-carboxylique et ses sels acceptables pour l'usage pharmaceutique.

21. Compositions pharmaceutiques contenant un composé selon l'une des revendications 19 et 20 avec un véhicule approprié à l'usage pharmaceutique.

22. Les composés des revendications 19 et 20 pour l'utilisation dans un procédé pour le traitement thérapeutique de l'organisme humain ou animal.

23. Les composés des revendications 19 et 20 en tant qu'antagonistes du NMDA.

24. Utilisation des composés des revendications 19 et 20 pour la préparation de compositions pharmaceutiques.

25. Utilisation des composés des revendications 19 et 20 pour la préparation de compositions pharmaceutiques utilisées en tant qu'antagonistes du NMDA.

26. Procédé de préparation des composés de formule I de la revendication 1, dans laquelle tous les symboles ont les significations indiquées dans la revendication 1, et de leurs sels, caractérisé en ce que:

a) on condense un aldéhyde ou une cétone de formule VII

Het—A'　　VII

dans laquelle Het et $R^1$ ont les significations indiquées en référence à la formule I mais $R^1$ et les groupes amino sont à l'état protégé, et A' représente un groupe alkyle en $C_1$—$C_3$ substitué par un groupe oxo et contenant un atome de carbone de moins que le groupe alcénylène A, avec un tétraester de l'acide méthylène-diphosphonique en milieu basique, et lorsque c'est nécessaire, on élimine les groupes protecteurs du produit obtenu, et l'on obtient un composé de formule I dans laquelle la double liaison à l'intérieur du groupe A est directement voisine du groupe phosphono; ou bien

b) on condense un composé de formule VIII

Het—A—X　　VIII

dans laquelle Het, A et $R^1$ ont les significations indiquées en référence à la formule I et X représente un groupe hydroxy estérifié réactif, avec un composé permettant d'introduire le radical d'acide phosphonique et qui répond à l'une des formules IX et X

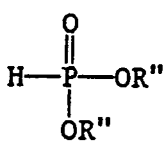 (IX)　　P(R''')$_3$　　(X)

dans lesquelles R'' représente un groupe alkyle en $C_1$—$C_4$ et R''' un halogène ou un groupe alcoxy en $C_1$—$C_4$, et lorsque c'est nécessaire, on convertit le dérivé d'acide phosphonique ainsi formé en l'acide phosphonique ou un autre ester de celui-ci; ou bien

c) on convertit en $R^1$ un substituant autre que $R^1$ en position 2 de l'hétérocycle dans un composé par ailleurs identique à un composé selon l'invention; en outre on met ces procédés en oeuvre avec, lorsque c'est nécessaire, protection transitoire de tous les groupes réactifs gênants et libération subséquente du composé formé selon l'invention; si on le désire, on convertit un composé obtenu répondant à l'invention en un autre composé selon l'invention et/ou si on le désire, on convertit un composé obtenu à l'état libre en un sel ou un sel obtenu en le composé libre ou en un autre sel; et/ou on sépare un mélange d'isomères ou un mélanges de racémates en les isomères ou racémates individuels; et/ou si on le désire, on résout un racémate en les antipodes optiques.

27. Procédé selon la revendication 26, caractérisé en ce que l'on convertit un composé de formule I dans laquelle Het est substitué à l'azote par un groupe alcanoyle en $C_2$—$C_7$ ou alcoxycarbonyle en $C_2$—$C_7$, R et R' représentent des groupes alkyle en $C_1$—$C_4$, benzyle, benzyle substitué dans la partie phényle par des halogènes, des groupes alkyle en $C_1$—$C_4$ ou alcoxy en $C_1$—$C_4$, alcanoyloxyméthyle en $C_2$—$C_7$ ou alcanoyloxyméthyle en $C_2$—$C_7$ substitué dans la partie oxyméthyle par un groupe alkyle en $C_1$—$C_4$ ou cycloalkyle en $C_3$—$C_8$, et $R^1$ représente un groupe (alcoxy en $C_1$—$C_4$)-carbonyle, carbamyle ou N-mono- ou N,N-di-(alkyle en $C_1$—$C_4$)-carbonyle, par traitement à l'aide d'un acide minéral ou d'un alcali aqueux, en un composé correspondant de formule I non substitué à l'azote et pour lequel R et R' représentent l'hydrogène et $R^1$ un groupe carboxy.

28. Procédé selon la revendication 26, caractérisé en ce que l'on convertit un composé de formule I dans laquelle Het est substitué à l'azote par un groupe alcanoyle en $C_2$—$C_7$ ou alcoxycarbonyle en $C_2$—$C_7$, R et R' représentent des groupes alkyle en $C_1$—$C_4$ et $R^1$ un groupe (alcoxy en $C_1$—$C_4$)-carbonyle, par traitement à l'aide d'un acide minéral ou d'un alcali aqueux, en un composé correspondant de formule I non substitué à l'azote et pour lequel R et R' représentent l'hydrogène et $R^1$ un groupe carboxy.

**Revendications pour les Etats contractants: ES GR**

1. Procédé de préparation des composés selon la revendication 1 répondant à la formule I

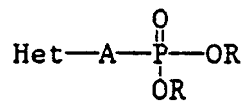 (I)

dans laquelle Het représente un groupe 2-$R^1$-pyrrolidinyle, 2-$R^1$-2,5-dihydropyrrolyle, 2-$R^1$-1,2,3,6-tétrahydropyridinyle, 2-$R^1$-1,2,5,6-tétrahydropyridinyle, 2-$R^1$-pipéridinyle, 2-$R^1$-tétrahydroquinoléinyle, 2-$R^1$-perhydroquinoléinyle, 2-$R^1$-2,3-dihydroindolyle ou 2-$R^1$-perhydroindolyle qui peut également être substitué à l'azote par des groupes alkyle en $C_1$—$C_4$, phényl-alkyle en $C_1$—$C_4$, alcoxycarbonyle en $C_2$—$C_7$, benzyloxycarbonyle ou alcanoyle en $C_2$—$C_7$ et/ou substitué sur le carbone dans le noyau hétérocyclique à 5 ou 6 chaînons par des groupes alkyle en $C_1$—$C_4$ ou phénylalkyle en $C_1$—$C_4$ et/ou substitué sur le carbone dans un radical benzo ou cyclohexano condensé éventuel par des groupes alkyle en $C_1$—$C_4$, alcoxy en $C_1$—$C_4$, des halogènes ou des groupes trifluorométhyle, $R^1$ représente un groupe carboxy, (alcoxy en $C_1$—$C_4$)-carbonyle, carbamyle ou N-mono- ou N,N-di-(alkyle en $C_1$—$C_4$)-carbamyle, A représente un groupe alcénylène en $C_2$—$C_4$ et R et R' représentent chacun, indépendamment l'un de l'autre, l'hydrogène, un groupe alkyle en $C_1$—$C_4$, benzyle, benzyle substitué dans la partie phényle par des halogènes, des groupes alkyle en $C_1$—$C_4$ ou alcoxy en $C_1$—$C_4$, alcanoyloxyméthyle en $C_2$—$C_7$ ou alcanoyloxyméthyle en $C_2$—$C_7$ substitué dans la partie oxyméthyle par un groupe alkyle en $C_1$—$C_4$ ou cycloalkyle en $C_3$—$C_8$; et de leurs sels, caractérisé en ce que

a) on condense un aldéhyde ou une cétone de formule VII

Het—A' VII

dans laquelle Het et $R^1$ ont les significations indiquées en référence à la formule I mais $R^1$ et les groupes amino sont à l'état protégé, et A' représente un groupe alkyle en $C_1$—$C_3$ substitué par un groupe oxo et contenant un atome de carbone de moins que le groupe alcénylène A, avec un tétraester de l'acide méthylène-diphosphonique en milieu basique, et lorsque c'est nécessaire, on élimine les groupes protecteurs du produit obtenu, et l'on obtient un composé de formule I dans laquelle la double liaison à l'intérieur du groupe A est directement voisine du groupe phosphono; ou bien

b) on condense un composé de formule VIII

Het—A—X VIII

dans laquelle Het, A et $R^1$ ont les significations indiquées en référence à la formule I et X représente un groupe hydroxy estérifié réactif, avec un composé permettant d'introduire le radical d'acide phosphonique et qui répond à l'une des formules IX et X

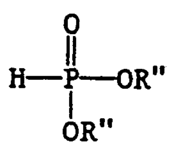 (IX) $P(R''')_3$ (X)

dans lesquelles R'' représente un groupe alkyle en $C_1$—$C_4$ et R''' un halogène ou un groupe alcoxy en $C_1$—$C_4$, et lorsque c'est nécessaire, on convertit le dérivé d'acide phosphonique ainsi formé en l'acide phosphonique ou un autre ester de celui-ci; ou bien

c) on convertit en $R^1$ un substituant autre que $R^1$ en position 2 de l'hétérocycle dans un composé par ailleurs identique à un composé selon l'invention; en outre on met ces procédés en oeuvre avec, lorsque c'est nécessaire, protection transitoire de tous les groupes réactifs gênants et libération subséquente du composé formé selon l'invention; si on le désire, on convertit un composé obtenu répondant à l'invention en un autre composé selon l'invention et/ou si on le désire, on convertit un composé obtenu à l'état libre en un sel ou un sel obtenu en le composé libre ou en un autre sel et/ou on sépare un mélange d'isomères ou un mélanges de racémates en les isomères ou racémates individuels; et/ou si on le désire, on résout un racémate en les antipodes optiques.

2. Procédé selon la revendication 1, caractérisé en ce que l'on convertit un composé de formule I dans laquelle Het est substitué à l'azote par un groupe alcanoyle en $C_2$—$C_7$ ou alcoxycarbonyle en $C_2$—$C_7$, R et R' représentent des groupes alkyle en $C_1$—$C_4$, benzyle, benzyle substitué dans la partie phényle par des halogènes, des groupes alkyle en $C_1$—$C_4$ ou alcoxy en $C_1$—$C_4$, alcanoyloxyméthyle en $C_2$—$C_7$ ou alcanoyloxyméthyle en $C_2$—$C_7$ substitué dans la partie oxyméthyle par un groupe alkyle en $C_1$—$C_4$ ou cycloalkyle en $C_3$—$C_8$, et $R^1$ représente un groupe (alcoxy en $C_1$—$C_4$)-carbonyle, carbamyle ou N-mono- ou N,N-di-(alkyle en $C_1$—$C_4$)-carbonyle, par traitement à l'aide d'un acide minéral ou d'un alcali aqueux, en un

composé correspondant de formule I non substitué à l'azote et pour lequel R et R' représentent l'hydrogène et $R^1$ un groupe carboxy.

3. Procédé selon la revendication 1, caractérisé en ce que l'on convertit un composé de formule I dans laquelle Het est substitué à l'azote par un groupe alcanoyle en $C_2$—$C_7$ ou alcoxycarbonyle en $C_2$—$C_7$, R et R' représentent des groupes alkyle en $C_1$—$C_4$ et $R^1$ un groupe (alcoxy en $C_1$—$C_4$)-carbonyle, par traitement à l'aide d'un acide minéral ou d'un alcali aqueux, en un composé correspondant de formule I non substitué à l'azote et pour lequel R et R' représentent l'hydrogène et $R^1$ un groupe carboxy.

4. Procédé selon l'une des revendications 1 à 3, caractérisé en ce que l'on prépare des composés de formule II

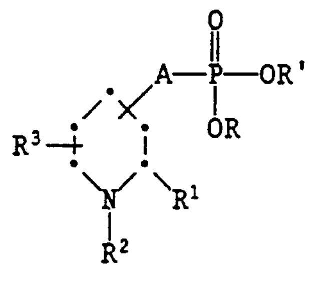

(II),

ou des composés de formule II contenant une double liaison entre les carbones C-3 et C-4 ou entre les carbones C-4 et C-5 du cycle pipéridinyle, pour lesquels R et R' représentent chacun, indépendamment l'un de l'autre, l'hydrogène, un groupe alkyle en $C_1$—$C_4$, benzyle, benzyle substitué dans la partie phényle par des halogènes, des groupes alkyle en $C_1$—$C_4$ ou alcoxy en $C_1$—$C_4$, alcanoyloxyméthyle en $C_2$—$C_7$ ou alcanoyloxyméthyle en $C_2$—$C_7$ substitué dans la partie oxyméthyle par un groupe alkyle en $C_1$—$C_4$ ou cycloalkyle en $C_3$—$C_8$, $R^1$ représente un groupe carboxy, (alcoxy en $C_1$—$C_4$)-carbonyle, carbamyle ou N-mono- ou N,N-di-(alkyle en $C_1$—$C_4$)-carbamyle, $R^2$ représente l'hydrogène, un groupe alkyle en $C_1$—$C_4$, phénylalkyle en $C_1$—$C_4$, alcoxycarbonyle en $C_2$—$C_7$, benzyloxycarbonyle ou alcanoyle en $C_2$—$C_7$, $R^3$ représente l'hydrogène, un groupe alkyle en $C_1$—$C_4$ ou phényl-alkyle en $C_1$—$C_4$ et A représente un groupe alcénylène en $C_2$—$C_4$; et leurs sels.

5. Procédé selon l'une des revendications 1 à 3, caractérisé en ce que l'on prépare des composés de formule II selon la revendication 2, pour lesquels R et R' représentent chacun, indépendamment l'un de l'autre, l'hydrogène, un groupe alkyle en $C_1$—$C_4$, benzyle, alcanoyloxyméthyle en $C_2$—$C_7$ ou alcanoyloxyméthyle en $C_2$—$C_7$ substitué dans la partie oxyméthyle par un groupe alkyle en $C_1$—$C_4$, cyclohexyle ou cyclopentyle, $R^1$ représente un groupe carboxy, carbamoyle ou (alcoxy en $C_1$—$C_4$)-carbonyle, $R^2$ et $R^3$ représentent l'hydrogène ou des groupes alkyle en $C_1$—$C_4$ et A représente un groupe alcénylène en $C_2$—$C_4$; et leurs sels acceptables pour l'usage pharmaceutique.

6. Procédé selon l'une des revendications 1 à 3, caractérisé en ce que l'on prépare des composés de formule II selon la revendication 2 pour lesquels R et R' représentent chacun, indépendamment l'un de l'autre, l'hydrogène, un groupe alcanoyloxyméthyle en $C_2$—$C_7$ ou alcanoyloxyméthyle en $C_2$—$C_7$ substitué dans la partie oxyméthyle par un groupe alkyle en $C_1$—$C_4$, $R^1$ représente un groupe carboxy, carbamoyle ou (alcoxy en $C_1$—$C_4$)-carbonyle, $R^2$ et $R^3$ représentent l'hydrogène, A est en position 4 et représente un groupe alcénylène en $C_3$ ou $C_4$ avec la double liaison voisine du groupe phosphono; et leurs sels acceptables pour l'usage pharmaceutique.

7. Procédé selon l'une des revendications 1 à 3 et 6, caractérisé en ce que l'on prépare des composés de formule III

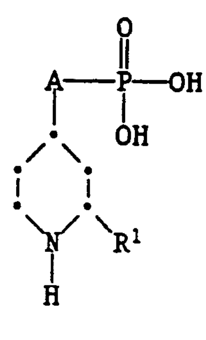

(III),

dans laquelle A représente un groupe 1,3-propénylène, de préférence avec la double liaison voisine du groupe phosphono et $R^1$ représente un groupe carboxy ou (alcoxy en $C_1$—$C_4$)-carbonyle; ou leurs sels acceptables pour l'usage pharmaceutique.

8. Procédé selon l'une des revendications 1 à 3, caractérisé en ce que l'on prépare l'acide 4-[1-(3-phosphonopropa-2-ényl)]-pipéridine-2-carboxylique ou ses sels acceptables pour l'usage pharmaceutique.

9. Procédé selon l'une des revendications 1 à 3, caractérisé en ce que l'on prépare l'acide 4-[1-(3-phosphonopropa-1-ényl)]-pipéridine-2-carboxylique ou ses sels acceptables pour l'usage pharmaceutique.

10. Procédé selon l'une des revendications 1 à 3, caractérisé en ce que l'on prépare des composés selon la revendication 1, répondant à la formule IV

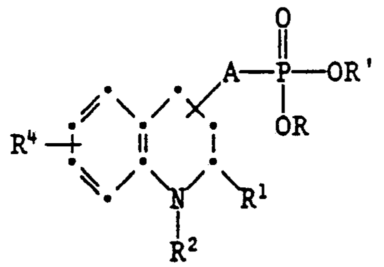

(IV)

ou leurs dérivés perhydrogénés, pour lesquels R et R' représentent chacun, indépendamment l'un de l'autre, l'hydrogène, un groupe alkyle en $C_1$—$C_4$, benzyle ou benzyle substitué dans la partie phényle par des halogènes, des groupes alkyle en $C_1$—$C_4$ ou alcoxy en $C_1$—$C_4$, un groupe alcanoyloxyméthyle en $C_2$—$C_7$ ou alcanoyloxyméthyle en $C_2$—$C_7$ substitué dans la partie oxyméthyle par un groupe alkyle en $C_1$—$C_4$ ou cycloalkyle en $C_3$—$C_8$, $R^1$ représente un groupe carboxy ou (alcoxy en $C_1$—$C_4$)-carbonyle, carbamyle ou N-mono- ou N,N'-di-(alkyle en $C_1$—$C_4$)-carbamyle, $R^2$ représente l'hydrogène, un groupe alkyle en $C_1$—$C_4$, phényl-alkyle en $C_1$—$C_4$, (alcoxy en $C_1$—$C_7$)-carbonyle, benzyloxycarbonyle ou alcanoyle en $C_2$—$C_7$; $R^4$ représente l'hydrogène, un groupe alkyle en $C_1$—$C_4$, alcoxy en $C_1$—$C_4$; un halogène ou un groupe trifluorométhyle et A représente un groupe alcénylène en $C_2$—$C_4$; et leurs sels.

11. Procédé selon l'une des revendications 1 à 3, caractérisé en ce que l'on prépare des composés de formule V

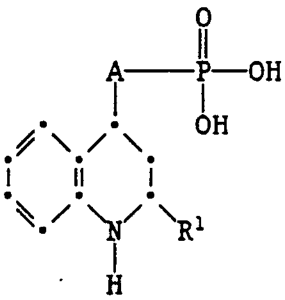

(V)

ou leurs dérivés perhydroquinoléiniques, pour lesquels A représente un groups 1,3-propénylène dont la double liaison est voisine du groupe phosphono et $R^1$ représente un groupe carboxy ou (alcoxy en $C_1$—$C_4$)-carbonyle; et les sels acceptables pour l'usage pharmaceutique de ces composés qui contiennent un groupe fonctionnel salifiable.

12. Procédé selon l'une des revendications 1 à 3, caractérisé en ce que l'on prépare des composés de formule VI

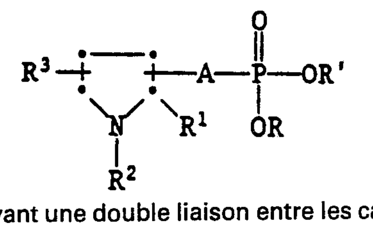

(VI)

ou les composés de formule VI ayant une double liaison entre les carbone C-3 et C-4 du cycle pyrrolidinyle, pour lesquels R et R' représentent chacun, indépendamment l'un de l'autre, l'hydrogène, un groupe alkyle en $C_1$—$C_4$, benzyle, benzyle substitué dans la partie phényle par des halogènes, des groupes alkyle en $C_1$—$C_4$ ou alcoxy en $C_1$—$C_4$, un groupe alcanoyloxyméthyle en $C_2$—$C_7$ ou alcanoyloxyméthyle en $C_2$—$C_7$ substitué dans la partie oxyméthyle par un groupe alkyle en $C_1$—$C_4$ ou cycloalkyle en $C_3$—$C_8$, $R^1$ représente un groupe carboxy ou (alcoxy en $C_1$—$C_4$)-carbonyle, carbamyle ou N-mono- ou N,N-di-(alkyle en $C_1$—$C_4$)-carbamyle, $R^2$ représente l'hydrogène, un groupe alkyle en $C_1$—$C_4$, (alcoxy en $C_1$—$C_7$)-carbonyle, benzyloxycarbonyle ou alcanoyle en $C_2$—$C_7$, $R^3$ représente l'hydrogène, un groupe alkyle en $C_1$—$C_4$ ou aryl-alkyle en $C_1$—$C_4$ et A représente un groupe alcénylène en $C_2$—$C_4$; et leurs sels.

13. Procédé selon l'une des revendications 1 à 3, caractérisé en ce que l'on prépare des composés de formule VI selon la revendication 12 pour lesquels le groupe portant la fonction phosphono est fixé en position 3; R et R' représentent l'hydrogène, $R^1$ représente un groupe carboxy ou (alcoxy en $C_1$—$C_4$)-carbonyle, $R^2$ et $R^3$ représentent l'hydrogène et A un groupe 1,3-propénylène dont la double liaison est voisine du groupe phosphono; et leurs sels acceptables pour l'usage pharmaceutique.

14. Procédé selon l'une des revendications 1 à 3, caractérisé en ce que l'on prépare l'acide trans-3-[1-(4-phosphonobuta-3-ényl)]-pyrrolidine-2-carboxylique ou ses sels acceptables pour l'usage pharmaceutique.

15. Procédé selon l'une des revendications 1 à 3, caractérisé en ce que l'on prépare l'acide trans-3-[1-(4-phosphonobuta-2-ényl)]-pyrrolidine-2-carboxylique ou ses sels acceptables pour l'usage pharmaceutique.

16. Procédé de préparation de compositions pharmaceutiques, caractérisé en ce que l'on mélange un composé obtenu selon l'une des revendications 1 à 15 avec un véhicule approprié à l'usage pharmaceutique.

17. Procédé selon la revendication 16, pour la preparation de compositions pharmaceutiques utilisées en tant qu'antagonistes du NMDA.

18. Procédé selon l'une des revendications 1 à 3, pour la préparation de composés choisis dans le groupe consistant en l'acide cis-4-[1-(3-phosphonopropa-2-ényl)]-pipéridine-2-carboxylique, l'acide trans-4-[1-(3-phosphonopropa-2-ényl)]-pipéridine-2-carboxylique, l'acide cis-4-[1-(3-phosphonopropa-1-ényl)]-pipéridine-2-carboxylique et l'acide trans-4-[1-(3-phosphonopropa-1-ényl)]-pipéridine-2-carboxylique et leurs sels acceptables pour l'usage pharmaceutique.

19. Procédé selon l'une des revendications 1 à 3, pour la préparation de l'acide cis-3-[1-(4-phosphonobuta-3-ényl)]-pyrrolidine-2-carboxylique et de ses sels acceptables pour l'usage pharmaceutique.

20. Procédé de préparation de compositions pharmaceutiques, caractérisé en ce que l'on mélange un composé obtenu selon l'une des revendications 18 et 19 avec un véhicule approprié à l'usage pharmaceutique.

21. Procédé selon la revendication 20, pour la préparation de compositions pharmaceutiques utilisées en tant qu'antagonistes du NMDA.

**Claims for the Contracting States: AT BE CH DE FR GB IT LI LU NL SE**

1. Compounds of formula I,

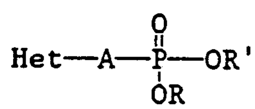

(I)

in which Het is a 2-$R^1$-pyrrolidinyl, 2-$R^1$-2,5-dihydropyrrolyl, 2-$R^1$-1,2,3,6-tetrahydropyridinyl, 2-$R^1$-1,2,5,6-tetrahydropyridinyl, 2-$R^1$-piperidinyl, 2-$R^1$-tetrahydroquinolinyl, 2-$R^1$-perhydroquinolinyl, 2-$R^1$-2,3-dihydroindolyl or 2-$R^1$-perhydroindolyl group which also may be N-substituted by $C_1$—$C_4$alkyl, phenyl-$C_1$—$C_4$alkyl, $C_2$—$C_7$alkoxycarbonyl, benzyloxycarbonyl or by $C_2$—$C_7$alkanoyl and/or C-substituted in the heterocyclic 5- or 6-ring by $C_1$—$C_4$alkyl or by phenyl-$C_1$—$C_4$alkyl and/or, in the fused benzo or cyclohexano radical that may be present, by $C_1$—$C_4$alkyl, $C_1$—$C_4$alkoxy, halogen or by trifluoromethyl, $R^1$ is carboxy, $C_1$—$C_4$alkoxycarbonyl, carbamoyl or N-mono- or N,N-di-$C_1$—$C_4$alkylcarbamoyl, A is $C_2$—$C_4$alkenylene and R and R' are each independently hydrogen, $C_1$—$C_4$alkyl, benzyl, benzyl substituted by the phenyl moiety by halogen, by $C_1$—$C_4$alkyl or by $C_1$—$C_4$alkoxy, $C_2$—$C_7$alkanoyloxymethyl, or $C_2$—$C_7$alkanoyloxymethyl substituted in the oxymethyl moiety by $C_1$—$C_4$alkyl or by $C_3$—$C_8$cycloalkyl; and salts thereof.

2. Compounds according to claim 1 of formula II

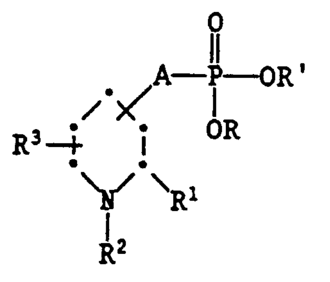

(II),

and compounds of formula II with a double bond present between C-3 and C-4 or between C-4 and C-5 of the piperidinyl ring, wherein R and R' are each independently hydrogen, $C_1$—$C_4$alkyl, benzyl, benzyl substituted in the phenyl moiety by halogen, by $C_1$—$C_4$alkyl or by $C_1$—$C_4$alkoxy, $C_2$—$C_7$alkanoyloxymethyl, or $C_2$—$C_7$alkanoyloxymethyl substituted in the oxymethyl moiety by $C_1$—$C_4$alkyl or by $C_3$—$C_8$cycloalkyl, $R^1$ is carboxy, $C_1$—$C_4$alkoxycarbonyl, carbamoyl or N-mono- or N,N-di-$C_1$—$C_4$alkylcarbamoyl, $R^2$ is hydrogen, $C_1$—$C_4$alkyl, phenyl-$C_1$—$C_4$alkyl, $C_2$—$C_7$alkoxycarbonyl, benzyloxycarbonyl or $C_2$—$C_7$alkanoyl, $R^3$ is hydrogen, $C_1$—$C_4$alkyl or phenyl-$C_1$—$C_4$alkyl, and A is $C_2$—$C_4$alkenylene; and salts thereof.

3. Compounds of formula II according to claim 2, wherein R and R' are each independently hydrogen, $C_1$—$C_4$alkyl, benzyl, $C_2$—$C_7$alkanoyloxymethyl, or $C_2$—$C_7$alkanoyloxymethyl substituted in the oxymethyl moiety by $C_1$—$C_4$alkyl, by cyclohexyl or by cyclopentyl, $R^1$ is carboxy, carbamoyl or $C_1$—$C_4$alkoxycarbonyl; $R^2$ and $R^3$ are hydrogen or $C_1$—$C_4$alkyl, and A is alkenylene having from 2 to 4 carbon atoms; and pharmaceutically acceptable salts thereof.

4. Compounds of formula II according to claim 2, wherein R and R' are each independently hydrogen, $C_2$—$C_7$alkanoyloxymethyl or $C_2$—$C_7$alkanoyloxymethyl substituted in the oxymethyl moiety by $C_1$—$C_4$alkyl, $R^1$ is carboxy, carbamoyl or $C_1$—$C_4$alkoxycarbonyl, $R^2$ and $R^3$ are hydrogen, and A is in the 4-position and is alkenylene having 3 or 4 carbon atoms with the double bond adjacent to the phosphono group; and pharmaceutically acceptable salts thereof.

5. Compounds according to claim 4 of formula III

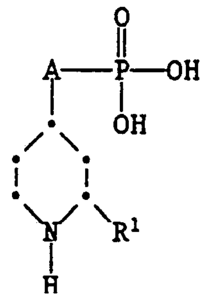

(III),

wherein A is 1,3-propenylene, preferably with the double bond adjacent to the phosphono group, and $R^1$ is carboxy or $C_1$—$C_4$alkoxycarbonyl; and pharmaceutically acceptable salts thereof.

6. 4-[1-(3-Phosphonoprop-2-enyl)]-piperidine-2-carboxylic acid according to claim 1 and pharmaceutically acceptable salts thereof.

7. 4-[1-(3-Phosphonoprop-1-enyl)]-piperidine-2-carboxylic acid according to claim 1 and pharmaceutically acceptable salts thereof.

8. Compounds according to claim 1 of formula IV

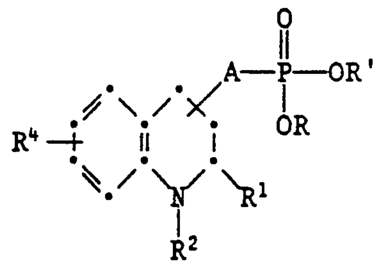

(IV)

or perhydro derivatives thereof, wherein R and R′ are each independently hydrogen, $C_1$—$C_4$alkyl, benzyl, benzyl substituted in the phenyl moiety by halogen, by $C_1$—$C_4$alkyl or by $C_1$—$C_4$alkoxy, $C_2$—$C_7$alkanoyloxymethyl, or $C_2$—$C_7$alkanoyloxymethyl substituted in the oxymethyl moiety by $C_1$—$C_4$alkyl or by $C_3$—$C_8$-cycloalkyl, $R^1$ is carboxy, $C_1$—$C_4$alkoxycarbonyl, carbamoyl or N-mono- or N,N′-di-$C_1$—$C_4$alkylcarbamoyl, $R^2$ is hydrogen, $C_1$—$C_4$alkyl, phenyl-$C_1$—$C_4$alkyl, $C_1$—$C_7$alkoxycarbonyl, benzyloxycarbonyl or $C_2$—$C_7$-alkanoyl, $R^4$ is hydrogen, $C_1$—$C_4$alkyl, $C_1$—$C_4$alkoxy, halogen or trifluoromethyl, and A is $C_2$—$C_4$-alkenylene; and salts thereof.

9. Compounds according to claim 8 of formula V

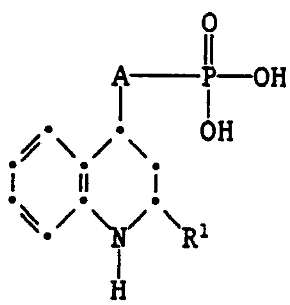

(V)

or perhydroquinoline derivatives thereof, wherein A is 1,3-propenylene with the double bond adjacent to the phosphono group, and $R^1$ is carboxy or $C_1$—$C_4$alkoxycarbonyl; and pharmaceutically acceptable salts of said compounds having a salt-forming functional group.

10. Compounds according to claim 1 of formula VI

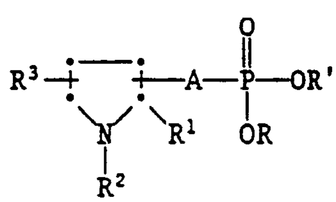

(VI)

and the compounds of formula VI with a double bond present between C-3 and C-4 of the pyrrolidinyl ring, wherein R and R′ are each independently hydrogen, $C_1$—$C_4$alkyl, benzyl, benzyl substituted in the phenyl moiety by halogen, by $C_1$—$C_4$alkyl or by $C_1$—$C_4$alkoxy, $C_2$—$C_7$alkanoyloxymethyl, or $C_2$—$C_7$alkanoyloxy-

methyl substituted in the oxymethyl moiety by $C_1$—$C_4$alkyl or by $C_3$—$C_8$cycloalkyl, $R^1$ is carboxy, $C_1$—$C_4$-alkoxycarbonyl, carbamoyl or N-mono- or N,N-di-$C_1$—$C_4$alkylcarbamoyl, $R^2$ is hydrogen, $C_1$—$C_4$alkyl, $C_1$—$C_7$alkoxycarbonyl, benzyloxycarbonyl or $C_2$—$C_7$alkanoyl, $R^3$ is hydrogen, $C_1$—$C_4$alkyl or aryl-$C_1$—$C_4$alkyl, and A is $C_2$—$C_4$alkenylene; and salts thereof.

11. Compounds of formula VI according to claim 10, wherein the phosphono-bearing group is attached at the 3-position; R and R′ are hydrogen, $R^1$ is carboxy or $C_1$—$C_4$alkoxycarbonyl, $R^2$ and $R^3$ are hydrogen, and A is 1,3-propenylene with the double bond adjacent to the phosphono group; and pharmaceutically acceptable salts thereof.

12. *trans*-3-[1-(4-Phosphonobut-3-enyl)]-pyrrolidine-2-carboxylic acid according to claim 1 and pharmaceutically acceptable salts thereof.

13. *trans*-3-[1-(4-Phosphonobut-2-enyl)]-pyrrolidine-2-carboxylic acid according to claim 1 and pharmaceutically acceptable salts thereof.

14. The compounds mentioned in claims 1 to 13 for use in a method for the therapeutic treatment of the human or animal body.

15. Pharmaceutical preparations containing a compound according to any one of claims 1 to 14 together with a pharmaceutically suitable carrier.

16. The compounds mentioned in claims 1 to 14 as NMDA-antagonists.

17. The use of the compounds mentioned in claims 1 to 14 for the manufacture of pharmaceutical preparations.

18. The use of the compounds mentioned in claims 1 to 14 for the manufacture of pharmaceutical preparations for use as NMDA-antagonists.

19. Compounds according to claim 5, selected from the group consisting of *cis*-4-[1-(3-phosphonoprop-2-enyl)]-piperidine-2-carboxylic acid, *trans*-4-[1-(3-phosphonoprop-2-enyl])-piperidine-2-carboxylic acid, *cis*-4-[1-(3-phosphonoprop-1-enyl)]-piperidine-2-carboxylic acid and *trans*-4-[1-(3-phosphonoprop-1-enyl)]-piperidine-2-carboxylic acid, and pharmaceutically acceptable salts thereof.

20. Compounds according to claim 10, selected from *cis*-3-[1-(4-phosphonobut-3-enyl)]-pyrrolidine-2-carboxylic acid and pharmaceutically acceptable salts thereof.

21. Pharmaceutical preparations containing a compound according to claim 19 or 20 together with a pharmaceutically suitable carrier.

22. The compounds mentioned in claims 19 and 20 for use in a method for the therapeutic treatment of the human or animal body.

23. The compounds mentioned in claims 19 and 20 as NMDA-antagonists.

24. The use of the compounds mentioned in claims 19 and 20 for the manufacture of pharmaceutical preparations.

25. The use of the compounds mentioned in claims 19 and 20 for the manufacture of pharmaceutical preparations for use as NMDA-antagonists.

26. A process for the manufacture of compounds according to claim 1 of formula I in which all of the symbols have the meanings given in claim 1, and salts thereof, which comprises

a) condensing an aldehyde or ketone of formula VII

Het—A′ (VII)

wherein Het and $R^1$ are as defined for formula I but $R^1$ and amino groups are in protected form, and A′ is $C_1$—$C_3$alkyl substituted by oxo and containing one carbon atom less than the alkenylene group A, with a tetraester derivative of methylenediphosphonic acid under basic conditions and, if required, removing protecting groups from the resulting product to obtain a compound of formula I wherein the double bond within group A is directly adjacent to the phosphono group; or

b) condensing a compound of formula VIII

Het—A—X (VIII)

wherein Het, A and $R^1$ are as defined for formula I and X is reactive esterified hydroxy, with a compound capable of introducing the phosphonic acid moiety and having one of the formulae IX and X

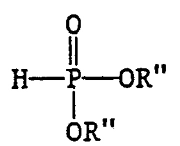 (IX) $P(R''')_3$ (X)

wherein R″ is $C_1$—$C_4$alkyl and R‴ is halogen or $C_1$—$C_4$alkoxy and, if required, converting the resulting phosphonic acid derivative to the phosphonic acid or another ester derivative thereof; or

c) converting to $R^1$ a substituent other than $R^1$ at position 2 of the heterocyclic ring in a compound

otherwise identical to a compound of the invention; and carrying out the said processes with, if necessary, temporary protection of any interfering reactive groups, and then freeing the resulting compound of the invention; if desired converting a resulting compound of the invention into another compound of the invention and/or, if desired, converting a resulting free compound into a salt or a resulting salt into the free compound or into another salt; and/or separating a mixture of isomers or of racemates into the individual isomers or racemates; and/or, if desired, resolving a racemate into the optical antipodes.

27. A process according to claim 26, which comprises converting a compound of formula I in which Het is N-substituted by $C_2$—$C_7$alkanoyl or by $C_2$—$C_7$alkoxycarbonyl, R and R′ are $C_1$—$C_4$alkyl, benzyl, benzyl substituted in the phenyl moiety by halogen, by $C_1$—$C_4$alkyl or by $C_1$—$C_4$alkoxy, $C_2$—$C_7$alkanoyloxymethyl, or $C_2$—$C_7$alkanoyloxymethyl substituted in the oxymethyl moiety by $C_1$—$C_4$alkyl or by $C_3$—$C_8$cycloalkyl, and $R^1$ is $C_1$—$C_4$alkoxycarbonyl, carbamoyl or N-mono- or N,N-di-$C_1$—$C_4$alkylcarbonyl, into a corresponding N-unsubstituted compound of formula I in which R and R′ are hydrogen and $R^1$ is carboxy, by treatment with an inorganic acid or with aqueous alkalis.

28. A process according to claim 26, which comprises converting a compound of formula I in which Het is N-substituted by $C_2$—$C_7$alkanoyl or by $C_2$—$C_7$alkoxycarbonyl, R and R′ are $C_1$—$C_4$alkyl and $R^1$ is $C_1$—$C_4$-alkoxycarbonyl, into a corresponding N-unsubstituted compound of formula I in which R and R′ are hydrogen and $R^1$ is carboxy, by treatment with an inorganic acid or with aqueous alkalis.

**Claims for the Contracting States: ES GR**

1. A process for the manufacture of compounds according to claim 1 of formula I,

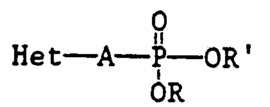

(I)

in which Het is a 2-$R^1$-pyrrolidinyl, 2-$R^1$-2,5-dihydropyrrolyl, 2-$R^1$-1,2,3,6-tetrahydropyridinyl, 2-$R^1$-1,2,5,6-tetrahydropyridinyl, 2-$R^1$-piperidinyl, 2-$R^1$-tetrahydroquinolinyl, 2-$R^1$-perhydroquinolinyl, 2-$R^1$-2,3-dihydroindolyl or 2-$R^1$-perhydroindolyl group which also may be N-substituted by $C_1$—$C_4$alkyl, phenyl-$C_1$—$C_4$alkyl, $C_2$—$C_7$alkoxycarbonyl, or by $C_2$—$C_7$alkanoyl and/or C-substituted in the heterocyclic 5- or 6-ring by $C_1$—$C_4$alkyl or by phenyl-$C_1$—$C_4$alkyl and/or, in the fused benzo or cyclohexano radical that may be present, by $C_1$—$C_4$alkyl, $C_1$—$C_4$alkoxy, halogen or by trifluoromethyl, $R^1$ is carboxy, $C_1$—$C_4$alkoxycarbonyl, carbamoyl or N-mono- or N,N-di-$C_1$—$C_4$alkylcarbamoyl, A is $C_2$—$C_4$alkenylene and R and R′ are each independently hydrogen, $C_1$—$C_4$alkyl, benzyl, benzyl substituted in the phenyl moiety by halogen, by $C_1$—$C_4$alkyl or by $C_1$—$C_4$alkoxy, $C_2$—$C_7$alkanoyloxymethyl, or $C_2$—$C_7$alkanoyloxymethyl substituted in the oxymethyl moiety by $C_1$—$C_4$alkyl or by $C_3$—$C_8$cycloalkyl; and salts thereof, which comprises

a) condensing an aldehyde or ketone of formula VII

Het—A′ (VII)

wherein Het and $R^1$ are as defined for formula I but $R^1$ and amino groups are in protected form, and A′ is $C_1$—$C_3$alkyl substituted by oxo and containing one carbon atom less than the alkenylene group A, with a tetraester derivative of methylenediphosphonic acid under basic conditions and, if required, removing protecting groups from the resulting product to obtain a compound of formula I wherein the double bond within group A is directly adjacent to the phosphono group; or

b) condensing a compound of formula VIII

Het—A—X (VIII)

wherein Het, A and $R^1$ are as defined for formula I and X is reactive esterified hydroxy, with a compound capable of introducing the phosphonic acid moiety and having one of the formulae IX and X

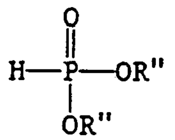

(IX) $P(R''')_3$ (X)

wherein R″ is $C_1$—$C_4$alkyl and R‴ is halogen or $C_1$—$C_4$alkoxy and, if required, converting the resulting phosphonic acid derivative to the phosphonic acid or another ester derivative thereof; or

c) converting to $R^1$ a substituent other than $R^1$ at position 2 of the heterocyclic ring in a compound otherwise identical to a compound of the invention; and carrying out the said processes with, if necessary, temporary protection of any interfering reactive groups, and then freeing the resulting compound of the

invention; if desired converting a resulting compound of the invention into another compound of the invention and/or, if desired, converting the resulting free compound into a salt or a resulting salt into the free compound or into another salt; and/or separating a mixture of isomers or of racemates into the individual isomers or racemates; and/or, if desired, resolving a racemate into the optical antipodes.

2. A process according to claim 1, which comprises converting a compound of formula I in which Het is N-substituted by $C_2$—$C_7$alkanoyl or by $C_2$—$C_7$alkoxycarbonyl, R and R' are $C_1$—$C_4$alkyl, benzyl, benzyl substituted in the phenyl moiety by halogen, by $C_1$—$C_4$alkyl or by $C_1$—$C_4$alkoxy, $C_2$—$C_7$alkanoyloxymethyl, or $C_2$—$C_7$alkanoyloxymethyl substituted in the oxymethyl moiety by $C_1$—$C_4$alkyl or by $C_3$—$C_8$cycloalkyl, and $R^1$ is $C_1$—$C_4$alkoxycarbonyl, carbamoyl or N-mono- or N,N-di-$C_1$—$C_4$alkylcarbonyl, into a corresponding N-unsubstituted compound of formula I in which R and R' are hydrogen and $R^1$ is carboxy, by treatment with an inorganic acid or with aqueous alkalis.

3. A process according to claim 1, which comprises converting a compound of formula I in which Het is N-substituted by $C_2$—$C_7$alkanoyl or by $C_2$—$C_7$alkoxycarbonyl, R and R' are $C_1$—$C_4$alkyl and $R^1$ is $C_1$—$C_4$-alkoxycarbonyl, into a corresponding N-unsubstituted compound of formula I in which R and R' are hydrogen and $R^1$ is carboxy, by treatment with an inorganic acid or with aqueous alkalis.

4. A process according to any one of claims 1 to 3, which comprises manufacturing compounds of formula II

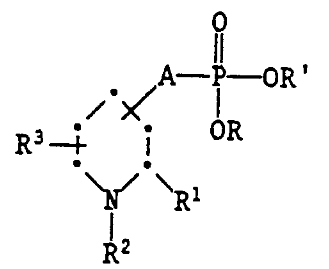

(II),

or compounds of formula II with a double bond present between C-3 and C-4 or between C-4 and C-5 of the piperidinyl ring, wherein R and R' are each independently hydrogen, $C_1$—$C_4$alkyl, benzyl, benzyl substituted in the phenyl moiety by halogen, by $C_1$—$C_4$alkyl or by $C_1$—$C_4$alkoxy, $C_2$—$C_7$alkanoyloxymethyl, or $C_2$—$C_7$-alkanoyloxymethyl substituted in the oxymethyl moiety by $C_1$—$C_4$alkyl or by $C_3$—$C_8$cycloalkyl, $R^1$ is carboxy, $C_1$—$C_4$alkoxycarbonyl, carbamoyl or N-mono- or N,N-di-$C_1$—$C_4$alkylcarbamoyl, $R^2$ is hydrogen, $C_1$—$C_4$alkyl, phenyl-$C_1$—$C_4$alkyl, $C_2$—$C_7$alkoxycarbonyl, benzyloxycarbonyl or $C_2$—$C_7$alkanoyl, $R^3$ is hydrogen, $C_1$—$C_4$alkyl or phenyl-$C_1$—$C_4$alkyl, and A is $C_2$—$C_4$alkenylene; or salts thereof.

5. A process according to any one of claims 1 to 3, which comprises manufacturing compounds of formula II according to claim 2, wherein R and R' are each independently hydrogen, $C_1$—$C_4$alkyl, benzyl, $C_2$—$C_7$alkanoyloxymethyl, or $C_2$—$C_7$alkanoyloxymethyl substituted in the oxymethyl moiety by $C_1$—$C_4$alkyl, by cyclohexyl or by cyclopentyl, $R^1$ is carboxy, carbamoyl or $C_1$—$C_4$alkoxycarbonyl; $R^2$ and $R^3$ are hydrogen or $C_1$—$C_4$alkyl, and A is alkenylene having from 2 to 4 carbon atoms; or pharmaceutically acceptable salts thereof.

6. A process according to any one of claims 1 to 3, which comprises manufacturing compounds of formula II according to claim 2, wherein R and R' are each independently hydrogen, $C_2$—$C_7$alkanoyloxymethyl or $C_2$—$C_7$alkanoyloxymethyl substituted in the oxymethyl moiety by $C_1$—$C_4$alkyl, $R^1$ is carboxy, carbamoyl or $C_1$—$C_4$alkoxycarbonyl, $R^2$ and $R^3$ are hydrogen, and A is in the 4-position and is alkenylene having 3 or 4 carbon atoms with the double bond adjacent to the phosphono group; or pharmaceutically acceptable salts thereof.

7. A process according to any one of claims 1 to 3 and 6, which comprises manufacturing compounds of formula III

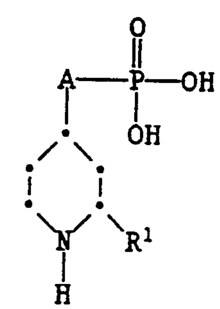

(III),

wherein A is 1,3-propenylene, preferably with the double bond adjacent to the phosphono group, and $R^1$ is carboxy or $C_1$—$C_4$alkoxycarbonyl; or pharmaceutically acceptable salts thereof.

8. A process according to any one of claims 1 to 3, which comprises manufacturing 4-[1-(3-phosphonoprop-2-enyl)]-piperidine-2-carboxylic acid or pharmaceutically acceptable salts thereof.

9. A process according to any one of claims 1 to 3, which comprises manufacturing 4-[1-(3-phosphonoprop-1-enyl)]-piperidine-2-carboxylic acid or pharmaceutically acceptable salts thereof.

10. A process according to any one of claims 1 to 3, which comprises manufacturing compounds according to claim 1 of formula IV

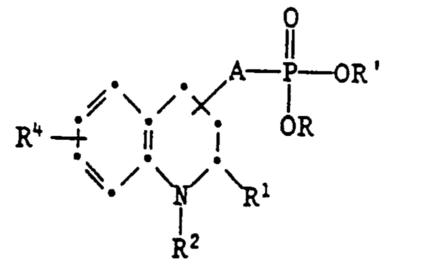

(IV)

or perhydro derivatives thereof, wherein R and R′ are each independently hydrogen, $C_1$—$C_4$alkyl, benzyl, benzyl substituted in the phenyl moiety by halogen, by $C_1$—$C_4$alkyl or by $C_1$—$C_4$alkoxy, $C_2$—$C_7$alkanoyloxymethyl, or $C_2$—$C_7$alkanoyloxymethyl substituted in the oxymethyl moiety by $C_1$—$C_4$alkyl or by $C_3$—$C_8$cycloalkyl, $R^1$ is carboxy, $C_1$—$C_4$alkoxycarbonyl, carbamoyl or N-mono- or N,N′-di-$C_1$—$C_4$alkylcarbamoyl, $R^2$ is hydrogen, $C_1$—$C_4$alkyl, phenyl-$C_1$—$C_4$alkyl, $C_1$—$C_7$alkoxycarbonyl, benzyloxycarbonyl or $C_2$—$C_7$alkanoyl, $R^4$ is hydrogen, $C_1$—$C_4$alkyl, $C_1$—$C_4$alkoxy, halogen or trifluoromethyl, and A is $C_2$—$C_4$-alkenylene; or salts thereof.

11. A process according to any one of claims 1 to 3, which comprises manufacturing compounds of formula V

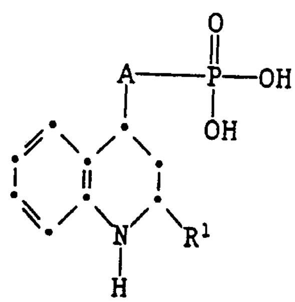

(V)

or perhydroquinoline derivatives thereof, wherein A is 1,3-propenylene with the double bond adjacent to the phosphono group, and $R^1$ is carboxy or $C_1$—$C_4$alkoxycarbonyl; or pharmaceutically acceptable salts of said compounds having a salt-forming functional group.

12. A process according to any one of claims 1 to 3, which comprises manufacturing compounds of formula VI

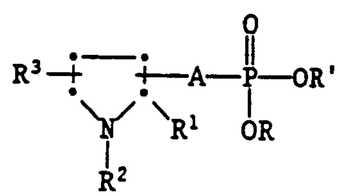

(VI)

or compounds of formula VI with a double bond present between C-3 and C-4 of the pyrrolidinyl ring, wherein R and R′ are each independently hydrogen, $C_1$—$C_4$alkyl, benzyl, benzyl substituted in the phenyl moiety by halogen, by $C_1$—$C_4$alkyl or by $C_1$—$C_4$alkoxy, $C_2$—$C_7$alkanoyloxymethyl, or $C_2$—$C_7$alkanoyloxymethyl substituted in the oxymethyl moiety by $C_1$—$C_4$alkyl or by $C_3$—$C_8$cycloalkyl, $R^1$ is carboxy, $C_1$—$C_4$-alkoxycarbonyl, carbamoyl or N-mono- or N,N-di-$C_1$—$C_4$alkylcarbamoyl, $R^2$ is hydrogen, $C_1$—$C_4$alkyl, $C_1$—$C_7$alkoxycarbonyl, benzyloxycarbonyl or $C_2$—$C_7$alkanoyl, $R^3$ is hydrogen, $C_1$—$C_4$alkyl or aryl-$C_1$—$C_4$alkyl, and A is $C_2$—$C_4$alkenylene; or salts thereof.

13. A process according to any one of claims 1 to 3, which comprises manufacturing compounds of formula VI according to claim 12, wherein the phosphono-bearing group is attached at the 3-position; R and R′ are hydrogen, $R^1$ is carboxy or $C_1$—$C_4$alkoxycarbonyl, $R^2$ and $R^3$ are hydrogen, and A is 1,3-propenylene with the double bond adjacent to the phosphono group; or pharmaceutically acceptable salts thereof.

14. A process according to any one of claims 1 to 3, which comprises manufacturing *trans*-3-[1-(4-phosphonobut-3-enyl)]-pyrrolidine-2-carboxylic acid or pharmaceutically acceptable salts thereof.

15. A process according to any one of claims 1 to 3, which comprises manufacturing *trans*-3-[1-(4-phosphonobut-2-enyl)]-pyrrolidine-2-carboxylic acid or pharmaceutically acceptable salts thereof.

16. A process for the manufacture of pharmaceutical preparations, which comprises mixing a compound obtainable in accordance with any one of claims 1 to 15 with a pharmaceutically suitable carrier.

17. A process according to claim 16 for the manufacture of pharmaceutical preparations for use as NMDA-antagonists.

18. A process according to any one of claims 1 to 3 for the manufacture of compounds selected from the group consisting of *cis*-4-[1-(3-phosphonoprop-2-enyl)]-piperidine-2-carboxylic acid, *trans*-4-[1-(3-phosphonoprop-2-enyl)]-piperidine-2-carboxylic acid, *cis*-4-[1-(3-phosphonoprop-1-enyl)]-piperidine-2-carboxylic acid and *trans*-4-[1-(3-phosphonoprop-1-enyl)]-piperidine-2-carboxylic acid, and pharmaceutically acceptable salts thereof.

19. A process according to any one of claims 1 to 3 for the manufacture of *cis*-3-[1-(4-phosphonobut-3-enyl)]-pyrrolidine-2-carboxylic acid and pharmaceutically acceptable salts thereof.

20. A process for the manufacture of pharmaceutical preparations, which comprises mixing a compound obtainable in accordance with claim 18 or 19 with a pharmaceutically suitable carrier.

21. A process according to claim 20 for the manufacture of pharmaceutical preparations for use as NMDA-antagonists.